# EUROPEAN PATENT SPECIFICATION

(11) **EP 0 640 141 B1**
(45) Date of publication and mention of the grant of the patent: **14.05.2003**
(21) Application number: 93908395.2
(22) Date of filing: 18.03.1993
(51) Int. Cl.: C12N 15/82, C12N 15/54, C12N 15/60, C12N 15/53, C12N 15/11, A01H 1/00, A01H 5/00, A01H 5/10

(54) **NUCLEIC ACID FRAGMENTS AND METHODS FOR INCREASING THE LYSINE AND THREONINE CONTENT OF THE SEEDS OF PLANTS**
NUKLEINSÄUREFRAGMENTE UND VERFAHREN ZUR STEIGERUNG DES LYSIN- UND THREONINGEHALTES DER PFLANZENSAMEN
FRAGMENTS D'ACIDE NUCLEIQUE ET PROCEDE D'AUGMENTATION DE LA TENEUR EN LYSINE ET EN THREONINE DE GRAINES DE PLANTES

(30) Priority: 19.03.1992 US 855414
(43) Date of publication of application: 01.03.1995
(73) Proprietor: E.I. DU PONT DE NEMOURS AND COMPANY, Wilmington Delaware 19898 (US)
(72) Inventor: FALCO, Saverio, Carl, Arden, DE 19810 (US)
(74) Representative: Dzieglewska, Hanna Eva
(86) International application number: US9302480
(87) International publication number: WO93019190

(56) References cited:
- EP-A- 0 435 132
- EP-A- 0 485 970
- WO-A-89/11789
- PLANT PHYSIOLOGY. vol. 98, no. 3, March 1992, ROCKVILLE, MD, USA. pages 1139 - 1147 BROCHETTA-BRAGA, M.R., ET AL. 'Partial purification and characterization of lysine-ketoglutarate reductase in normal and opaque-2 maize endosperms'
- THE PLANT JOURNAL vol. 2, no. 2, March 1992, pages 203 - 209 SHAUL, O., ET AL. 'Increased lysine synthesis in tobacco plants that express high levels of bacterial dihydropiconilate synthase in their chloroplasts'
- DATABASE WPIL Section Ch, Week 9214, Derwent Publications Ltd., London, GB; Class C06, AN 92-113940
- JOURNAL OF BIOLOGICAL CHEMISTRY vol. 261, no. 3, 25 January 1986, BALTIMORE, MD US pages 1052 - 1057 CASSAN, M., ET AL. 'Nucleotide sequence of lysC gene encoding the lysine-sensitive Aspartokinase III of Escherichia coli K12'

### SEQUENCE LISTING

(1) GENERAL INFORMATION:
   (i) APPLICANT: E. I. DU PONT DE NEMOURS AND COMPANY
   (ii) TITLE OF INVENTION: NUCLEIC ACID FRAGMENTS AND METHODS FOR INCREASING THE LYSINE AND THREONINE CONTENT OF THE SEEDS OF PLANTS
   (iii) NUMBER OF SEQUENCES: 24
   (iv) CORRESPONDENCE ADDRESS:
      (A) ADDRESSEE: E. I. DU PONT DE NEMOURS AND COMPANY
      (B) STREET: 1007 MARKET STREET
      (C) CITY: WILMINGTON
      (D) STATE: DELAWARE
      (E) COUNTRY: U.S.A.
      (F) ZIP: 19898
   (v) COMPUTER READABLE FORM:
      (A) MEDIUM TYPE: DISKETTE, 3.50 INCH
      (B) COMPUTER: MACINTOSH
      (C) OPERATING SYSTEM: MACINTOSH, 6.0
      (D) SOFTWARE: MICROSOFT WORD, 4.0
   (vi) CURRENT APPLICATION DATA:
      (A) APPLICATION NUMBER: BB-1037-A
      (B) FILING DATE:
      (C) CLASSIFICATION:
   (vii) PRIOR APPLICATION DATA:
      (A) APPLICATION NUMBER: 07/855,414
      (B) FILING DATE: 19 MARCH 1992
   (viii) ATTORNEY/AGENT INFORMATION:
      (A) NAME: LINDA AXAMETHY FLOYD
      (B) REGISTRATION NUMBER: 33,692
      (C) REFERENCE/DOCKET NUMBER: BB-1037-A
   (ix) TELECOMMUNICATION INFORMATION:
      (A) TELEPHONE: 302-992-4929
      (B) TELEFAX: 302-892-7949
      (C) TELEX: 835420
(2) INFORMATION FOR SEQ ID NO:1:
   (i) SEQUENCE CHARACTERISTICS:
      (A) LENGTH: 1350 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 1..1350
   (xi) SEQUENCE DESCRIPTION: SEQ ID NO:1:
   ATG GCT GAA ATT GTT GTC TCC AAA TTT GGC GGT ACC AGC GTA GCT GAT 48
   Met Ala Glu Ile Val Val Ser Lys Phe Gly Gly Thr Ser Val Ala Asp
   1 5 10 15
   TTT GAC GCC ATG AAC CGC AGC GCT GAT ATT GTG CTT TCT GAT GCC AAC 96
   Phe Asp Ala Met Asn Arg Ser Ala Asp Ile Val Leu Ser Asp Ala Asn
   20 25 30
   GTG CGT TTA GTT GTC CTC TCG GCT TCT GCT GGT ATC ACT AAT CTG CTG 144
   Val Arg Leu Val Val Leu Ser Ala Ser Ala Gly Ile Thr Asn Leu Leu
   35 40 45
   GTC GCT TTA GCT GAA GGA CTG GAA CCT GGC GAG CGA TTC GAA AAA CTC 192
   Val Ala Leu Ala Glu Gly Leu Glu Pro Gly Glu Arg Phe Glu Lys Leu
   50 55 60
   GAC GCT ATC CGC AAC ATC CAG TTT GCC ATT CTG GAA CGT CTG CGT TAC    240
   Asp Ala Ile Arg Asn Ile Gln Phe Ala Ile Leu Glu Arg Leu Arg Tyr
    65                  70                  75                  80
   CCG AAC GTT ATC CGT GAA GAG ATT GAA CGT CTG CTG GAG AAC ATT ACT    288
   Pro Asn Val Ile Arg Glu Glu Ile Glu Arg Leu Leu Glu Asn Ile Thr
                    85                  90                  95
   GTT CTG GCA GAA GCG GCG GCG CTG GCA ACG TCT CCG GCG CTG ACA GAT    336
   Val Leu Ala Glu Ala Ala Ala Leu Ala Thr Ser Pro Ala Leu Thr Asp
               100                 105                 110
   GAG CTG GTC AGC CAC GGC GAG CTG ATG TCG ACC CTG CTG TTT GTT GAG    384
   Glu Leu Val Ser His Gly Glu Leu Met Ser Thr Leu Leu Phe Val Glu
           115                 120                 125
   ATC CTG CGC GAA CGC GAT GTT CAG GCA CAG TGG TTT GAT GTA CGT AAA    432
   Ile Leu Arg Glu Arg Asp Val Gln Ala Gln Trp Phe Asp Val Arg Lys
       130                 135                 140
   GTG ATG CGT ACC AAC GAC CGA TTT GGT CGT GCA GAG CCA GAT ATA GCC    480
   Val Met Arg Thr Asn Asp Arg Phe Gly Arg Ala Glu Pro Asp Ile Ala
   145                 150                 155                 160
   GCG CTG GCG GAA CTG GCC GCG CTG CAG CTG CTC CCA CGT CTC AAT GAA    528
   Ala Leu Ala Glu Leu Ala Ala Leu Gln Leu Leu Pro Arg Leu Asn Glu
                   165                 170                 175
   GGC TTA GTG ATC ACC CAG GGA TTT ATC GGT AGC GAA AAT AAA GGT CGT    576
   Gly Leu Val Ile Thr Gln Gly Phe Ile Gly Ser Glu Asn Lys Gly Arg
               180                 185                 190
   ACA ACG ACG CTT GGC CGT GGA GGC AGC GAT TAT ACG GCA GCC TTG CTG    624
   Thr Thr Thr Leu Gly Arg Gly Gly Ser Asp Tyr Thr Ala Ala Leu Leu
           195                 200                 205
   GCG GAG GCT TTA CAC GCA TCT CGT GTT GAT ATC TGG ACC GAC GTC CCG    672
   Ala Glu Ala Leu His Ala Ser Arg Val Asp Ile Trp Thr Asp Val Pro
       210                 215                 220
   GGC ATC TAC ACC ACC GAT CCA CGC GTA GTT TCC GCA GCA AAA CGC ATT    720
   Gly Ile Tyr Thr Thr Asp Pro Arg Val Val Ser Ala Ala Lys Arg Ile
   225                 230                 235                 240
   GAT GAA ATC GCG TTT GCC GAA GCG GCA GAG ATG GCA ACT TTT GGT GCA    768
   Asp Glu Ile Ala Phe Ala Glu Ala Ala Glu Met Ala Thr Phe Gly Ala
                   245                 250                 255
   AAA GTA CTG CAT CCG GCA ACG TTG CTA CCC GCA GTA CGC AGC GAT ATC    816
   Lys Val Leu His Pro Ala Thr Leu Leu Pro Ala Val Arg Ser Asp Ile
               260                 265                 270
   CCG GTC TTT GTC GGC TCC AGC AAA GAC CCA CGC GCA GGT GGT ACG CTG    864
   Pro Val Phe Val Gly Ser Ser Lys Asp Pro Arg Ala Gly Gly Thr Leu
           275                 280                 285
   GTG TGC AAT AAA ACT GAA AAT CCG CCG CTG TTC CGC GCT CTG GCG CTT    912
   Val Cys Asn Lys Thr Glu Asn Pro Pro Leu Phe Arg Ala Leu Ala Leu
       290                 295                 300
   CGT CGC AAT CAG ACT CTG CTC ACT TTG CAC AGC CTG AAT ATG CTG CAT    960
   Arg Arg Asn Gln Thr Leu Leu Thr Leu His Ser Leu Asn Met Leu His
   305                 310                 315                 320
   TCT CGC GGT TTC CTC GCG GAA GTT TTC GGC ATC CTC GCG CGG CAT AAT   1008
   Ser Arg Gly Phe Leu Ala Glu Val Phe Gly Ile Leu Ala Arg His Asn
                   325                 330                 335
   ATT TCG GTA GAC TTA ATC ACC ACG TCA GAA GTG AGC GTG GCA TTA ACC  1056
   Ile Ser Val Asp Leu Ile Thr Thr Ser Glu Val Ser Val Ala Leu Thr
                   340                 345                 350
   CTT GAT ACC ACC GGT TCA ACC TCC ACT GGC GAT ACG TTG CTG ACG CAA  1104
   Leu Asp Thr Thr Gly Ser Thr Ser Thr Gly Asp Thr Leu Leu Thr Gln
               355                 360                 365
   TCT CTG CTG ATG GAG CTT TCC GCA CTG TGT CGG GTG GAG GTG GAA GAA  1152
   Ser Leu Leu Met Glu Leu Ser Ala Leu Cys Arg Val Glu Val Glu Glu
           370                 375                 380
   GGT CTG GCG CTG GTC GCG TTG ATT GGC AAT GAC CTG TCA AAA GCC TGC  1200
   Gly Leu Ala Leu Val Ala Leu Ile Gly Asn Asp Leu Ser Lys Ala Cys
   385                 390                 395                 400
   GCC GTT GGC AAA GAG GTA TTC GGC GTA CTG GAA CCG TTC AAC ATT CGC  1248
   Ala Val Gly Lys Glu Val Phe Gly Val Leu Glu Pro Phe Asn Ile Arg
                   405                 410                 415
   ATG ATT TGT TAT GGC GCA TCC AGC CAT AAC CTG TGC TTC CTG GTG CCC  1296
   Met Ile Cys Tyr Gly Ala Ser Ser His Asn Leu Cys Phe Leu Val Pro
               420                 425                 430
   GGC GAA GAT GCC GAG CAG GTG GTG CAA AAA CTG CAT AGT AAT TTG TTT  1344
   Gly Glu Asp Ala Glu Gln Val Val Gln Lys Leu His Ser Asn Leu Phe
           435                 440                 445
   GAG TAA                                                          1350
   Glu  *
       450
(2) INFORMATION FOR SEQ ID NO:2:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:2:
   GATCCATGGC TGAAATTGTT GTCTCCAAAT TTGGCG 36
(2) INFORMATION FOR SEQ ID NO:3:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:3:
   GTACCGCCAA ATTTGGAGAC AACAATTTCA GCCATG 36
(2) INFORMATION FOR SEQ ID NO:4:
   (i) SEQUENCE CHARACTERISTICS:
      (A) LENGTH: 48 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:4:
   CCCGGGCCAT GGCTACAGGT TTAACAGCTA AGACCGGAGT AGAGCACT 48
(2) INFORMATION FOR SEQ ID NO:5:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:5:
   GATATCGAAT TCTCATTATA GAACTCCAGC TTTTTTC 37
(2) INFORMATION FOR SEQ ID NO:6:
   (i) SEQUENCE CHARACTERISTICS:
      (A) LENGTH: 917 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 3..911
   (xi) SEQUENCE DESCRIPTION: SEQ ID NO:6:
(2) INFORMATION FOR SEQ ID NO:7:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:7:
   CTTCCCGTGA CCATGGGCCA TC                                          22
(2) INFORMATION FOR SEQ ID NO:8:
   (i) SEQUENCE CHARACTERISTICS:
      (A) LENGTH: 75 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:8:
   CATGGCTGGC TTCCCCACGA GGAAGACCAA CAATGACATT ACCTCCATTG CTAGCAACGG   60
   TGGAAGAGTA CAATG                                                    75
(2) INFORMATION FOR SEQ ID NO:9:
   (i) SEQUENCE CHARACTERISTICS:
      (A) LENGTH: 75 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:9:
   CATGCATTGT ACTCTTCCAC CGTTGCTAGC AATGGAGGTA ATGTCATTGT TGGTCTTCCT   60
   CGTGGGGAAG CCAGC                                                    75
(2) INFORMATION FOR SEQ ID NO:10:
   (i) SEQUENCE CHARACTERISTICS:
      (A) LENGTH: 90 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:10:
   CATGGCTTCC TCAATGATCT CCTCCCCAGC TGTTACCACC GTCAACCGTG CCGGTGCCGG   60
   CATGGTTGCT CCATTCACCG GCCTCAAAAG                                    90
(2) INFORMATION FOR SEQ ID NO:11:
   (i) SEQUENCE CHARACTERISTICS:
      (A) LENGTH: 90 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:11:
   CATGCTTTTG AGGCCGGTGA ATGGAGCAAC CATGCCGGCA CCGGCACGGT TGACGGTGGT 60
   AACAGCTGGG GAGGAGATCA TTGAGGAAGC 90
(2) INFORMATION FOR SEQ ID NO:12:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:12:
   CCGGTTTGCT GTAATAGGTA CCA 23
(2) INFORMATION FOR SEQ ID NO:13:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:13:
   AGCTTGGTAC CTATTACAGC AAACCGGCAT G 31
(2) INFORMATION FOR SEQ ID NO:14:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:14:
   GCTTCCTCAA TGATCTCCTC CCCAGCT 27
(2) INFORMATION FOR SEQ ID NO:15:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:15:
   CATTGTACTC TTCCACCGTT GCTAGCAA 28
(2) INFORMATION FOR SEQ ID NO:16:
   (i) SEQUENCE CHARACTERISTICS:
      (A) LENGTH: 839 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:16:
   GGATCCCCCG GGCTGCAGGA ATTCTACGTA CCATATAGTA AGACTTTGTA TATAAGACGT    60
   CACCTCTTAC GTGCATGGTT ATATGTGACA TGTGCAGTGA CGTTGTACCA TATAGTAAGA   120
   CTTTGTATAT AAGACGTCAC CTCTTACGTG CATGGTTATA TGTGACATGT GCAGTGACGT   180
   TAACCGCACC CTCCTTCCCG TCGTTTCCCA TCTCTTCCTC CTTTAGAGCT ACCACTATAT   240
   AAATCAGGGC TCATTTTCTC GCTCCTCACA GGCTCATCAG CACCCCGGCA GTGCCACCCC   300
   GACTCCCTGC ACCTGCCATG GGTACGCTAG CCCGGGAGAT CTGACAAAGC AGCATTAGTC   360
   CGTTGATCGG TGGAAGACCA CTCGTCAGTG TTGAGTTGAA TGTTTGATCA ATAAAATACG   420
   GCAATGCTGT AAGGGTTGTT TTTTATGCCA TTGATAATAC ACTGTACTGT TCAGTTGTTG   480
   AACTCTATTT CTTAGCCATG CCAGTGCTTT TCTTATTTTG AATAACATTA CAGCAAAAAG   540
   TTGAAAGACA AAAAAANNNN NCCCCGAACA GAGTGCTTTG GGTCCCAAGC TTCTTTAGAC   600
   TGTGTTCGGC GTTCCCCCTA AATTTCTCCC CTATATCTCA CTCACTTGTC ACATCAGCGT   660
   TCTCTTTCCC CTATATCTCC ACGCTCTACA GCAGTTCCAC CTATATCAAA CCTCTATACC   720
   CCACCACAAC AATATTATAT ACTTTCATCT TCACCTAACT CATGTACCTT CCAATTTTTT   780
   TCTACTAATA ATTATTTACG TGCACAGAAA CTTAGGCAAG GGAGAGAGAG AGCGGTACC    839
(2) INFORMATION FOR SEQ ID NO:17:
   (i) SEQUENCE CHARACTERISTICS:
      (A) LENGTH: 43 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:17:
   CTAGAAGCCT CGGCAACGTC AGCAACGGCG GAAGAATCCG GTG 43
(2) INFORMATION FOR SEQ ID NO:18:
   (i) SEQUENCE CHARACTERISTICS:
      (A) LENGTH: 43 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:18:
   CATGCACCGG ATTCTTCCGC CGTTGCTGAC GTTGCCGAGG CTT 43
(2) INFORMATION FOR SEQ ID NO:19:
   (i) SEQUENCE CHARACTERISTICS:
      (A) LENGTH: 55 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:19:
   GATCCCATGG CGCCCCTTAA GTCCACCGCC AGCCTCCCCG TCGCCCGCCG CTCCT 55
(2) INFORMATION FOR SEQ ID NO:20:
   (i) SEQUENCE CHARACTERISTICS:
      (A) LENGTH: 55 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:20:
   CTAGAGGAGC GGCGGGCGAC GGGGAGGCTG GCGGTGGACT TAAGGGGCGC CATGG 55
(2) INFORMATION FOR SEQ ID NO:21:
   (i) SEQUENCE CHARACTERISTICS:
      (A) LENGTH: 59 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:21:
   CATGGCGCCC ACCGTGATGA TGGCCTCGTC GGCCACCGCC GTCGCTCCGT TCCAGGGGC 59
(2) INFORMATION FOR SEQ ID NO:22:
   (i) SEQUENCE CHARACTERISTICS:
      (A) LENGTH: 59 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:22:
   TTAAGCCCCT GGAACGGAGC GACGGCGGTG GCCGACGAGG CCATCATCAC GGTGGGCGC 59
(2) INFORMATION FOR SEQ ID NO:23:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:23:
   GCGCCCACCG TGATGA 16
(2) INFORMATION FOR SEQ ID NO:24:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:24:
   CACCGGATTC TTCCGC 16

## Description

### TECHNICAL FIELD

This invention relates to a unique synthetic nucleic acid subfragment encoding aspartokinase (AK) which is insensitive to inhibition by lysine. Methods for its use to produce increased levels of lysine or threonine in transformed organisms are also provided.

### BACKGROUND OF THE INVENTION

Human food and animal feed derived from many grains are deficient in some of the ten essential amino acids which are required in the animal diet. In corn (Zea mays L.), lysine is the most limiting amino acid for the dietary requirements of many animals. Soybean (Glycine max L.) meal is used as an additive to corn based animal feeds primarily as a lysine supplement. Thus an increase in the lysine content of either corn or soybean would reduce or eliminate the need to supplement mixed grain feeds with lysine produced via fermentation of microbes.

Plant breeders have long been interested in using naturally occuring variations to improve protein quality and quantity in crop plants. Maize lines containing higher than normal levels of lysine (70%) have been identified (Mertz, Science, 145, 279 (1964) and Nelson, Science, 150, 1469-70 (1965)). However, these lines which incorporate a mutant gene, opaque-2, exhibit poor agronomic qualities (increased susceptibility to disease and pests, 8-14% reduction in yield, low kernel weight, slower drying, lower dry milling yield of flaking grits, and increased storage problems) and thus are not commercially useful [Deutscher, D. Adv. Exp. Medicine and Biology, 105, 281-300 (1978)]. Quality Protein Maize (QPM) bred at CIMMYT using the opaque-2 and sugary-2 genes and associated modifiers has a hard endosperm and enriched levels of lysine and tryptophan in the kernels [Vasal, S. K., et al. Proceedings of the 3rd seed protein symposium, Gatersleben, August 31 - September 2, 1983]. However, the gene pools represented in the QPM lines are tropical and subtropical. Quality Protein Maize is a genetically complex trait and the existing lines are not easily adapted to the dent germplasm in use in the United States, preventing the adoption of QPM by corn breeders.

The amino acid content of seeds is determined primarily (90-99%) by the amino acid composition of the proteins in the seed and to a lesser extent (1-10%) by the free amino acid pools. The quantity of total protein in seeds varies from about 10% of the dry weight in cereals to 20-40% of the dry weight of legumes. Much of the protein-bound amino acids is contained in the seed storage proteins which are synthesized during seed development and which serve as a major nutrient reserve following germination. In many seeds the storage proteins account for 50% or more of the total protein.

To improve the amino acid composition of seeds genetic engineering technology is being used to isolate, and express genes for storage proteins in transgenic plants. For example, a gene from Brazil nut for a seed 2S albumin composed of 26% sulfur-containing amino acids has been isolated [Altenbach et al. (1987) Plant Mol. Biol. 8:239-250] and expressed in the seeds of transformed tobacco under the control of the regulatory sequences from a bean phaseolin storage protein gene. The accumulation of the sulfur-rich protein in the tobacco seeds resulted in an up to 30% increase in the level of methionine in the seeds [Altenbach et al. (1989) Plant Mol. Biol. 13:513-522]. However, no plant seed storage proteins similarly enriched in lysine relative to average lysine content of plant proteins have been identified to date, preventing this approach from being used to increase lysine.

Lysine, along with threonine, methionine and isoleucine, are amino acids derived from aspartate, and regulation of the biosynthesis of each member of this family is interconnected. Regulation of the metabolic flow in the pathway appears to be primarily via end products. The first step in the pathway is the phosphorylation of aspartate by the enzyme aspartokinase (AK), and this enzyme has been found to be an important target for regulation in many organisms. However, detailed physiological studies on the flux of 4-carbon molecules through the aspartate pathway have been carried out in the model plant system Lemna paucicostata [Giovanelli et al. (1989) Plant Physiol. 90:1584-1599]. The authors state "These data now provide definitive evidence that the step catalyzed by aspartokinase is not normally an important site for regulation of the entry of 4-carbon units into the aspartate family of amino acids [in plants]."

The aspartate family pathway is also regulated at the branch-point reactions. For lysine this is the condensation of aspartyl β-semialdehyde with pyruvate catalyzed by dihydrodipicolinic acid synthase (DHDPS), while for threonine and methionine the reduction of aspartyl β-semialdehyde by homoserine dehydrogenase (HDH) followed by the phosphorylation of homoserine by homoserine kinase (HK) are important points of control.

Many attempts have been made to isolate lysine over-producing mutants of plants by selecting for resistance to the lysine analog S(2-aminoethyl)-cysteine (AEC), either alone or in conjunction with lysine plus threonine resistance selections. No examples of increases in seed lysine levels that would be commercially valuable have yet been reported, however.

An alternative approach is to increase the production and accumulation of specific free amino acids such as lysine via genetic engineering technology. A number of genes for key regulatory enzymes of the aspartate family pathway have been isolated from various sources. The E. coli dapA gene encodes a DHDPS enzyme that is about 20-fold less sensitive to inhibition by lysine than wheat germ DHDPS. The E. coli dapA gene has been linked to plant gene expression sequences, introduced into tobacco cells via transformation and shown to cause a substantial increase in free lysine levels in leaves [Glassman et al. (1989) PCT Patent Appl. PCT/US89/01309]. However, no evidence that increased lysine was found in seeds was presented (even though seeds were available to test). The E. coli dapA gene, linked to plant gene expression sequences, has also been introduced into tobacco cells by Galili et al. [(1991) Abstr. 422 from Third Int. Cong. of Int. Soc. Plant Mol. Biol.] and by Shaul et al. [(1992) Plant Jour. 2:203-209] and shown to result in an increase in free lysine levels in leaves. Again, no evidence for increased levels of free lysine in seeds was presented. These workers have recently reported on the introduction of an E. coli lysC gene that encodes a lysine-insensitive AK enzyme into tobacco cells via transformation [Galili et al. (1992) Eur. Patent Appl. 91119328.2; Shaul et al. (1992) Plant Physiol. 100:1157-1163]. Expression of the E. coli enzyme results in increases in the levels of free threonine in the leaves and seeds of transformed plants. Crosses of plants expressing E. coli DHDPS and AK resulted in progeny that accumulated more free lysine in leaves than the parental DHDPS plant, but less free threonine in leaves than the parental AK plant. No evidence for increased levels of free lysine in seeds was presented.

The limited understanding of the details of the regulation of the biosynthetic pathway in plants makes the application of genetic engineering technology, particularly to seeds, uncertain. There is little information available on the source of the aspartate-derived amino acids in seeds. It is not known, for example, whether they are synthesized in seeds, or transported to the seeds from leaves, or both, from most plants. In addition, free amino acids make up only a small fraction of the total amino acid content of seeds. Therefore, over-accumulation of free amino acids must be many-fold in order to significantly affect the total amino acid composition of the seeds. Furthermore, little is known about catabolism of free amino acids in seeds. Catabolism of free lysine has been observed in developing endosperm of corn and barley. The first step in the catabolism of lysine is believed to be catalyzed by lysine-ketoglutarate reductase [Brochetto-Braga et al. (1992) Plant Physiol. 98:1139-1147]. Whether such catabolic pathways are widespread in plants and whether they affect the level of accumulation of free amino acids is unknown.

Before this application no method to increase the level of lysine, or any other amino acid, in seeds via genetic engineering was known. Thus, there is a need for genes, chimeric genes, and methods for expressing them in seeds so that an over-accumulation of free amino acids in seeds will result in an improvement in nutritional quality.

### SUMMARY OF THE INVENTION

Applicant's invention is an isolated nucleic acid fragment comprising (a) a first nucleic acid subfragment encoding AK which is insensitive to inhibition by lysine and (b) a second nucleic acid subfragment encoding dihydrodipicolinic acid synthase which is at least 20-fold less sensitive to inhibition by lysine than plant DHDPS and (c) a third nucleic acid subfragment encoding antisense lysine ketoglutarate reductase. In a further embodiment, the first and second subfragments are operably linked to a suitable plant chloroplast transit sequence and each subfragment is also linked to a suitable regulatory sequence to promote expression in plants. A preferred embodiment is either of the inventions described above in which:
(a) the first nucleic acid subfragment comprises a nucleotide sequence substantially homologous to the sequence shown in SEQ ID NO:1 encoding a lysine-insensitive variant of E. coli AKIII and further characterized in that at least one of the following conditions is met:
   (1) the amino acid at position 318 is an amino acid other than methionine, or
   (2) the amino acid at position 352 is an amino acid other than threonine; or
(b) the second nucleic acid subfragment is derived from bacteria.

A further embodiment is the invention in which:
(a) the first nucleic acid subfragment is further characterized in that at least one of the following conditions is met:
   (1) the amino acid at position 318 is isoleucine, or
   (2) the amino acid at position 352 is isoleucine; or
(b) the second nucleic acid subfragment is derived from bacteria, preferably either E. coli or Corynebacterium glutamicum.

The subject matter also includes an isolated nucleic acid fragment comprising a nucleic acid subfragment encoding lysine ketoglutarate reductase.

Plants, seeds and microorganisms transformed with the nucleic acid fragments described are also embodiments of the invention. Further embodiments of the invention are methods for increasing the threonine or lysine content of seeds by transforming plants with the unique nucleic acid fragments described.

### BRIEF DESCRIPTION OF THE DRAWINGS AND SEQUENCE DESCRIPTIONS

The invention can be more fully understood from the following detailed description and the accompanying drawings and the sequence descriptions which form a part of this application.

Figure 1 shows a schematic representation of gene expression cassettes.

Figure 2 shows a map of the binary plasmid vector pZS97K.

Figure 3 shows a map of the binary plasmid vector pZS97.

Figure 4 shows a map of the binary plasmid vector PZS199.

Figure 5 shows a map of the plasmid vector pBT603.

SEQ ID NO:1 shows the nucleotide and amino acid sequence of the coding region of the wild type E. coli lysC gene, which encodes AKIII, described in Example 1.

SEQ ID NOS: 2 and 3 were used in Example 2 to create an Nco I site at the translation start codon of the E. coli lysC gene.

SEQ ID NOS:4 and 5 were used in Example 3 as PCR primers for the isolation of the Corynebacterium dapA gene.

SEQ ID NO:6 shows the nucleotide and amino acid sequence of the coding region of the wild type Corynebacterium dapA gene, which encodes lysine-insensitive DHDPS, described in Example 3.

SEQ ID NO:7 was used in Example 4 to create an Nco I site at the translation start codon of the E. coli dapA gene.

SEQ ID NOS:8, 9, 10 and 11 were used in Example 6 to create a chloroplast transit sequence and link the sequence to the E. coli lysC, E. coli lysC-M4, E. coli dapA and Corynebacteria dapA genes.

SEQ ID NOS:12 and 13 were used in Example 6 to create a Kpn I site immediately following the translation stop codon of the E. coli dapA gene.

SEQ ID NOS:14 and 15 were used in Example 6 as PCR primers to create a chloroplast transit sequence and link the sequence to the Corynebacterium dapA gene.

SEQ ID NO:16 was used in Example 6 as a constitutive expression cassette for corn.

SEQ ID NOS:17-22 were used in Example 6 to create a corn chloroplast transit sequence and link the sequence to the E. coli lysC-M4 gene.

SEQ ID NOS:23 and 24 were used in Example 6 as PCR primers to create a corn chloroplast transit sequence and link the sequence to the E. coli dapA gene.

The Sequence Descriptions contain the one letter code for nucleotide sequence characters and the three letter codes for amino acids as defined in conformity with the IUPAC-IYUB standards described in Nucleic Acids Research 13:3021-3030(1985) and in the Biochemical Journal 219 (No. 2):345-373(1984) which are incorporated by reference herein.

### DETAILED DESCRIPTION OF THE INVENTION

In order to increase the accumulation of specific free amino acids in the seeds of plants via genetic engineering, Applicant determined which enyzmes in this pathway controlled the pathway. In order to accomplish this, genes encoding enzymes in the pathway were isolated from bacteria. In some cases, mutations in the genes were obtained so that the enzyme encoded was made insensitive to end-product inhibition. Because the genes were not of plant origin, they had to be linked to intracellular localization sequences and suitable regulatory sequences for expression in plants. The genes, individually or in combinations, were then introduced into plants via transformation and assessed for their ability to elicit accumulation of the desired amino acid(s) in the desired plant organ(s).

The teachings below describe nucleic acid fragments and procedures useful for increasing the accumulation of lysine and threonine in the seeds of transformed plants as compared to levels of the amino acids of untransformed plants. Specifically, Applicant has provided a unique first nucleic acid fragment comprised of two nucleic acid subfragments, one encoding AK which is insensitive to inhibition by lysine and the other encoding DHDPS which is at least 20-fold less sensitive to feedback inhibition by lysine than a typical plant DHDPS, e.g., wheat DHDPS. It is the combination of subfragments which Applicant found successfully increases the lysine accumulated in seeds of transformed plants as compared to untransformed host plants. To achieve a greater accumulation of lysine in seeds of transformed plants, Applicant has also provided a nucleic acid fragment containing an antisense Lysine Ketoglutarate Reductase (LKR) chimeric gene. This fragment can be linked to the first fragment or combined via crossing of plants transformed with the first fragment with plants transformed with the nucleic acid fragment containing an antisense LKR chimeric gene.

In the context of this disclosure, a number of terms shall be utilized. As used herein, the term "nucleic acid" refers to a large molecule which can be single-stranded or double-stranded, composed of monomers (nucleotides) containing a sugar, phosphate and either a purine or pyrimidine. A "nucleic acid fragment" is a fraction of a given nucleic acid molecule. In higher plants, deoxyribonucleic acid (DNA) is the genetic material while ribonucleic acid (RNA) is involved in the transfer of the information in DNA into proteins. A "genome" is the entire body of genetic material contained in each cell of an organism. The term "nucleotide sequence" refers to a polymer of DNA or RNA which can be single- or double-stranded, optionally containing synthetic, non-natural or altered nucleotide bases capable of incorporation into DNA or RNA polymers.

As used herein, the term "homologous to" refers to the complementarity between the nucleotide sequence of two nucleic acid molecules or between the amino acid sequences of two protein molecules. Estimates of such homology are provided by either DNA-DNA or DNA-RNA hybridization under conditions of stringency as is well understood by those skilled in the art [as described in Hames and Higgins (eds.) Nucleic Acid Hybridisation, IRL Press, Oxford, U.K.]; or by the comparison of sequence similarity between two nucleic acids or proteins.

As used herein, "substantially homologous" refers to nucleic acid molecules which require less stringent conditions of hybridization than those for homologous sequences, and also refers to coding DNA sequence which may involve base changes that do not cause a change in the encoded amino acid, or which involve base changes which may alter one or more amino acids, but not affect the functional properties of the protein encoded by the DNA sequence. Thus, the nucleic acid fragments described herein include molecules which comprise possible variations of the nucleotide bases derived from deletion, rearrangement, random or controlled mutagenesis of the nucleic acid fragment, and even occasional nucleotide sequencing errors so long as the DNA sequences are substantially homologous.

"Gene" refers to a nucleic acid fragment that expresses a specific protein, including regulatory sequences preceding (5' non-coding) and following (3' non-coding) the coding region. "Native" gene refers to the gene as found in nature with its own regulatory sequences. "Chimeric" gene refers to a gene comprising heterogeneous regulatory and coding sequences. "Endogenous" gene refers to the native gene normally found in its natural location in the genome. A "foreign" gene refers to a gene not normally found in the host organism but that is introduced by gene transfer.

"Coding sequence" refers to a DNA sequence that codes for a specific protein and excludes the non-coding sequences.

"Initiation codon" and "termination codon" refer to a unit of three adjacent nucleotides in a coding sequence that specifies initiation and chain termination, respectively, of protein synthesis (mRNA translation). "Open reading frame" refers to the amino acid sequence encoded between translation initiation and termination codons of a coding sequence.

"RNA transcript" refers to the product resulting from RNA polymerase-catalyzed transcription of a DNA sequence. When the RNA transcript is a perfect complementary copy of the DNA sequence, it is referred to as the primary transcript or it may be a RNA sequence derived from posttranscriptional processing of the primary transcript. "Messenger RNA (mRNA) refers to RNA that can be translated into protein by the cell. "cDNA" refers to a double-stranded DNA that is complementary to and derived from mRNA. "Sense" RNA refers to RNA transcript that includes the mRNA. "Antisense RNA" refers to a RNA transcript that is complementary to all or part of a target primary transcript or mRNA and that blocks the expression of a target gene by interfering with the processing, transport and/or translation of its primary transcript or mRNA. The complementarity of an antisense RNA may be with any part of the specific gene transcript, i.e., at the 5' non-coding sequence, 3' non-coding sequence, introns, or the coding sequence. In addition, as used herein, antisense RNA may contain regions of ribozyme sequences that increase the efficacy of antisense RNA to block gene expression. "Ribozyme" refers to a catalytic RNA and includes sequence-specific endoribonucleases.

As used herein, suitable "regulatory sequences" refer to nucleotide sequences located upstream (5'), within, and/or downstream (3') to a coding sequence, which control the transcription and/or expression of the coding sequences, potentially in conjunction with the protein biosynthetic apparatus of the cell. These regulatory sequences include promoters, translation leader sequences, transcription termination sequences, and polyadenylation sequences.

"Promoter" refers to a DNA sequence in a gene, usually upstream (5') to its coding sequence, which controls the expression of the coding sequence by providing the recognition for RNA polymerase and other factors required for proper transcription. A promoter may also contain DNA sequences that are involved in the binding of protein factors which control the effectiveness of transcription initiation in response to physiological or developmental conditions. It may also contain enhancer elements.

An "enhancer" is a DNA sequence which can stimulate promoter activity. It may be an innate element of the promoter or a heterologous element inserted to enhance the level and/or tissue-specificity of a promoter. "Constitutive promoters" refers to those that direct gene expression in all tissues and at all times. "Organ-specific" or "development-specific" promoters as referred to herein are those that direct gene expression almost exclusively in specific organs, such as leaves or seeds, or at specific development stages in an organ, such as in early or late embryogenesis, respectively.

The term "operably linked" refers to nucleic acid sequences on a single nucleic acid molecule which are associated so that the function of one is affected by the other. For example, a promoter is operably linked with a structure gene (i.e., a gene encoding aspartokinase that is lysine-insensitive as given herein) when it is capable of affecting the expression of that structural gene (i.e., that the structural gene is under the transcriptional control of the promoter).

The term "expression", as used herein, is intended to mean the production of the protein product encoded by a gene. More particularly, "expression" refers to the transcription and stable accumulation of the sense (mRNA) or tha antisense RNA derived from the nucleic acid fragment(s) of the invention that, in conjuction with the protein apparatus of the cell, results in altered levels of protein product. "Antisense inhibition" refers to the production of antisense RNA transcripts capable of preventing the expression of the target protein. "Overexpression" refers to the production of a gene product in transgenic organisms that exceeds levels of production in normal or non-transformed organisms. "Cosuppression" refers to the expression of a foreign gene which has substantial homology to an endogenous gene resulting in the suppression of expression of both the foreign and the endogenous gene. "Altered levels" refers to the production of gene product(s) in transgenic organisms in amounts or proportions that differ from that of normal or non-transformed organisms.

The "3' non-coding sequences" refers to the DNA sequence portion of a gene that contains a polyadenylation signal and any other regulatory signal capable of affecting mRNA processing or gene expression. The polyadenylation signal is usually characterized by affecting the addition of polyadenylic acid tracts to the 3' end of the mRNA precursor.

The "translation leader sequence" refers to that DNA sequence portion of a gene between the promoter and coding sequence that is transcribed into RNA and is present in the fully processed mRNA upstream (5') of the translation start codon. The translation leader sequence may affect processing of the primary transcript to mRNA, mRNA stability or translation efficiency.

"Mature" protein refers to a post-translationally processed polypeptide without its targeting signal. "Precursor" protein refers to the primary product of translation of mRNA. A "chloroplast targeting signal" is an amino acid sequence which is translated in conjunction with a protein and directs it to the chloroplast. "Chloroplast transit sequence" refers to a nucleotide sequence that encodes a chloroplast targeting signal.

"Transformation" herein refers to the transfer of a foreign gene into the genome of a host organism and its genetically stable inheritance. Examples of methods of plant transformation include Agrobacterium-mediated transformation and particle-accelerated or "gene gun" transformation technology.

### Isolation of AK Genes

The E. coli lysC gene has been cloned, restriction endonuclease mapped and sequenced previously [Cassan et al. (1986) J. Biol. Chem. 261:1052-1057]. For the present invention the lysC gene was obtained on a bacteriphage lambda clone from an ordered library of 3400 overlapping segments of cloned E. coli DNA constructed by Kohara, Akiyama and Isono [Kohara et al. (1987) Cell 50:595-508]. The E. coil lysC gene encodes the enzyme AKIII, which is sensitive to lysine inhibition. Mutations were obtained in the lysC gene that cause the AKIII enzyme to be resistant to lysine.

To determine the molecular basis for lysine-resistance,the sequence of the wild type lysC gene and three mutant genes were determined. The sequence of the cloned wild type lysC gene, indicated in SEQ ID NO:1:, differed from the published lysC sequence in the coding region at 5 positions.

The sequences of the three mutant lysC genes that encoded lysine-insensitive aspartokinase each differed from the wild type sequnce by a single nucleotide, resulting in a single amino acid substitution in the protein. One mutant (M2) had an A substituted for a G at nucleotide 954 of SEQ ID NO:1: resulting in an isoleucine for methionine substitution in the amino acid sequence of AKIII and two mutants (M3 and M4) had identical T for C substitutions at nucleotide 1055 of SEQ ID NO:1 resulting in an isoleucine for threonine substitution.

Other mutations could be generated, either in vivo as described in Example 1 or in vitro by site-directed mutagenesis by methods known to those skilled in the art, that result in amino acid substitutions for the methionine or threonine residue present in the wild type AKIII at these positions. Such mutations would be expected to result in a lysine-insensitive enzyme. Furthermore, the method described in Example 1 could be used to easily isolate and characterize as many additional mutant lysC genes encoding lysine insensitive AKIII as desired.

A number of other AK genes have been isolated and sequenced. These include the thrA gene of E. coli (Katinka et al. (1980) Proc. Natl. Acad. Sci. USA 77:5730-5733], the metL gene of E. coli (Zakin et al. (1983) J. Biol. Chem. 258:3028-3031], the HOM3 gene of S. cerevisiae [Rafalski et al. (1988) J. Biol. Chem. 263:2146-2151]. The thrA gene of E. coli encodes a bifunctional protein, AKI-HDHI. The AK activity of this enzyme is insensitive to lysine, but sensitive to threonine. The metL gene of E. coli also encodes a bifunctional protein, AKII-HDHII, and the AK activity of this enzyme is also insensitive to lysine. The HOM3 gene of yeast encodes an AK which is insensitive to lysine, but sensitive to threonine.

In addition to these genes, several plant genes encoding lysine-insensitive AK are known. In barley lysine plus threonine-resistant mutants bearing mutations in two unlinked genes that result in two different lysine-insensitive AK isoenzymes have been described [Bright et al. (1982) Nature 299:278-279, Rognes et al. (1983) Planta 157:32-38, Arruda et al. (1984) Plant Phsiol. 76:442-446]. In corn, a lysine plus threonine-resistant cell line had AK activity that was less sensitive to lysine inhibition than its parent line [Hibberd et al. (1980) Planta 148:183-187]. A subsequently isolated lysine plus threonine-resistant corn mutant is altered at a different genetic locus and also produces lysine-insensitive AK [Diedrick et al. (1990) Theor. Appl. Genet. 79:209-215, Dotson et al. (1990) Planta 182:546-552]. In tobacco there are two AK enzymes in leaves, one lysine-sensitive and one threonine-sensitive. A lysine plus threonine-resistant tobacco mutant that expressed completely lysine-insensitive AK has been described [Frankard et al. (1991) Theor. Appl. Genet. 82:273-282]. These plant mutants could serve as sources of genes encoding lysine-insensitive AK and used, based on the teachings herein, to increase the accumulation of lysine and threonine in the seeds of transformed plants.

A partial amino acid sequence of AK from carrot has been reported [Wilson et al. (1991) Plant Physiol. 97:1323:1328]. Using this information a set of degenerate DNA oligonucleotides could be designed, synthesized and used as hybridization probes to permit the isolation of the carrot AK gene. Recently the carrot AK gene has been isolated and its nucleotide sequence has been determined [Matthews et al. (1991) U.S.S.N. 07/746,705]. This gene can be used as a heterologous hybridization probe to isolate the genes encoding lysine-insensitive AK described above.

### High level expression of wild type and mutant lysC genes in E. coli

To achieve high level expression of the lysC genes in E. coli, a bacterial expression vector which employs the bacteriphage T7 RNA polymerase/T7 promoter system [Rosenberg et al. (1987) Gene 56:125-135] was used. The expression vector and lysC gene were modified as described in Example 2 to construct a lysC expression vector. For expression of the mutant lysC genes (M2, M3 and M4), the wild type lysC gene was replaced with the mutant genes as described in Example 2.

For high level expression, each of the expression vectors was transformed into E. coli strain B121(DE3) [Studier et al. (1986) J. Mol. Biol. 189:113-130]. Cultures were grown, expression was induced, cells were collected, and extracts were prepared as described in Example 2. Supernatant and pellet fractions of extracts from uninduced and induces cultures were analyzed by SDS polyacrylamide gel electrophoresis and by AK enzyme assays as described in Example 2. The major protein visible by Coomassie blue staining in the supernatant and pellet fractions of induced cultures was AKIII. About 80% of the AKIII protein was in the supernatant and AKIII represented 10-20% of the total E. coli protein in the extract.

Approximately 80% of the AKIII enzyme activity was in the supernatant fraction. The specific activity of wild type and mutant crude extracts was 5-7 µmoles product per minute per milligram total protein. Wild type AKIII was sensitive to the presence of L-lysine in the assay. Fifty percent inhibition was found at a concentration of about 0.4mM and 90 percent inhibition at about 0.1mM. In contrast, mutants AKIII-M2, M3 and M4 were not inhibited at all by 15mM L-lysine.

Wild type AKIII protein was purified from the supernatant of an induced culture as described in Example 2. Rabbit antibodies were raised against the purified AKIII protein.

Many other microbial expression vectors have been described in the literature. One skilled in the art could make use of any of these to construct lysC expression vectors. These lysC expression vectors could then be introduced into appropriate microorganisms via transformation to provide a system for high level expression of AKIII.

### Isolation of DHDPS genes

The E. coli dapA gene (ecodapA) has been cloned, restriction endonuclease mapped and sequenced previously [Richaud et al. (1986) J. Bacteriol. 166:297-300]. For the present invention the dapA gene was obtained on a bacteriophage lambda clone from an ordered library of 3400 overlapping segments of cloned E. coli. DNA constructed by Kohara, Akiyama and Isono [Kohara et al. (1987) Cell 50:595-508]. The ecodapA gene encodes a DHDPS enzyme that is sensitive to lysine inhibition. However, it is about 20-fold less sensitive to inhibition by lysine than a typical plant DHDPS, e.g., wheat germ DHDPS.

The Corynebacterium dapA gene (cordapA) was isolated from genomic DNA from ATCC strain 13032 using polymerase chain reaction (PCR). The nucleotide sequence of the Corynebacterium dapA gene has been published [Bonnassie et al. (1990) Nucleic Acids Res. 18:6421]. From the sequence it was possible to design oligonucleotide primers for polymerase chain reaction (PCR) that would allow amplification of a DNA fragment containing the gene, and at the same time add unique restriction endonuclease sites at the start codon and just past the stop codon of the gene to facilitate further constructions involving the gene. The details of the isolation of the cordapA gene are presented in Example 3. The cordapA gene encodes a DHDPS enzyme that is insensitive to lysine inhibiton.

In addition to introducing a restriction endonuclease site at the translation start codon, the PCR primers also changed the second codon of the cordapA gene from AGC coding for serine to GCT coding for alanine. Several cloned DNA fragments that expressed active, lysine-insensitive DHDPS were isolated, indicating that the second codon amino acid substitution did not affect enzyme activity.

The PCR-generated corynebacterium dapA gene was subcloned into the phagemid vactor pGEM-9zf(-) from Promega, and single-stranded DNA was generated and sequenced (SEQ ID NO:6). Aside from the differenced in the second codon already mentioned, the sequence matched the published sequence except at two positions, nucleotides 798 and 799. In the published sequence these are TC, while in the gene shown in SEQ ID NO:6 they are CT. This change results in an amino acid substitution of leucine for serine. The reason for this difference is not known. The difference has no apparent effect on DHDPS enzyme activity.

The isolation of other genes encoding DHDPS has been described in the literature. A cDNA encoding DHDPS from wheat [Kaneko et al. (1990) J. Biol. Chem. 265:17451-17455], and a cDNA encoding DHDPS from corn [Frisch et al. (1991) Mol. Gen. Genet. 228:287-293] are two examples. These genes encode wild type lysine-sensitive DHDPS enzymes. However, Negrutui et al. [(1984) Theor. Appl. Genet. 68:11-20], obtained two AEC-resistant tobacco mutants in which DHDPS activity was less sensitive to lysine inhibition than the wild type enzyme. These genes could be isolated using the methods already described for isolating the wheat or corn genes or, alternatively, by using the wheat or corn genes as heterologous hybridization probes.

Still other genes encoding DHDPS could be isolated by one skilled in the art by using either the ecodapA gene, the cordapA gene, or either of the plant DHDPS genes as DNA hybridization probes. Alternatively, other genes encoding DHDPS could be isolated by functional complementation of an E. coli dapA mutant, as was done to isolate the cordapA gene [Yeh et al. (1988) Mol. Gen. Genet. 212:105-111] and the corn DHDPS gene.

### High level expression of ecodapA and cordapA genes in E. coli

To achieve high level expression of the ecodapA and cordapA genes in E. coli, a bacterial expression vector which employs the bacteriophage T7 RNA polymerase/T7 promoter system [Rosenberg et al. (1987) Gene 56:127-135] was used. The vector and dapA genes were modified as described below to construct ecodapA and cordapA expression vectors.

For high level expression each of the expression vectors was transformed into E. coli strain BL21(DE3) [Studier et al. (1986) J. Mol. Biol. 189:113-130]. Cultures were grown, expression was induced, cells were collected, and extracts were prepared as described in Example 4. Supernatant and pellet fractions of extracts from uninduced and induced cultures were analyzed by SDS polyacrylamide gel electrophoresis and by DHDPS enzyme assays as described in Example 4. The major protein visible by Coomassie blue staining in the supernatant and pellet fractions of both induced cultures had a molecular weight of 32-34 kd, the expected size for DHDPS. Even in the uninduced cultures this protein was the most prominent protein produced.

In the induced culture with the ecodapA gene about 80% of the DHDPS protein was in the supernatant and DHDPS represented 10-20% of the total protein in the extract. In the induced culture with the cordapA gene more than 50% of the DHDPS protein was in the pellet fraction. The pellet fractions in both cases were 90-95% pure DHDPS, with no other single protein present in significant amounts. Thus, these fractions were pure enough for use in the generation of rabbit antibodies.

The specific activity of E. coli DHDPS in the supernatant fraction of induced extracts was about 50 OD₅₄₀ units per milligram protein. E. coli DHDPS was sensitive to the presence of L-lysine in the assay. Fifty percent inhibition was found at a concentration of about 0.5mM. For Corynebacterium DHDPS, enzyme activity was measured in the supernatant fraction of uninduced extracts, rather than induced extracts. Enzyme activity was about 4 OD₅₃₀ units per minute per milligram protein. In contrast to E. coli DHDPS, Corynebacterium DHDPS was not inhibited at all by L-lysine, even at a concentration of 70mM.

Many other microbial expression vectors have been described in the literature. One skilled in the art could make use of any of these to construct ecodapA or cordapA expression vectors. These expression vectors could then be introduced into appropriate microorganisms via transformation to provide a system for high level expression of DHDPS.

### Excretion of amino acids by E. coli expressing high levels of DHDPS and/or AKIII

The E. coli expression cassettes were inserted into expression vectors and then transformed into E. coli strain BL21(DE3) [Studier et al. (1986) J. Mol. Biol. 189:113-130] to induce E. coli to produce and excrete amino acids. Details of the procedures used and results are presented in Example 5.

Other microbial expression vectors known to those skilled in the art could be used to make and combine expression cassettes for the lysC and dapA genes. These expression vectors could then be introduced into appropriate microorganisms via transformation to provide alternative systems for production and excretion of lysine, theronine and methionine.

### Construction of Chimeric Genes for Expression of lysC and dapA Coding Regions in Plants

The expression of foreign genes in plants is well-established [De Blaere et al. (1987) Meth. Enzymol. 143:277-291]. Proper level of expression of lysC and dapA mRNAs may require the use of different chimeric genes utilizing different promoters. Such chimeric genes can be transferred into host plants either together in a single expression vector or sequentially using more than one vector. A preferred class of heterologous hosts for the expression of the coding sequence of lysC and dapA genes are eukaryotic hosts, particularly the cells of higher plants. Particularly preferred among the higher plants and the seeds derived from them are soybean, rapeseed (Brassica napus, B. campestris), sunflower (Helianthus annus), cotton (Gossypium hirsutum), corn, tobacco (Nicotiana Tubacum), alfalfa (Medicago sativa), wheat (Triticum sp), barley (Hordeum vulgare), oats (Avena sativa, L), sorghum (Sorghum bicolor), rice (Oryza sativa), and forage grasses. Expression in plants will use regulatory sequences functional in such plants.

The origin of promoter chosen to drive the expression of the coding sequence is not critical as long as it has sufficient transcriptional activity to accomplish the invention by expressing translatable mRNA for lysC or dapA genes in the desired host tissue. Preferred promoters for expression in all plant organs, and especially for expression in leaves include those directing the 19S and 35S transcripts in Cauliflower mosaic virus [Odell et al.(1985) Nature 313:810-812; Hull et al. (1987) Virology 86:482-493], small subunit of ribulose 1,5-bisphosphate carboxylase [Morelli et al. (1985) Nature 315:200; Broglie et al. (1984) Science 224:838; Hererra-Estrella et al.(1984) Nature 310:115; Coruzzi et al.(1984) EMBO J. 3:1671; Faciotti et al. (1985) Bio/Technology 3:241], maize zein protein [Matzke et al.(1984) EMBO J. 3:1525], and chlorophyll a/b binding protein [Lampa et al. (1986) Nature 316:750-752].

Depending upon the application, it may be desirable to select promoters that are specific for expression in one or more organs of the plant. Examples include the light-inducible promoters of the small subunit of ribulose 1,5-bisphosphate carboxylase, if the expression is desired in photosynthetic organs, or promoters active specifically in seeds.

Preferred promoters are those that allow expression of the protein specifically in seeds. This may be especially useful, since seeds are the primary source of vegetable amino acids and also since seed-specific expression will avoid any potential deleterious effect in non-seed organs. Examples of seed-specific promoters include, but are not limited to, the promoters of seed storage proteins. The seed storage proteins are strictly regulated, being expressed almost exclusively in seeds in a highly organ-specific and stage-specific manner [Higgins et al. (1984) Ann. Rev. Plant Physiol. 35:191-221; Goldberg et al.(1989) Cell 56:149-160; Thompson et al. (1989) BioEssays 10:108-113]. Moreover, different seed storage proteins may be expressed at different stages of seed development.

There are currently numerous examples for seed-specific expression of seed storage protein genes in transgenic dicotyledonous plants. These include genes from dicotyledonous plants for bean β-phaseolin [Sengupta-Goplalan et al. (1985) Proc. Natl. Acad. Sci. USA 82:3320-3324; Hoffman et al. (1988) Plant Mol. Biol. 11:717-729], bean lectin [Voelker et al. (1987) EMBO J. 6: 3571-3577], soybean lectin [Okamuro et al. (1986) Proc. Natl. Acad. Sci. USA 83:8240-8244], soybean kunitz trypsin inhibitor [Perez-Grau et al. (1989) Plant Cell 1:095-1109], soybean β-conglycinin [Beachy et al. (1985) EMBO J. 4:3047-3053; Barker et al. (1988) Proc. Natl. Acad. Sci. USA 85:458-462; Chen et al. (1988) EMBO J. 7:297-302; Chen et al. (1989) Dev. Genet. 10:112-122; Naito et al. (1988) Plant Mol. Biol. 11:109-123], pea vicilin [Higgins et al. (1988) Plant Mol. Biol. 11:683-695], pea convicilin [Newbigin et al. (1990) Planta 180:461], pea legumin [Shirsat et al. (1989) Mol. Gen. Genetics 215:326]; rapeseed napin [Radke et al. (1988) Theor. Appl. Genet. 75:685-694] as well as genes from monocotyledonous plants such as for maize 15 kD zein [Hoffman et al. (1987) EMBO J. 6:3213-3221; Schernthaner et al. (1988) EMBO J. 7:1249-1253; Williamson et al. (1988) Plant Physiol. 88:1002-1007], barley β-hordein [Marris et al. (1988) Plant Mol. Biol. 10:359-366] and wheat glutenin [Colot et al. (1987) EMBO J. 6:3559-3564]. Moreover, promoters of seed-specific genes, operably linked to heterologous coding sequences in chimeric gene constructs, also maintain their temporal and spatial expression pattern in transgenic plants. Such examples include Arabidopsis thaliana 2S seed storage protein gene promoter to express enkephalin peptides in Arabidopsis and B. napus seeds [Vandekerckhove et al. (1989) Bio/Technology 7:929-932], bean lectin and bean β-phaseolin promoters to express luciferase [Riggs et al. (1989) Plant Sci. 63:47-57], and wheat glutenin promoters to express chloramphenicol acetyl transferase [Colot et al. (1987) EMBO J. 6:3559-3564].

Of particular use in the expression of the nucleic acid fragment of the invention will be the heterologous promoters from several extensively-characterized soybean seed storage protein genes such as those for the Kunitz trypsin inhibitor [Jofuku et al. (1989) Plant Cell 1:1079-1093; Perez-Grau et al. (1989) Plant Cell 1:1095-1109], glycinin [Nielson et al. (1989) Plant Cell 1:313-328], β-conglycinin [Harada et al. (1989) Plant Cell 1:415-425]. Promoters of genes for α'- and β-subunits of soybean β-conglycinin storage protein will be particularly useful in expressing the lysC and dapA mRNAs in the cotyledons at mid- to late-stages of soybean seed development [Beachy et al. (1985) EMBO J. 4:3047-3053; Barker et al. (1988) Proc. Natl. Acad. Sci. USA 85:458-462; Chen et al. (1988) EMBO J. 7:297-302; Chen et al. (1989) Dev. Genet. 10:112-122; Naito et al. (1988) Plant Mol. Biol. 11:109-123] in transgenic plants, since: a) there is very little position effect on their expression in transgenic seeds, and b) the two promoters show different temporal regulation: the promoter for the α'-subunit gene is expressed a few days before that for the β-subunit gene.

Also of particular use in the expression of the nucleic acid fragments of the invention will be the heterologous promoters from several extensively characterized corn seed storage protein genes such as endosperm-specific promoters from the 10 kD zein [Kirihara et al. (1988) Gene 71:359-370], the 27 kD zein [Prat et al. (1987) Gene 52:51-49; Gallardo et al. (1988) Plant Sci. 54:211-281], and the 19 kD zein [Marks et al. (1985) J. Biol. Chem. 260:16451-16459]. The relative transcriptional activities of these promoters in corn have been reported [Kodrzyck et al. (1989) Plant Cell 1:105-114] providing a basis for choosing a promoter for use in chimeric gene constructs for corn. For expression in corn embryos, the strong embryo-specific promoter from the GLB1 gene [Kriz (1989) Biochemical Genetics 27:239-251, Wallace et al. (1991) Plant Physiol. 95:973-975] can be used.

It is envisioned that the introduction of enhancers or enhancer-like elements into other promoter constructs will also provide increased levels of primary transcription for lysC and dapA genes to accomplish the invention. These would include viral enhancers such as that found in the 35S promoter [Odell et al. (1988) Plant Mol. Biol. 10:263-272], enhancers from the opine genes [Fromm et al. (1989) Plant Cell 1:977-984], or enhancers from any other source that result in increased transcription when placed into a promoter operably linked to the nucleic acid fragment of the invention.

Of particular importance is the DNA sequence element isolated from the gene for the α'-subunit of β-conglycinin that can confer 40-fold seed-specific enhancement to a constitutive promoter [Chen et al. (1988) EMBO J. 7:297-302; Chen et al. (1989) Dev. Genet. 10:112-122]. One skilled in the art can readily isolate this element and insert it within the promoter region of any gene in order to obtain seed-specific enhanced expression with the promoter in transgenic plants. Insertion of such an element in any seed-specific gene that is expressed at different times than the β-conglycinin gene will result in expression in transgenic plants for a longer period during seed development.

Any 3' non-coding region capable of providing a polyadenylation signal and other regulatory sequences that may be required for the proper expression of the lysC or dapA coding regions can be used to accomplish the invention. This would include the 3' end from any storage protein such as the 3' end of the bean phaseolin gene, the 3' end of the soybean β-conglycinin gene, the 3' end from viral genes such as the 3' end of the 35S or the 19S cauliflower mosaic virus transcripts, the 3' end from the opine synthesis genes, the 3' ends of ribulose 1,5-bisphosphate carboxylase or chlorophyll a/b binding protein, or 3' end sequences from any source such that the sequence employed provides the necessary regulatory information within its nucleic acid sequence to result in the proper expression of the promoter/lysC coding region combination to which it is operably linked. There are numerous examples in the art that teach the usefulness of different 3' non-coding regions [for example, see Ingelbrecht et al. (1989) Plant Cell 1:671-680].

DNA sequences coding for intracellular localization sequences may be added to the lysC and dapA coding sequence if required for the proper expression of the proteins to accomplish the invention. Plant amino acid biosynthetic enzymes are known to be localized in the chloroplasts and therefore are synthesized with a chloroplast targeting signal. Bacterial proteins such as DHDPS and AKIII have no such signal. A chloroplast transit sequence could, therefore, be fused to the dapA and lysC coding sequences. Preferred chloroplast transit sequences are those of the small subunit of ribulose 1,5-bisphosphate carboxylase, e.g. from soybean [Berry-Lowe et al. (1982) J. Mol. Appl. Genet. 1:483-498] for use in dicotyledonous plants and from corn [Lebrun et al. (1987) Nucleic Acids Res. 15:4360] for use in monocotyledonous plants.

### Introduction of lysC and dapA Chimeric Genes into Plants

Various methods of introducing a DNA sequence (i.e., of transforming) into eukaryotic cells of higher plants are available to those skilled in the art (see EPO publications 0 295 959 A2 and 0 138 341 A1). Such methods include those based on transformation vectors based on the Ti and Ri plasmids of Agrobacterium spp. It is particularly preferred to use the binary type of these vectors. Ti-derived vectors transform a wide variety of higher plants, including monocotyledonous and dicotyledonous plants, such as soybean, cotton and rape [Pacciotti et al. (1985) Bio/Technology 3:241; Byrne et al. (1987) Plant Cell, Tissue and Organ Culture 8:3; Sukhapinda et al. (1987) Plant Mol. Biol. 8:209-216; Lorz et al. (1985) Mol. Gen. Genet. 199:178; Potrykus (1985) Mol. Gen. Genet. 199:183].

For introduction into plants the chimeric genes of the invention can be inserted into binary vectors as described in Examples 7-12 and 14-16. The vectors are part of a binary Ti plasmid vector system [Bevan, (1984) Nucl. Acids. Res. 12:8711-8720] of Agrobacterium tumefaciens.

Other transformation methods are available to those skilled in the art, such as direct uptake of foreign DNA constructs [see EPO publication 0 295 959 A2], techniques of electroporation [see Fromm et al. (1986) Nature (London) 319:791] or high-velocity ballistic bombardment with metal particles coated with the nucleic acid constructs [see Kline et al. (1987) Nature (London) 327:70, and see U.S. Pat. No. 4,945,050]. Once transformed, the cells can be regenerated by those skilled in the art.

Of particular relevance are the recently described methods to transform foreign genes into commercially important crops, such as rapeseed [see De Block et al. (1989) Plant Physiol. 91:694-701], sunflower [Everett et al. (1987) Bio/Technology 5:1201], soybean [McCabe et al. (1988) Bio/Technology 6:923; Hinchee et al. (1988) Bio/Technology 6:915; Chee et al. (1989) Plant Physiol. 91:1212-1218; Christou et al. (1989) Proc. Natl. Acad. Sci USA 86:7500-7504; EPO Publication 0 301 749 A2], and corn [Gordon-Kamm et al. (1990) Plant Cell 2:603-618; Fromm et al. (1990) Biotechnology 8:833-839].

For introduction into plants by high-velocity ballistic bombardment, the chimeric genes of the invention can be inserted into suitable vectors as described in Example 6. Transformed plants can be obtained as described in Examples 17-19.

### Expression of lysC and dapA Chimeric Genes in Plants

To assay for expression of the chimeric genes in leaves or seeds of the transformed plants, the AKIII or DHDPS proteins can be detected and quantitated enzymatically and/or immunologically by methods known to those skilled in the art. In this way lines producing high levels of expressed protein can be easily identified.

In order to measure the free amino acid composition of the leaves, free amino acids can be extracted by various methods including those as described in Example 7. To measure the free or total amino acid composition of seeds, extracts can be prepared by various methods including those as described in Example 8.

There was no significant effect of expression of AKIII or AKIII-M4 (with a chloroplast targeting signal) on the free lysine or threonine (or any other amino acid) levels in the leaves (see Table 2 in Example 7). Since AKIII-M4 is insensitive to feedback inhibition by any of the end-products of the pathway, this indicates that control must be exerted at other steps in the biosynthetic pathway in leaves.

In contrast, expression of the AKIII or AKIII-M4 (with a chloroplast targeting signal) in the seeds resulted in 2 to 4-fold or 4 to 23-fold increases, respectively, in the level of free threonine in the seeds compared to untransformed plants and 2 to 3-fold increases in the level of free lysine in some cases (Table 3, Example 8). There was a good correlation between transformants expressing higher levels of AKIII or AKIII-M4 protein and those having higher levels of free threonine, but this was not the case for lysine. The relatively small increases of free threonine or lysine achieved with the AKIII protein were not sufficient to yield detectable increases compared to untransformed plants, in the levels of total threonine or lysine in the seeds. The larger increases of free threonine achieved via expression of the AKIII-M4 protein were sufficient to yield detectable increases, compared to seeds from untransformed plants, in the levels of total threonine in the seeds. Sixteen to twenty-five percent increases in total threonine content of the seeds were observed. The lines that showed increased total threonine were the same ones the showed the highest levels of increase in free threonine and high expression of the AKIII-M4 protein.

The above teachings show that amino acid biosynthesis takes place in seeds and can be modulated by the expression of foreign genes encoding amino acid biosynthetic enzymes. Furthermore, they show that control of an amino acid biosynthetic pathway can differ markedly from one plant organ to another, e.g. seeds and leaves. The importance of this observation is emphasized upon considering the different effects of expressing a foreign DHDPS in leaves and seeds described below. It can be concluded that threonine biosynthesis in seeds is controlled primarily via end-product inhibition of AK. Therefore, threonine accumulation in the seeds of plants can be increased by expression of a gene, introduced via transformation, that encodes AK which is insensitive to lysine inhibition and which is localized in the chloroplast.

The above teachings also demonstrate that transformed plants which express higher levels of the introduced enzyme in seeds accumulate higher levels of free threonine in seeds. Furthermore, the teachings demonstrate that transformed plants which express a lysine-insensitive AK in seeds accumulate higher levels of free threonine in seeds than do transformed plants which express similar levels of a lysine-sensitive AK. To achieve commercially valuable increases in free threonine, a completely lysine-insensitive AK is preferred.

These teachings indicate that the level of free lysine in seeds controls the accumulation of another aspartate-derived amino acid, threonine, through end-product inhibition of AK. In order to accumulate high levels of free lysine itself, it will be necessary to bypass lysine inhibition of AK via expression of a lysine-insensitive AK.

Expression of active E. coli DHDPS enzyme was achieved in both young and mature leaves of the transformed tobacco plants (Table 4, Example 9). High levels of free lysine, 50 to 100-fold higher than normal tobacco plants, accumulated in the young leaves of the plants expressing the enzyme with a chloroplast targeting signal, but not without such a targeting signal. However, a much smaller accumulation of free lysine (2 to 8-fold) was seen in the larger leaves. Experiments that measure lysine in the phloem suggest that lysine is exported from the large leaves. This exported lysine may contribute to the accumulation of lysine in the small growing leaves, which are known to take up, rather than export nutrients. No effect on the free lysine levels in the seeds of these plants was observed even though E. coli DHDPS enzyme was expressed in the seeds as well as the leaves.

High level seed-specific expression of E. coli DHDPS enzyme, either with or without a chloroplast targeting signal, had no effect on the total, or free, lysine or threonine (or any other amino acid) composition of the seeds in any transformed line (Table 5, Example 10). These results demonstrate that expression in seeds of a DHDPS enzyme that is less sensitive to lysine than the plant enzyme is not sufficient to lead to increased production or accumulation of free lysine.

These teachings from transformants expressing the E. coli DHDPS enzyme indicate that lysine biosynthesis in leaves is controlled primarily via end-product inhibition of DHDPS, while in seeds there must be at least one additional point of control in the pathway. The teachings from transformants expressing the E. coli AKIII and AKIII-M4 enzymes indicate that the level of free lysine in seeds controls the accumulation of all aspartate-derived amino acids through end-product inhibition of AK. AK is therefore an additional control point.

To achieve simultaneous, high level expression of both E. coli DHDPS and AKIII-M4 in leaves and seeds, plants that express each of the genes could be crossed and hybrids that express both could be selected. Another method would be to construct vectors that contain both genes on the same DNA fragment and introduce the linked genes into plants via transformation. This is preferred because the genes would remain linked thoughout subsequent plant breeding efforts. Representative vectors carrying both genes on the same DNA fragment are described in Examples 11, 12, 16, and 18.

Tobacco plants transformed with a vector carrying both E. coli DHDPS and AKIII-M4 genes linked to the 35S promoter are described in Example 11. In transformants that express little or no AKIII-M4, the level of expression of E. coli DHDPS determines the level of lysine accumulation in leaves (Example 11, Table 6). However, in transformants that express both AKIII-M4 and E. coli DHDPS, the level of expression of each protein plays a role in controlling the level of lysine accumulation. Transformed lines that express DHDPS at comparable levels accumulate more lysine when AKIII-M4 is also expressed (Table 6, compare lines 564-18A, 564-56A, 564-36E, 564-55B, and 564-47A). Thus, expression of a lysine-insensitive AK increases lysine accumulation in leaves when expressed in concert with a DHDPS enzyme that is 20-fold less sensitive to lysine than the endogenous plant enzyme.

These leaf results, taken together with the seed results derived from expressing E. coli AKIII-M4 and E. coli DHDPS separately in seeds, suggest that simultaneous expression of both E. coli AKIII-M4 and E. coli DHDPS in seeds would lead to increased accumulation of free lysine and would also lead to an increased accumulation of free threonine. Tobacco plants transformed with a vector carrying both E. coli DHDPS and AKIII-M4 genes linked to the phaseolin promoter are described in Example 12. There is an increased accumulation of free lysine and free threonine in these plants. The increased level of free threonine was 4-fold over normal seeds, rather than the 20-fold increase seen in seeds expressing AKIII-M4 alone. The reduction in accumulation of free threonine indicates that pathway intermediates are being diverted down the lysine branch of the biosynthetic pathway. The increased level of free lysine was 2-fold over normal seeds (or seeds expressing E. coli DHDPS alone). However, the lysine increase in seeds is not equivalent to the 100-fold increase seen in leaves.

The E. coli DHDPS enzyme is less sensitive to lysine inhibition than plant DHDPS, but is still inhibited by lysine. The above teachings on the AK proteins indicate that expression of a completely lysine-insensitive enzyme can lead to a much greater accumulation of the aspartate pathway end-product threonine than expression of an enzyme which, while less sensitive than the plant enzyme, is still inhibited by lysine. Therefore vectors carrying both Corynebacterium DHDPS and AKIII-M4 genes linked to the seed-specific promoters were constructed as described in Examples 15 and 19. Tobacco plants transformed with vectors carrying both Corynebacterium DHDPS and AKIII-M4 genes linked to seed-specific promoters are described in Example 15. As shown in Table 9, these plants did not show a greater accumulation of free lysine in seeds than previously described plants expressing the E. coli DHDPS enzyme in concert with the lysine-insensitive AK. In hindsight this result can be explained by the fact that lysine accumulation in seeds never reached a level high enough to inhibit the E. coli DHDPS, so replacement of this enzyme with lysine-insensitive Corynebacterium DHDPS had no effect.

In transformed lines expressing high levels of E. coli AKIII-M4 and E. coli DHDPS or Corynebacterium DHDPS, it was possible to detect substantial amounts of α-aminoadipic acid. This compound is thought to be an intermediate in the catabolism of lysine in cereal seeds, but is normally detected only via radioactive tracer experiments due to its low level of accumulation. The discovery of high levels of this intermediate, comparable to levels of free amino acids, indicates that a large amount of lysine is being produced in the seeds of these transformed lines and is entering the catabolic pathway. The build-up of α-aminoadipic acid was not observed in transformants expressing only E. coli DHDPS or only AKIII-M4 in seeds. These results show that it is necessary to express both enzymes simultaneously to produce high levels of free lysine in seeds. To accumulate high levels of free lysine it may also be necessary to prevent lysine catabolism. Alternatively, it may be desirable to convert the high levels of lysine produced into a form that is insensitive to breakdown, e.g. by incorporating it into a di-, tri- or oligopeptide, or a lysine-rich storage protein.

### Isolation of a Plant Lysine Ketoglutarate Reductase Gene

There is little information yet available on lysine catabolism in plants. Available evidence indicates that lysine is catabolized via the saccharopine pathway. The first enzymatic evidence for the existence of this pathway was the detection of lysine ketoglutarate reductase (LKR) activity in immature endosperm of developing maize seeds [Arruda et al. (1982) Plant Physiol. 69:988-989]. LKR catalyzes the first step in lysine catabolism, the condensation of L-lysine with α-ketoglutarate into saccharopine using NADPH as a cofactor. LKR activity increases sharply from the onset of endosperm development, reaches a peak level at about 20 days after pollination, and then declines [Arruda et al. (1983) Phytochemistry 22:2687-2689]. In order to prevent the catabolism of lysine it would be desirable to reduce or eliminate LKR expression or activity. This could be accomplished by cloning the LKR gene, preparing a chimeric gene to express antisense RNA (Eur. Patent Applic. No. 84112647.7) for LKR, and introducing the chimeric gene into plants via transformation.

In order to clone the corn LKR gene, RNA was isolated from developing seeds 19 days after pollination. This RNA was sent to Clontech Laboratories, Inc., (Palo Alto, CA) for the custom synthesis of a cDNA library in the vector Lambda Zap II. The conversion of the Lambda Zap II library into a phagemid library, and then into a plasmid library was accomplished following the protocol provided by Clontech. Once converted into a plasmid library the ampicillin-resistant clones obtained carry the cDNA insert in the vector pBluescript SK(-). Expression of the cDNA is under control of the lacZ promoter on the vector.

To select clones that carried the LKR gene, a specially designed E. coli host, DE126, was constructed as described in Example 20. DE126 has the genotype F'16/malE52::Tn10, arg⁻, ilvA296, thrA1101, metL100, lysC⁺, λ⁻, rpsL9, malT1, xyl-7, mtl-2, thi-1?, supE44?. Its growth is inhibited by 20 µg/mL of L-lysine in a synthetic medium. Expression of LKR in DE126 is expected to reverse the growth inhibition by reducing the lysine concentration. Use of DE126 to select clones from the corn cDNA library that lead to lysine-resistant growth is described in Example 20.

The corn LKR cDNA obtained in this way can be used as a DNA hybridization probe to identify and isolate LKR genes from other plant species. A chimeric gene designed to express antisense RNA for LKR can be constructed by linking the LKR gene in reverse orientation to any of the plant promoter sequences described above. Preferred promoters would be seed-specific promoters. For corn, a strong endosperm-specific promoter, e.g., the 10 kD or 27 kD zein promoter, would be preferred.

In order to obtain plants that express a chimeric gene for antisense LKR, as well as genes encoding lysine-insensitive AK and DHDPS, the antisense LKR gene could be linked to genes encoding lysine-insensitive AK and DHDPS and all three genes could be introduced into plants via transformation. Alternatively, the chimeric gene for antisense LKR could be introduced into previously transformed plants that express lysine-insensitive AK and DHDPS, or the antisense LKR gene could be introduced into normal plants and the transformants obtained could be crossed with plants that express lysine-insensitive AK and DHDPS.

### Use of the cts/lysC-M4 Chimeric Gene as a Selectable Marker for Plant Transformation

Growth of cell cultures and seedlings of many plants is inhibited by high concentrations of lysine plus threonine. Growth is restored by addition of methionine (or homoserine which is converted to methionine in vivo). Lysine plus threonine inhibition is thought to result from feedback inhibition of endogenous AK, which reduces flux through the pathway leading to starvation for methionine. In tobacco there are two AK enzymes in leaves, one lysine-sensitive and one threonine sensitive.[Negrutui et al. (1984) Theor. Appl. Genet. 68:11-20]. High concentrations of lysine plus threonine inhibit growth of shoots from tobacco leaf disks and inhibition is reversed by addition of low concentrations of methionine. Thus, growth inhibition is presumably due to inhibition of the two AK isozymes.

Expression of active lysine and threonine insensitive AKIII-M4 also reverses lysine plus threonine growth inhibition (Table 2, Example 7). There is a good correlation between the level of AKIII-M4 protein expressed and the resistance to lysine plus threonine. Expression of lysine-sensitive wild type AKIII does not have a similar effect. Since expression of the AKIII-M4 protein permits growth under normally inhibitory conditions, a chimeric gene that causes expression of AKIII-M4 in plants can be used as a selectable genetic marker for transformation as illustrated in Examples 13 and 17.

### EXAMPLES

The present invention is further defined in the following Examples, in which all parts and percentages are by weight and degrees are Celsius, unless otherwise stated. It should be understood that these Examples, while indicating preferred embodiments of the invention, are given by way of illustration only. From the above discussion and these Examples, one skilled in the art can ascertain the essential characteristics of this invention, and without departing from the spirit and scope thereof, can make various changes and modifications of the invention to adapt it to various usages and conditions.

### EXAMPLE 1

### Isolation of the E. coli lysC Gene and mutations in lysC resulting in lysine-insensitive AKIII

The E. coli lysC gene has been cloned, restriction endonuclease mapped and sequenced previously [Cassan et al. (1986) J. Biol. Chem. 261:1052-1057]. For the present invention the lysC gene was obtained on a bacteriophage lambda clone from an ordered library of 3400 overlapping segments of cloned E. coli DNA constructed by Kohara, Akiyama and Isono [Kohara et al. (1987) Cell 50:595-508]. This library provides a physical map of the whole E. coli chromosome and ties the physical map to the genetic map. From the knowledge of the map position of lysC at 90 min. on the E. coli genetic map [Theze et al. (1974) J. Bacteriol. 117:133-143], the restriction endonuclease map of the cloned gene [Cassan et al. (1986) J. Biol. Chem. 261:1052-1057], and the restriction endonuclease map of the cloned DNA fragments in the E. coli library [Kohara et al. (1987) Cell 50:595-508], it was possible to choose lambda phages 4E5 and 7A4 [Kohara et al. (1987) Cell 50:595-508] as likely candidates for carrying the lysC gene. The phages were grown in liquid culture from single plaques as described [see Current Protocols in Molecular Biology (1987) Ausubel et al. eds. John Wiley & Sons New York] using LE392 as host [see Sambrook et al. (1989) Molecular Cloning: a Laboratory Manual, Cold Spring Harbor Laboratory Press]. Phage DNA was prepared by phenol extraction as described [see Current Protocols in Molecular Biology (1987) Ausubel et al. eds. John Wiley & Sons New York].

From the sequence of the gene several restriction endonuclease fragments diagnostic for the lysC gene were predicted, including an 1860 bp EcoR I-Nhe I fragment, a 2140 bp EcoR I-Xmn I fragment and a 1600 bp EcoR I-BamH I fragment. Each of these fragments was detected in both of the phage DNAs confirming that these carried the lysC gene. The EcoR I-Nhe I fragment was isolated and subcloned in plasmid pBR322 digested with the same enzymes, yielding an ampicillin-resistant, tetracycline-sensitive E. coli transformant. The plasmid was designated pBT436.

To establish that the cloned lysC gene was functional, pBT436 was transformed into E. coli strain Gif106M1 (E. coli Genetic Stock Center strain CGSC-5074) which has mutations in each of the three E. coli AK genes [Theze et al. (1974) J. Bacteriol. 117:133-143]. This strain lacks all AK activity and therefore requires diaminopimelate (a precursor to lysine which is also essential for cell wall biosynthesis), threonine and methionine. In the transformed strain all these nutritional requirements were relieved demonstrating that the cloned lysC gene encoded functional AKIII.

Addition of lysine (or diaminopimelate which is readily converted to lysine in vivo) at a concentration of approximately 0.2mM to the growth medium inhibits the growth of Gif106M1 transformed with pBT436. M9 media [see Sambrook et al. (1989) Molecular Cloning: a Laboratory Manual, Cold Spring Harbor Laboratory Press] supplemented with the arginine and isoleucine, required for Gif106M1 growth, and ampicillin, to maintain selection for the pBT436 plasmid, was used. This inhibition is reversed by addition of threonine plus methionine to the growth media. These results indicated that AKIII could be inhibited by exogenously added lysine leading to starvation for the other amino acids derived from aspartate. This property of pBT436-transformed Gif106M1 was used to select for mutations in lysC that encoded lysine-insensitive AKIII.

Single colonies of Gif106M1 transformed with pBT436 were picked and resuspended in 200µL of a mixture of 100µL 1% lysine plus 100µL of M9 media. The entire cell suspension containing 10⁷-10⁸ cells was spread on a petri dish containing M9 media supplemented with the arginine, isoleucine, and ampicillin. Sixteen petri dishes were thus prepared. From 1 to 20 colonies appeared on 11 of the 16 petri dishes. One or two (if available) colonies were picked and retested for lysine resistance and from this nine lysine-resistant clones were obtained. Plasmid DNA was prepared from eight of these and re-transformed into Gif106M1 to determine whether the lysine resistance determinant was plasmid-borne. Six of the eight plasmid DNAs yielded lysine-resistant colonies. Three of these six carried lysC genes encoding AKIII that was uninhibited by 15mM lysine, whereas wild type AKIII is 50% inhibited by 0.3-0.4mM lysine and >90% inhibited by 1mM lysine (see Example 2 for details).

To determine the molecular basis for lysine-resistance the sequences of the wild type lysC gene and three mutant genes were determined. A method for "Using mini-prep plasmid DNA for sequencing double stranded templates with sequenase™" [Kraft et al. (1988) BioTechniques 6:544-545] was used. Oligonucleotide primers, based on the published lysC sequence and spaced approximately every 200 bp, were synthesized to facilitate the sequencing. The sequence of the wild type lysC gene cloned in pBT436 (SEQ ID NO:1) differed from the published lysC sequence in the coding region at 5 positions. Four of these nucleotide differences were at the third position in a codon and would not result in a change in the amino acid sequence of the AKIII protein. One of the differences would result in a cysteine to glycine substitution at amino acid 58 of AKIII. These differences are probably due to the different strains from which the lysC genes were cloned.

The sequences of the three mutant lysC genes that encoded lysine-insensitive AK each differed from the wild type sequence by a single nucleotide, resulting in a single amino acid substitution in the protein. Mutant M2 had an A substituted for a G at nucleotide 954 of SEQ ID NO:1 resulting in an isoleucine for methionine substitution at amino acid 318 and mutants M3 and M4 had identical T for C substitutions at nucleotide 1055 of SEQ ID NO:1 resulting in an isoleucine for threonine substitution at amino acid 352. Thus, either of these single amino acid substitutions is sufficient to render the AKIII enzyme insensitive to lysine inhibition.

### EXAMPLE 2

### High level expression of wild type and mutant lysC genes in E. coli

An Nco I (CCATGG) site was inserted at the translation initiation codon of the lysC gene using the following oligonucleotides: When annealled these oligonucleotides have BamH I and Asp718 "sticky" ends. The plasmid pBT436 was digested with BamH I, which cuts upstream of the lysC coding sequence and Asp718 which cuts 31 nucleotides downstream of the initiation codon. The annealled oligonucleotides were ligated to the plasmid vector and E. coli transformants were obtained. Plasmid DNA was prepared and screened for insertion of the oligonucleotides based on the presence of an Nco I site. A plasmid containing the site was sequenced to assure that the insertion was correct, and was designated pBT457. In addition to creating an Nco I site at the initiation codon of lysC, this oligonucleotide insertion changed the second codon from TCT, coding for serine, to GCT, coding for alanine. This amino acid substitution has no apparent effect on the AKIII enzyme activity.

To achieve high level expression of the lysC genes in E. coli, the bacterial expression vector pBT430 was used. This vector is a derivative of pET-3a [Rosenberg et al. (1987) Gene 56:125-135] which employs the bacteriophage T7 RNA polymerase/T7 promoter system. Plasmid pBT430 was constructed by first destroying the EcoR I and Hind III sites in pET-3a at their original positions. An oligonucleotide adaptor containing EcoR I and Hind III sites was inserted at the BamH I site of pET-3a. This created pET-3aM with additional unique cloning sites for insertion of genes into the expression vector. Then, the Nde I site at the position of translation initiation was converted to an Nco I site using oligonucleotide-directed mutagenesis. The DNA sequence of pET-3aM in this region, 5'-CATATGG, was converted to 5'-CCCATGG in pBT430.

The lysC gene was cut out of plasmid pBT457 as a 1560 bp Nco I-EcoR I fragment and inserted into the expression vector pBT430 digested with the same enzymes, yielding plasmid pBT461. For expression of the mutant lysC genes (M2, M3 and M4) pBT461 was digested with Kpn I-EcoR I, which removes the wild type lysC gene from about 30 nucleotides downstream from the translation start codon, and inserting the analogous Kpn I-EcoR I fragments from the mutant genes yielding plasmids pBT490, pBT491 and pBT492, respectively.

For high level expression each of the plasmids was transformed into E. coli strain BL21(DE3) [Studier et al. (1986) J. Mol. Biol. 189:113-130]. Cultures were grown in LB medium containing ampicillin (100 mg/L) at 25°C. At an optical density at 600 nm of approximately 1, IPTG (isopropylthio-β-galactoside, the inducer) was added to a final concentration of 0.4 mM and incubation was continued for 3 h at 25°C. The cells were collected by centrifugation and resuspended in 1/20th (or 1/100th) the original culture volume in 50 mM NaCl; 50 mM Tris-Cl, pH 7.5; 1 mM EDTA, and frozen at -20°C. Frozen aliquots of 1 mL were thawed at 37°C and sonicated, in an ice-water bath, to lyse the cells. The lysate was centrifuged at 4°C for 5 min at 15,000 rpm. The supernatant was removed and the pellet was resuspended in 1 mL of the above buffer.

The supernatant and pellet fractions of uninduced and IPTG-induced cultures of BL21(DE3)/pBT461 were analyzed by SDS polyacrylamide gel electrophoresis. The major protein visible by Coomassie blue staining in the supernatant of the induced culture had a molecular weight of about 48 kd, the expected size for AKIII. About 80% of the AKIII protein was in the supernatant and AKIII represented 10-20% of the total E. coli protein in the extract.

AK activity was assayed as shown below:
Assay mix (for 12 assay tubes):
   4.5 mL H₂O
   1.0 mL 8M KOH
   1.0 mL 8M NH₂OH-HCl
   1.0 mL 1M Tris-HCl pH 8.0
   0.5 mL 0.2M ATP (121 mg/mL in 0.2M NaOH)
   50 µL 1M MgSO₄
Each 1.5 mL eppendorf assay tube contained:
   0.64 mL assay mix
   0.04 mL 0.2M L-aspartic acid or 0.04 mL H₂O
   0.0005-0.12 mL extract
   H₂0 to total volume 0.8 mL

Assay tubes were incubated at 30°C for desired time (10-60 min). Then 0.4-mL FeCl₃ reagent (10% w/v FeCl₃, 3.3% trichloroacetic acid, 0.7M HCl) was added and the material centrifuged for 2 min in an eppendorf centrifuge. The supernatant was decanted. The OD was read at 540nm and compared to the aspartyl-hydroxamate standard.

Approximately 80% of the AKIII activity was in the supernatant fraction. The specific activity of wild type and mutant crude extracts was 5-7 µmoles product per min per milligram total protein. Wild type AKIII was sensitive to the presence of L-lysine in the assay. Fifty percent inhibition was found at a concentration of about 0.4mM and 90 percent inhibition at about 1.0mM. In contrast, mutants AKIII-M2, M3 and M4 (see Example 1) were not inhibited at all by 15mM L-lysine.

Wild type AKIII protein was purified from the supernatant of the IPTG-induced culture as follows. To 1 mL of extract, 0.25 mL of 10% streptomycin sulfate was added and kept at 4°C overnight. The mixture was centrifuged at 4°C for 15 min at 15,000 rpm. The supernatant was collected and desalted using a Sephadex G-25 M column (Column PD-10, Pharmacia). It was then run on a Mono-Q HPLC column and eluted with a 0-1M NaCl gradient. The two 1 mL fractions containing most of the AKIII activity were pooled, concentrated, desalted and run on an HPLC sizing column (TSK G3000SW). Fractions were eluted in 20mM KPO₄ buffer, pH7.2, 2mM MgSO₄, 10mM β-mercaptoethanol, 0,15M KCl, 0.5mM L-lysine and were found to be >95% pure by SDS polyacrylamide gel electrophoresis. Purified AKIII protein was sent to Hazelton Research Facility (310 Swampridge Road, Denver, PA 17517) to have rabbit antibodies raised against the protein.

### EXAMPLE 3

### Isolation of the E. coli and Corynebacterium glutamicum dapA genes

The E. coli dapA gene (ecodapA) has been cloned, restriction endonuclease mapped and sequenced previously [Richaud et al. (1986) J. Bacteriol. 166:297-300]. For the present invention the dapA gene was obtained on a bacteriophage lambda clone from an ordered library of 3400 overlapping segments of cloned E. coli DNA constructed by Kohara, Akiyama and Isono [Kohara et al. (1987) Cell 50:595-508, see Example 1]. From the knowledge of the map position of dapA at 53 min on the E. coli genetic map [Bachman (1983) Microbiol. Rev. 47:180-230], the restriction endonuclease map of the cloned gene [Richaud et al. (1986) J. Bacteriol. 166:297-300], and the restriction endonuclease map of the cloned DNA fragments in the E. coli library [Kohara et al. (1987) Cell 50:595-508], it was possible to choose lambda phages 4C11 and 5A8 [Kohara et al. (1987) Cell 50:595-508] as likely candidates for carrying the dapA gene. The phages were grown in liquid culture from single plaques as described [see Current Protocols in Molecular Biology (1987) Ausubel et al. eds., John Wiley & Sons New York] using LE392 as host [see Sambrook et al. (1989) Molecular Cloning: a Laboratory Manual, Cold Spring Harbor Laboratory Press]. Phage DNA was prepared by phenol extraction as described [see Current Protocols in Molecular Biology (1987) Ausubel et al. eds., John Wiley & Sons New York]. Both phages contained an approximately 2.8 kb Pst I DNA fragment expected for the dapA gene [Richaud et al. (1986) J. Bacteriol. 166:297-300]. The fragment was isolated from the digest of phage 5A8 and inserted into Pst I digested vector pBR322 yielding plasmid pBT427.

The Corynebacterium dapA gene (cordapA) was isolated from genomic DNA from ATCC strain 13032 using polymerase chain reaction (PCR). The nucleotide sequence of the Corynebacterium dapA gene has been published [Bonnassie et al. (1990) Nucleic Acids Res. 18:6421]. From the sequence it was possible to design oligonucleotide primers for PCR that would allow amplification of a DNA fragment containing the gene, and at the same time add unique restriction endonuclease sites at the start codon (Nco I) and just past the stop codon (EcoR I) of the gene. The oligonucleotide primers used were:

PCR was performed using a Perkin-Elmer Cetus kit according to the instructions of the vendor on a thermocycler manufactured by the same company. The reaction product, when run on an agarose gel and stained with ethidium bromide, showed a strong DNA band of the size expected for the Corynebacterium dapA gene, about 900 bp. The PCR-generated fragment was digested with restriction endonucleases Nco I and EcoR I and inserted into expression vector pBT430 (see Example 2) digested with the same enzymes. In addition to introducing an Nco I site at the translation start codon, the PCR primers also resulted in a change of the second codon from AGC coding for serine to GCT coding for alanine. Several clones that expressed active, lysine-insensitive DHDPS (see Example 4) were isolated, indicating that the second codon amino acid substitution did not affect activity; one clone was designated FS766.

The Nco I to EcoR I fragment carrying the PCR-generated Corynebacterium dapA gene was subcloned into the phagemid vector pGEM-9Zf(-) from Promega, single-stranded DNA was prepared and sequenced. This sequence is shown in SEQ ID NO:6.

Aside from the differences in the second codon already mentioned, the sequence matched the published sequence except at two positions, nucleotides 798 and 799. In the published sequence these are TC, while in the gene shown in SEQ ID NO:6 they are CT. This change results in an amino acid substitution of leucine for serine. The reason for this difference is not known. It may be due to an error in the published sequence, the difference in strains used to isolate the gene, or a PCR-generated error. The latter seems unlikely since the same change was observed in at least 3 independently isolated PCR-generated dapA genes. The difference has no apparent effect on DHDPS enzyme activity (see Example 4).

### EXAMPLE 4

### High level expression of the E. coli and Corynebacterium glutamicum dapA genes in E. coli

An Nco I (CCATGG) site was inserted at the translation initiation codon of the E. coli dapA gene using oligonucleotide-directed mutagenesis. The 2.8 kb Pst I DNA fragment carrying the dapA gene in plasmid pBT427 (see Example 3) was inserted into the Pst I site of phagemid vector pTZ18R (Pharmacia) yielding pBT431. The orientation of the dapA gene was such that the coding strand would be present on the single-stranded phagemid DNA. Oligonucleotide-directed mutagenesis was carried out using a Muta-Gene kit from Bio-Rad according to the manufacturer's protocol with the mutagenic primer shown below: Putative mutants were screened for the presence of an Nco I site and a plasmid, designated pBT437, was shown to have the the proper sequence in the vicinity of the mutation by DNA sequencing. The addition of an Nco I site at the translation start codon also resulted in a change of the second codon from TTC coding for phenylalanine to GTC coding for valine.

To achieve high level expression of the dapA genes in E. coli the bacterial expression vector pBT430 (see Example 2) was used. The E. coli dapA gene was cut out of plasmid pBT437 as an 1150 bp Nco I-Hind III fragment and inserted into the expression vector pBT430 digested with the same enzymes, yielding plasmid pBT442. For expression of the Corynebacterium dapA gene, the 910 bp Nco I to EcoR I fragment of SEQ ID NO:6 inserted in pBT430 (pFS766, see Example 3) was used.

For high level expression each of the plasmids was transformed into E. coli strain BL21(DE3) [Studier et al. (1986) J. Mol. Biol. 189:113-130]. Cultures were grown in LB medium containing ampicillin (100 mg/L) at 25°C. At an optical density at 600 nm of approximately 1, IPTG (isopropylthio-β-galactoside, the inducer) was added to a final concentration of 0.4 mM and incubation was continued for 3 h at 25°C. The cells were collected by centrifugation and resuspended in 1/20th (or 1/100th) the original culture volume in 50 mM NaCl; 50 mM Tris-Cl, pH 7.5; 1 mM EDTA, and frozen at -20°C. Frozen aliquots of 1 mL were thawed at 37°C and sonicated, in an ice-water bath, to lyse the cells. The lysate was centrifuged at 4°C for 5 min at 15,000 rpm. The supernatant was removed and the pellet was resuspended in 1 mL of the above buffer.

The supernatant and pellet fractions of uninduced and IPTG-induced cultures of BL21(DE3)/pBT442 or BL21(DE3)/pFS766 were analyzed by SDS polyacrylamide gel electrophoresis. The major protein visible by Coomassie blue staining in the supernatant and pellet fractions of both induced cultures had a molecular weight of 32-34 kd, the expected size for DHDPS. Even in the uninduced cultures this protein was the most prominent protein produced.

In the BL21(DE3)/pBT442 IPTG-induced culture about 80% of the DHDPS protein was in the supernatant and DHDPS represented 10-20% of the total protein in the extract. In the BL21(DE3)/pFS766 IPTG-induced culture more than 50% of the DHDPS protein was in the pellet fraction. The pellet fractions in both cases were 90-95% pure DHDPS, with no other single protein present in significant amounts. Thus, these fractions were pure enough for use in the generation of antibodies. The pellet fractions containing 2-4 milligrams of either E. coli DHDPS or Corynebacterium DHDPS were solubilized in 50 mM NaCl; 50 mM Tris-Cl, pH 7.5; 1 mM EDTA, 0.2mM dithiothreitol, 0.2% SDS and sent to Hazelton Research Facility (310 Swampridge Road, Denver, PA 17517) to have rabbit antibodies raised against the proteins.

DHDPS enzyme activity was assayed as follows:
Assay mix (for 10 X 1.0 mL assay tubes or 40 X 0.25 mL for microtiter dish); made fresh, just before use:

| | |
|---|---|
| 2.5mL | H₂0 |
| 0.5mL | 1.0M Tris-HCl pH8.0 |
| 0.5mL | 0.1M Na Pyruvate |
| 0.5mL | o-Aminobenzaldehyde (10mg/mL in ethanol) |
| 25µL | 1.0M DL-Aspartic-β-semialdehyde (ASA) in 1.0N HCl |

| | Assay (1.0mL): | MicroAssay (0.25mL): |
|---|---|---|
| DHDPS assay mix | 0.40mL | 0.10mL |
| enzyme extract + H₂O; | 0.10mL | .025mL |
| 10mM L-lysine | 5µL or 20µL | 1µL or 5µL |
| Incubate at 30°C for desired time. | | Stop by addition of: |
| 1.0N HCl | 0.50mL | 0.125mL |

Color allowed to develop for 30-60 min. Precipitate spun down in eppendorf centrifuge. OD₅₄₀ vs 0 min read as blank. For MicroAssay, aliquot 0.2mL into microtiter well and read at OD₅₃₀.

The specific activity of E. coli DHDPS in the supernatant fraction of induced extracts was about 50 OD₅₄₀ units per minute per milligram protein in a 1.0 mL assay. E. coli DHDPS was sensitive to the presence of L-lysine in the assay. Fifty percent inhibition was found at a concentration of about 0.5mM. For Corynebacterium DHDPS, the activity was measured in the supernatant fraction of uninduced extracts, rather than induced extracts. Enzyme activity was about 4 OD₅₃₀ units per minute per milligram protein in a 0.25 mL assay. In contrast to E. coli DHDPS, Corynebacterium DHDPS was not inhibited at all by L-lysine, even at a concentration of 70mM.

### EXAMPLE 5

### Excretion of amino acids by E. coli expressing high levels of DHDPS and/or AKIII

The E. coli expression cassette with the E. coli dapA gene linked to the T7 RNA polymerase promoter was isolated by digesting pBT442 (see Example 4) with Bgl II and BamH I separating the digestion products via agarose gel electrophoresis and eluting the approximately 1250 bp fragment from the gel. This fragment was inserted into the BamH I site of plasmids pBT461 (containing the T7 promoter/lysC gene) and pBT492 (containing the T7 promoter/lysC-M4 gene). Inserts where transcription of both genes would be in the same direction were identified by restriction endonuclease analysis yielding plasmids pBT517 (T7/dapA + T7/lysC-M4) and pBT519 (T7/dapA + T7/lysC).

In order to induce E. coli to produce and excrete amino acids, these plasmids, as well as plasmids pBT442, pBT461 and pBT492 (and pBR322 as a control) were transformed into E. coli strain BL21(DE3) [Studier et al. (1986) J. Mol. Biol. 189:113-130]. All of these plasmids, but especially pBT517 and pBT519, are somewhat unstable in this host strain, necessitating careful maintenance of selection for ampicillin resistance during growth.

All strains were grown in minimal salts M9 media [see Sambrook et al. (1989) Molecular Cloning: a Laboratory Manual, Cold Spring Harbor Laboratory Press] supplemented with ampicillin to maintain selection for the plasmids overnight at 37°C. Cultures were collected when they reached an OD₆₀₀ of 1. Cells were removed by centrifugation and the supernatants (3 mL) were passed through 0.2 micron filters to remove remaining cells and large molecules. Five microliter aliquots of the supernatant fractions were analyzed for amino acid composition with a Beckman Model 6300 amino acid analyzer using post-column ninhydrin detection. Results are shown in Table 1.

**TABLE 1**

| Amino Acid Concentration in Culture Supernatants [mM] | | | | | | | |
|---|---|---|---|---|---|---|---|
| Plasmid | Lys | Thr | Met | Ala | Val | Asp | Glu |
| pBR322 | 0 | 0 | 0 | 0.05 | 0.1 | 0 | 0 |
| pBT442 | 0.48 | 0 | 0 | 0.04 | 0.06 | 0 | 0 |
| pBT461 | 0.14 | 0.05 | 0 | 0.02 | 0.03 | 0 | 0 |
| pBT492 | 0.16 | 0.07 | 0 | 0.02 | 0.03 | 0 | 0 |
| pBT517 | 0.18 | 0 | 0.01 | 0 | 0 | 0.02 | 0.02 |
| pBT519 | 0.14 | 0 | 0.01 | 0 | 0 | 0.01 | 0 |

All of the plasmids, except the pBR322 control, lead to the excretion of lysine into the culture medium. Expression of the lysC or the lysC-M4 gene lead to both lysine and threonine excretion. Expression of lysC-M4 + dapA lead to excretion of lysine, methionine, aspartic acid and glutamic acid, but not threonine. In addition, alanine and valine were not detected in the culture supernatant. Similar results were obtained with lysC + dapA, except that no glutamic acid was excreted.

### EXAMPLE 6

### Construction of Chimeric dapA, lysC and lysC-M4 Genes for Expression in Plants

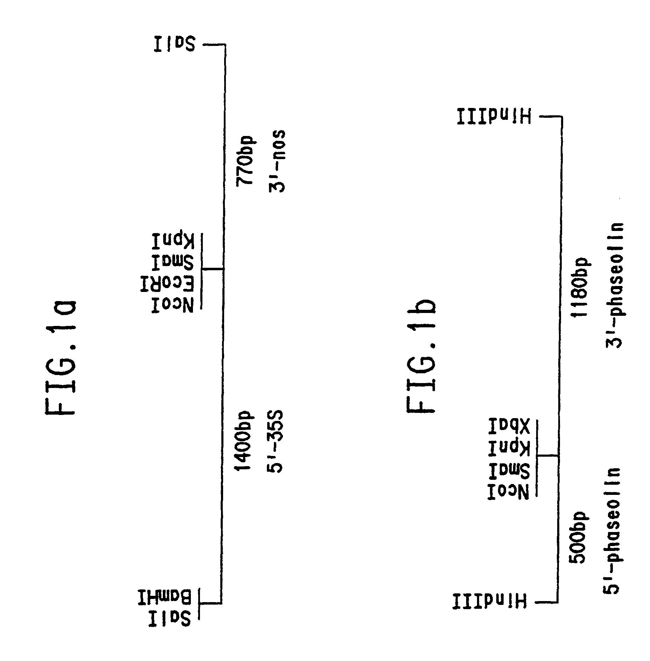

Several gene expression cassettes were used for construction of chimeric genes for expression of ecodapA, cordapA, lysC and lysC-M4 in plants. A leaf expression cassette (Figure 1a) is composed of the 35S promoter of cauliflower mosaic virus [Odell et al. (1985) Nature 313:810-812; Hull et al. (1987) Virology 86:482-493], the translation leader from the chlorophyll a/b binding protein (Cab) gene, [Dunsmuir (1985) Nucleic Acids Res. 13:2503-2518] and 3' transcription termination region from the nopaline synthase (Nos) gene [Depicker et al. (1982) J. Mol. Appl. Genet. 1:561-570]. Between the 5' and 3' regions are the restriction endonuclease sites Nco I (which includes the ATG translation initiation codon), EcoR I, Sma I and Kpn I. The entire cassette is flanked by Sal I sites; there is also a BamH I site upstream of the cassette.

A seed-specific expression cassette (Figure 1b) is composed of the promoter and transcription terminator from the gene encoding the β subunit of the seed storage protein phaseolin from the bean Phaseolus vulgaris [Doyle et al. (1986) J. Biol. Chem. 261:9228-9238]. The phaseolin cassette includes about 500 nucleotides upstream (5') from the translation initiation codon and about 1650 nucleotides downstream (3') from the translation stop codon of phaseolin. Between the 5' and 3' regions are the unique restriction endonuclease sites Nco I (which includes the ATG translation initiation codon), Sma I, Kpn I and Xba I. The entire cassette is flanked by Hind III sites.

A second seed expression cassette was used for the cordapA gene. This was composed of the promoter and transcription terminator from the soybean Kunitz trysin inhibitor 3 (KTI3) gene [Jofuku et al. (1989) Plant Cell 1:427-435]. The KTI3 cassette includes about 2000 nucleotides upstream (5') from the translation initiation codon and about 240 nucleotides downstream (3') from the translation stop codon of phaseolin. Between the 5' and 3' regions are the unique restriction endonuclease sites Nco I (which includes the ATG translation initiation codon), Xba I, Kpn I and Sma I. The entire cassette is flanked by BamH I sites.

A constitutive expression cassette for corn was used for expression of the lysC-M4 gene and the ecodapA gene. It was composed of a chimeric promoter derived from pieces of two corn promoters and modified by in vitro site-specific mutagenesis to yield a high level constitutive promoter and a 3' region from a corn gene of unknown function. Between the 5' and 3' regions are the unique restriction endonuclease sites Nco I (which includes the ATG translation initiation codon), Sma I and Bgl II. The nucleotide sequence of the constitutive corn expression cassette is shown in SEQ ID NO:16.

Plant amino acid biosynthetic enzymes are known to be localized in the chloroplasts and therefore are synthesized with a chloroplast targeting signal. Bacterial proteins such as DHDPS and AKIII have no such signal. A chloroplast transit sequence (cts) was therefore fused to the ecodapA, cordapA, lysC, and lysC-M4 coding sequence in some chimeric genes. The cts used was based on the the cts of the small subunit of ribulose 1,5-bisphosphate carboxylase from soybean [Berry-Lowe et al. (1982) J. Mol. Appl. Genet. 1:483-498]. The oligonucleotides SEQ ID NOS:8-11 were synthesized and used as described below. For corn the cts used was based on the the cts of the small subunit of ribulose 1,5-bisphosphate carboxylase from corn [Lebrun et al. (1987) Nucleic Acids Res. 15:4360] and is designated mcts to distinguish it from the soybean cts. The oligonucleotides SEQ ID NOS:17-22 were synthesized and used as described below.

Fourteen chimeric genes were created:

A 1440 bp Nco I-Hpa I fragment containing the entire lysC coding region plus about 90 bp of 3' non-coding sequence was isolated from an agarose gel following electrophoresis and inserted into the leaf expression cassette digested with Nco I and Sma I (chimeric gene No. 1), yielding plasmid pBT483.

Oligonucleotides SEQ ID NO:8 and SEQ ID NO:9, which encode the carboxy terminal part of the chloroplast targeting signal, were annealed, resulting in Nco I compatible ends, purified via polyacrylamide gel electrophoresis, and inserted into Nco I digested pBT461. The insertion of the correct sequence in the correct orientation was verified by DNA sequencing yielding pBT496. Oligonucleotides SEQ ID NO:10 and SEQ ID NO:11, which encode the amino terminal part of the chloroplast targeting signal, were annealed, resulting in Nco I compatible ends, purified via polyacrylamide gel electrophoresis, and inserted into Nco I digested pBT496. The insertion of the correct sequence in the correct orientation was verified by DNA sequencing yielding pBT521. Thus the cts was fused to the lysC gene.

To fuse the cts to the lysC-M4 gene, pBT521 was digested with Sal I, and an approximately 900 bp DNA fragment that included the cts and the amino terminal coding region of lysC was isolated. This fragment was inserted into Sal I digested pBT492, effectively replacing the amino terminal coding region of lysC-M4 with the fused cts and the amino terminal coding region of lysC. Since the mutation that resulted in lysine-insensitivity was not in the replaced fragment, the new plasmid, pBT523, carried the cts fused to lysC-M4.

The 1600 bp Nco I-Hpa I fragment containing the cts fused to lysC plus about 90 bp of 3' non-coding sequence was isolated and inserted into the leaf expression cassette digested with Nco I and Sma I (chimeric gene No. 2), yielding plasmid pBT541 and the seed-specific expression cassette digested with Nco I and Sma I (chimeric gene No. 4), yielding plasmid pBT543.

Similarly, the 1600 bp Nco I-Hpa I fragment containing the cts fused to lysC-M4 plus about 90 bp of 3' non-coding sequence was isolated and inserted into the leaf expression cassette digested with Nco I and Sma I (chimeric gene No. 3), yielding plasmid pBT540 and the seed-specific expression cassette digested with Nco I and Sma I (chimeric gene No. 5), yielding plasmid pBT544.

Before insertion into the expression cassettes, the ecodapA gene was modified to insert a restriction endonuclease site, Kpn I, just after the translation stop codon. The oligonucleotides SEQ ID NOS:12-13 were synthesized for this purpose:

Oligonucleotides SEQ ID NO:12 and SEQ ID NO:13 were annealed, resulting in an Sph I compatible end on one end and a Hind III compatible end on the other and inserted into Sph I plus Hind III digested pBT437. The insertion of the correct sequence was verified by DNA sequencing yielding pBT443.

An 880 bp Nco I-Kpn I fragment from pBT443 containing the entire ecodapA coding region was isolated from an agarose gel following electrophoresis and inserted into the leaf expression cassette digested with Nco I and Kpn I (chimeric gene No. 6), yielding plasmid pBT450 and into the seed-specific expression cassette digested with Nco I and Kpn I (chimeric gene No. 8), yielding plasmid pBT494.

Oligonucleotides SEQ ID NO:8 and SEQ ID NO:9, which encode the carboxy terminal part of the chloroplast targeting signal, were annealed resulting in Nco I compatible ends, purified via polyacrylamide gel electrophoresis, and inserted into Nco I digested pBT450. The insertion of the correct sequence in the correct orientation was verified by DNA sequencing yielding pBT451. A 950 bp Nco I-Kpn I fragment from pBT451 encoding the carboxy terminal part of the chloroplast targeting signal fused to the entire ecodapA coding region was isolated from an agarose gel following electrophoresis and inserted into the seed-specific expression cassette digested with Nco I and Kpn I, yielding plasmid pBT495. Oligonucleotides SEQ ID NO:10: and SEQ ID NO:11:, which encode the amino terminal part of the chloroplast targeting signal, were annealed resulting in Nco I compatible ends, purified via polyacrylamide gel electrophoresis, and inserted into Nco I digested pBT451 and pBT495. Insertion of the correct sequence in the correct orientation was verified by DNA sequencing yielding pBT455 and pBT520, respectively. Thus the cts was fused to the ecodapA gene in the leaf expression cassette (chimeric gene No. 7) and the seed-specific expression cassette (chimeric gene No. 9).

An 870 bp Nco I-EcoR I fragment from pFS766 containing the entire cordapA coding region was isolated from an agarose gel following electrophoresis and inserted into the leaf expression cassette digested with Nco I and EcoR I, yielding plasmid pFS789. To attach the cts to the cordapA gene a DNA fragment containing the entire cts was prepared using PCR. The template DNA was pBT540 and the oligonucleotide primers used were:

PCR was performed using a Perkin-Elmer Cetus kit according to the instructions of the vendor on a thermocycler manufactured by the same company. The PCR-generated 160 bp fragment was treated with T4 DNA polymerase in the presence of the 4 deoxyribonucleotide triphosphates to obtain a blunt-ended fragment. The cts fragment was inserted into pFS789 which had been digested with Nco I and treated with the Klenow fragment of DNA polymerase to fill in the 5' overhangs. The inserted fragment and the vector/insert junctions were determined to be correct by DNA sequencing, yielding pFS846 containing chimeric gene No. 10.

A 1030 bp Nco I-Kpn I fragment from pFS846 containing the cts attached to the cordapA coding region was isolated from an agarose gel following electrophoresis and inserted into the phaseolin seed expression cassette digested with Nco I and Kpn I, yielding plasmid pFS889 containing chimeric gene No. 11. Similarly, the 1030 bp Nco I-Kpn I fragment from pFS846 was inserted into the KTI3 seed expression cassette digested with Nco I and Kpn I, yielding plasmid pFS862 containing chimeric gene No. 12.

Oligonucleotides SEQ ID NO:17 and SEQ ID NO:18, which encode the carboxy terminal part of the corn chloroplast targeting signal, were annealed, resulting in Xba I and Nco I compatible ends, purified via polyacrylamide gel electrophoresis, and inserted into Xba I plus Nco I digested pBT492 (see Example 2). The insertion of the correct sequence was verified by DNA sequencing yielding pBT556. Oligonucleotides SEQ ID NO:19 and SEQ ID NO:20, which encode the middle part of the chloroplast targeting signal, were annealed, resulting in Bgl II and Xba I compatible ends, purified via polyacrylamide gel electrophoresis, and inserted into Bgl II and Xba I digested pBT556. The insertion of the correct sequence was verified by DNA sequencing yielding pBT557. Oligonucleotides SEQ ID NO:21 and SEQ ID NO:22, which encode the amino terminal part of the chloroplast targeting signal, were annealed, resulting in Nco I and Afl II compatible ends, purified via polyacrylamide gel electrophoresis, and inserted into Nco I and Afl II digested pBT557. The insertion of the correct sequence was verified by DNA sequencing yielding pBT558. Thus the mcts was fused to the lysC-M4 gene.

A 1.6 kb Nco I-Hpa I fragment from pBT558 containing the mcts attached to the lysC-M4 gene was isolated from an agarose gel following electrophoresis and inserted into the constitutive corn expression cassette digested with Nco I and Sma I, yielding plasmid pBT573 containing chimeric gene No. 13.

To attach the mcts to the ecodapA gene a DNA fragment containing the entire mcts was prepared using PCR as described above. The template DNA was pBT558 and the oligonucleotide primers used were:

The mcts fragment was inserted into pBT450 (above) which had been digested with Nco I and treated with the Klenow fragment of DNA polymerase to fill in the 5' overhangs. The inserted fragment and the vector/insert junctions were determined to be correct by DNA sequencing, yielding pBT576. Plasmid pBT576 was digested with Asp718, treated with the Klenow fragment of DNA polymerase to yield a blunt-ended fragment, and then digested with Nco I. The resulting 1030 bp Nco I-blunt-ended fragment containing the ecodapA gene attached to the mcts was isolated from an agarose gel following electrophoresis. This fragment was inserted into the constitutive corn expression cassette digested with Bgl II, treated with the Klenow fragment of DNA polymerase to yield a blunt-ended fragment, and then digested with Nco I, yielding plasmid pBT583 containing chimeric gene No. 14.

### EXAMPLE 7

### Transformation of Tobacco with the 35S Promoter/lysC Chimeric Genes

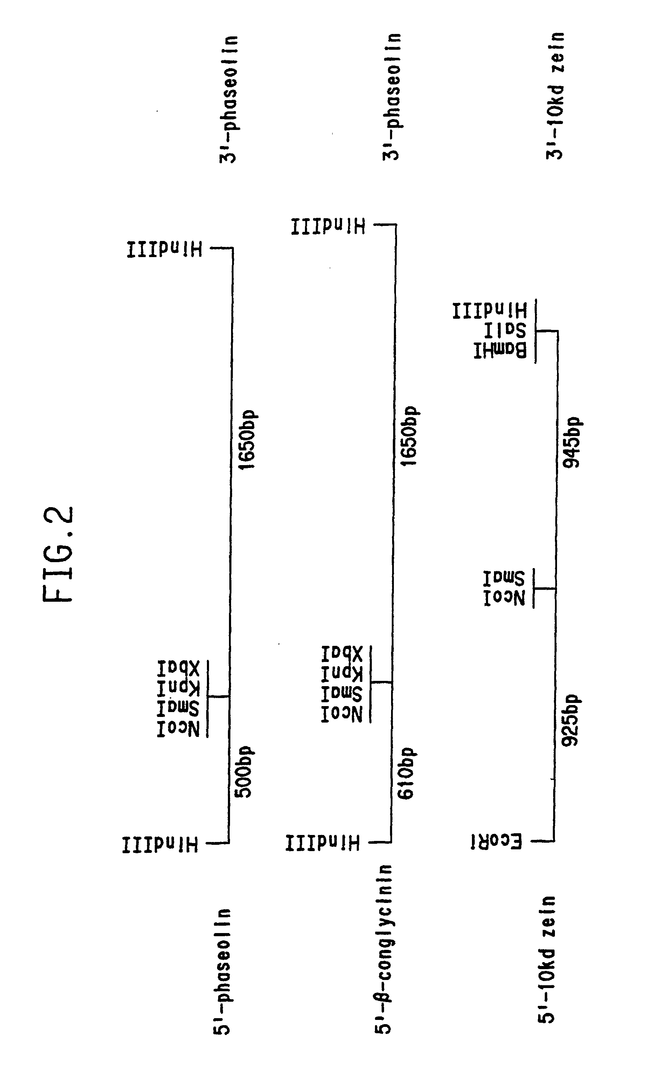

Transformation of tobacco with the 35S promoter/lysC chimeric genes was effected according to the following:
The 35S promoter/Cab leader/lysC/Nos 3', 35S promoter/Cab leader/cts/lysC/Nos 3', and 35S promoter/Cab leader/cts/lysC-M4/Nos 3' chimeric genes were isolated as 3.5-3.6 kb BamH I-EcoR I fragments and inserted into BamH I-EcoR I digested vector pZS97K (Figure 2), yielding plasmids pBT497, pBT545 and pBT542, respectively. The vector is part of a binary Ti plasmid vector system [Bevan, (1984) Nucl. Acids. Res. 12:8711-8720] of Agrobacterium tumefaciens. The vector contains:
   (1) the chimeric gene nopaline synthase promoter/neomycin phosphotransferase coding region (nos:NPT II) as a selectable marker for transformed plant cells [Bevan et al. (1983) Nature 304:184-186];
   (2) the left and right borders of the T-DNA of the Ti plasmid [Bevan (1984) Nucl. Acids. Res. 12:8711-8720];
   (3) the E. coli lacZ α-complementing segment [Vieria and Messing (1982) Gene 19:259-267] with unique restriction endonuclease sites for EcoR I, Kpn I, BamH I and Sal I;
   (4) the bacterial replication origin from the Pseudomonas plasmid pVS1 [Itoh et al. (1984) Plasmid 11:206-220]; and (5) the bacterial neomycin phosphotransferase gene from Tn5 [Berg et al. (1975) Proc. Natl. Acad Sci. U.S.A. 72:3628-3632] as a selectable marker for transformed A. tumefaciens.
The 35S promoter/Cab leader/cts/lysC/Nos 3', and 35S promoter/Cab leader/cts/lysC-M4/Nos 3' chimeric genes were also inserted into the binary vector pBT456, yielding pBT547 and pBT546, respectively. This vector is pZS97K, into which the chimeric gene 35S promoter/Cab leader/cts/dapA/Nos 3' had previously been inserted as a BamH I-Sal I fragment (see Example 9). In the cloning process large deletions of the dapA chimeric gene occurred. As a consequence these plasmids are equivalent to pBT545 and pBT542, in that the only transgene expressed in plants (other than the selectable marker gene, NPT II) was 35S promoter/Cab leader/cts/lysC/Nos 3' or 35S promoter/Cab leader/cts/lysC-M4/Nos 3'.

The binary vectors containing the chimeric lysC genes were transferred by tri-parental matings [Ruvkin et al. (1981) Nature 289:85-88] to Agrobacterium strain LBA4404/pAL4404 [Hockema et al (1983), Nature 303:179-180]. The Agrobacterium transformants were used to inoculate tobacco leaf disks [Horsch et al. (1985) Science 227:1229-1231]. Transgenic plants were regenerated in selective medium containing kanamycin.

To assay for expression of the chimeric genes in leaves of the transformed plants, protein was extracted as follows. Approximately 2.5 g of young plant leaves, with the midrib removed, were placed in a dounce homogenizer with 0.2 g of polyvinyl polypyrrolidone and 11 mL of 50mM Tris-HCl pH8.0, 50mM NaCl, 1mM EDTA (TNE) and ground thoroughly. The suspension was further homogenized by a 20 sec treatment with a Brinkman Polytron Homogenizer operated at setting 7. The resultant suspensions were centrifuged at 16,000 rpm for 20 min at 4°C in a Dupont-Sorvall superspeed centrifuge using an SS34 rotor to remove particulates. The supernatant was decanted, the volume was adjusted to be 10 mL by addition of TNE if necessary, and 8 mL of cold, saturated ammonium sulfate was added. The mixture was set on ice for 30 min and centrifuged as described above. The supernatant was decanted and the pellet, which contained the AKIII protein, was resuspended in 1 mL of TNE and desalted by passage over a Sephadex G-25 M column (Column PD-10, Pharmacia).

For immunological characterization, three volumes of extract were mixed with 1 volume of 4 X SDS-gel sample buffer (0.17M Tris-HCl pH6.8, 6.7% SDS, 16.7% β-mercaptoethanol, 33% glycerol) and 3 µL from each extract were run per lane on an SDS polyacrylamide gel, with bacterially produced AKIII serving as a size standard and protein extracted from untransformed tobacco leaves serving as a negative control. The proteins were then electrophoretically blotted onto a nitrocellulose membrane (Western Blot). The membranes were exposed to the AKIII antibodies prepared as described in Example 2 at a 1:5000 dilution of the rabbit serum using standard protocol provided by BioRad with their Immun-Blot Kit. Following rinsing to remove unbound primary antibody, the membranes were exposed to the secondary antibody, donkey anti-rabbit Ig conjugated to horseradish peroxidase (Amersham) at a 1:3000 dilution. Following rinsing to remove unbound secondary antibody, the membranes were exposed to Amersham chemiluminescence reagent and X-ray film.

Seven of thirteen transformants containing the chimeric gene, 35S promoter/Cab leader/cts/lysC-M4/Nos 3', and thirteen of seventeen transformants containing the chimeric gene, 35S promoter/Cab leader/cts/lysC/Nos 3', produced AKIII protein (Table 2). In all cases protein which reacted with the AKIII antibody was of several sizes. Approximately equal quantities of proteins equal in size to AKIII produced in E. coli, and a protein about 6 kd larger were evident in all samples, suggesting that the chloroplast targeting signal had been removed from about half of the protein synthesized. This further suggests that about half of the the protein entered the chloroplast. In addition, a considerable amount of protein of higher molecular weight was observed. The origin of this protein is unclear; the total amount present was equal or slightly greater than the amounts of the mature and putative AKIII precursor proteins combined.

The leaf extracts were assayed for AK activity as described in Example 2. AKIII could be distinguished from endogenous AK activity, if it were present, by its increased resistance to lysine plus threonine. Unfortunately, however, this assay was not sensitive enough to reliably detect AKIII activity in these extracts. Zero of four transformants containing the chimeric gene, 35S promoter/Cab leader/lysC/Nos 3', showed AKIII activity. Only one extract, from a transformant containing the 35S promoter/Cab leader/cts/lysC-M4/Nos 3' gene, produced a convincing level of enzyme activity. This came from transformant 546-49A, and was also the extract that showed the highest level of AKIII-M4 protein via Western blot.

An alternative method to detect the expression of active AKIII enzyme was to evaluate the sensitivity or resistance of leaf tissue to high concentrations of lysine plus threonine. Growth of cell cultures and seedlings of many plants is inhibited by high concentrations of lysine plus threonine; this is reversed by addition of methionine (or homoserine which is converted to methionine in vivo). Lysine plus threonine inhibition is thought to result from feedback inhibition of endogenous AK, which reduces flux through the pathway leading to starvation for methionine. In tobacco there are two AK enzymes in leaves, one lysine-sensitive and one threonine sensitive [Negrutui et al. (1984) Theor. Appl. Genet. 68:11-20]. High concentrations of lysine plus threonine inhibit growth of shoots from tobacco leaf disks and inhibition is reversed by addition of low concentrations of methionine. Thus, growth inhibition is presumably due to inhibition of the two AK isozymes.

Expression of active lysine and threonine insensitive AKIII-M4 would be predicted to reverse the growth inhibition. As can be seen in Table 2, this was observed. There is, in fact, a good correlation between the level of AKIII-M4 protein expressed and the resistance to lysine plus threonine inhibition. Expression of lysine-sensitive wild type AKIII does not have a similar effect. Only the highest expressing transformant showed any resistance to lysine plus threonine inhibition, and this was much less dramatic than that observed with AKIII-M4.

To measure free amino acid composition of the leaves, free amino acids were extracted as follows. Approximately 30-40 mg of young leaf tissue was chopped with a razor and dropped into 0.6 mL of methanol/chloroform/water mixed in ratio of 12v/5v/3v (MCW) on dry ice. After 10-30 min the suspensions were brought to room temperature and homogenized with an Omni 1000 Handheld Rechargeable Homogenizer and then centrifuged in an eppendorf microcentrifuge for 3 min.
Approximately 0.6mL of supernatant was decanted and an additional 0.2mL of MCW was added to the pellet which was then vortexed and centrifuged as above. The second supernatant, about 0.2mL, was added to the first. To this, 0.2mL of chloroform was added followed by 0.3mL of water. The mixture was vortexed and the centrifuged in an eppendorf microcentrifuge for about 3 min, the upper aqueous phase, approximately 1.0mL, was removed, and was dried down in a Savant Speed Vac Concentrator.
One-tenth of the sample was run on a Beckman Model 6300 amino acid analyzer using post-column ninhydrin detection. Relative free amino acid levels in the leaves were compared as ratios of lysine or threonine to leucine, thus using leucine as an internal standard. There was no consistent effect of expression of AKIII or AKIII-M4 on the lysine or threonine (or any other amino acid) levels in the leaves (Table 2).

**TABLE 2**

| BT542 transformants: 35S promoter/Cab leader/cts/lysC-M4/Nos 3' BT545 transformants: 35S promoter/Cab leader/cts/lysC/Nos 3' BT546 transformants: 35S promoter/Cab leader/cts/lysC-M4/Nos 3' BT547 transformants: 35S promoter/Cab leader/cts/lysC/Nos 3' | | | | | |
|---|---|---|---|---|---|
| | FREE AMINO ACIDS/LEAF | | AKIII ACTIVITY | WESTERN BLOT | RESISTANCE TO Lys 3mM+ Thr 3mM |
| LINE | K/L | T/L | U/MG/HR | | |
| 542-5B | 0.5 | 3.5 | 0 | - | - |
| 542-26A | 0.5 | 3.3 | 0 | - | - |
| 542-27B | 0.5 | 3.4 | 0 | ++ | +++ |
| 542-35A | 0.5 | 4.3 | 0.01 | - | - |
| 542-54A | 0.5 | 2.8 | 0 | - | - |
| 542-57B | 0.5 | 3.4 | 0 | - | + |
| 545-5A | n.d. | n.d. | 0.02 | ++ | |
| 545-7B | 0.5 | 3.4 | 0 | + | |
| 545-17B | 0.6 | 2.5 | 0.01 | + | |
| 545-27A | 0.6 | 3.5 | 0 | ++ | |
| 545-50E | 0.6 | 3.6 | 0.03 | ++ | |
| 545-52A | 0.5 | 3.6 | 0.02 | - | |
| | | | | | |
| 546-4A | 0.4 | 4.5 | 0 | + | + |
| 546-24B | 0.6 | 4.9 | 0.04 | ++ | ++ |
| 546-44A | 0.5 | 6.0 | 0.03 | + | ++ |
| 546-49A | 0.7 | 7.0 | 0.10 | +++ | +++ |
| 546-54A | 0.5 | 6.4 | 0 | + | + |
| 546-56B | 0.5 | 4.4 | 0.01 | - | - |
| 546-58B | 0.6 | 8.0 | 0 | + | ++ |
| | | | | | |
| 547-3D | 0.4 | 5.4 | 0 | ++ | - |
| 547-BB | 0.6 | 5.0 | 0.02 | - | |
| 547-9A | 0.5 | 4.3 | 0.03 | +++ | |
| | | | | | |
| 547-12A | 0.7 | 3.9 | 0 | +++ | + |
| 547-15B | 0.6 | 4.5 | 0 | + | - |
| 547-16A | 0.5 | 3.6 | 0 | ++ | |
| 547-18A | 0.5 | 4.0 | | +++ | - |
| 547-22A | 0.8 | 4.4 | | - | |
| 547-25C | 0.5 | 4.3 | | + | - |
| 547-28C | 0.6 | 5.6 | | - | |
| 547-29C | 0.5 | 3.8 | | +++ | + |

### EXAMPLE 8

### Transformation of Tobacco with the Phaseolin Promoter/lysC Chimeric Genes

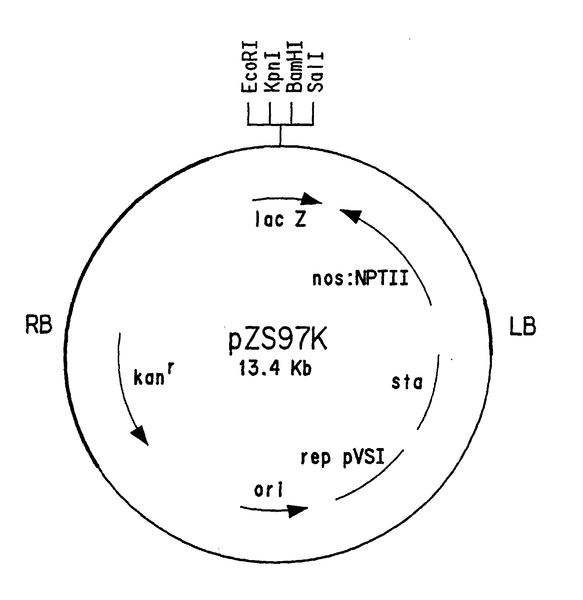

The phaseolin promoter/lysC chimeric gene cassettes, phaseolin 5' region/cts/lysC/phaseolin 3' region, and phaseolin 5' region/cts/lysC-M4/phaseolin 3' region (Example 6) were isolated as approximately 3.3 kb Hind III fragments. These fragments were inserted into the unique Hind III site of the binary vector pZS97 (Figure 3) yielding pBT548 and pBT549, respectively. This vector is similar to pZS97K described in Example 7 except for the presence of two additional unique cloning sites, Sma I and Hind III, and the bacterial β-lactamase gene (causing ampicillin resistance) as a selectable marker for transformed A. tumefaciens instead of the bacterial neomycin phosphotransferase gene.

The binary vectors containing the chimeric lysC genes were transferred by tri-parental matings to Agrobacterium strain LBA4404/pAL4404, the Agrobacterium transformants were used to inoculate tobacco leaf disks and transgenic plants regenerated by the methods set out in Example 7.

To assay for expression of the chimeric genes in the seeds of the transformed plants, the plants were allowed to flower, self-pollinate and go to seed. Total proteins were extracted from mature seeds as follows. Approximately 30-40 mg of seeds were put into a 1.5mL disposable plastic microfuge tube and ground in 0.25mL of 50mM Tris-HCl pH6.8, 2mM EDTA, 1% SDS, 1% β-mercaptoethanol. The grinding was done using a motorized grinder with disposable plastic shafts designed to fit into the microfuge tube. The resultant suspensions were centrifuged for 5 min at room temperature in a microfuge to remove particulates. Three volumes of extract was mixed with 1 volume of 4 X SDS-gel sample buffer (0.17M Tris-HCl pH6.8, 6.7% SDS, 16.7% β-mercaptoethanol, 33% glycerol) and 5µL from each extract were run per lane on an SDS polyacrylamide gel, with bacterially produced AKIII serving as a size standard and protein extracted from untransformed tobacco seeds serving as a negative control. The proteins were then electrophoretically blotted onto a nitrocellulose membrane. The membranes were exposed to the AKIII antibodies (prepared as described in Example 2) at a 1:5000 dilution of the rabbit serum using standard protocol provided by BioRad with their Immun-Blot Kit. Following rinsing to remove unbound primary antibody the membranes were exposed to the secondary antibody, donkey anti-rabbit Ig conjugated to horseradish peroxidase (Amersham) at a 1:3000 dilution. Following rinsing to remove unbound secondary antibody, the membranes were exposed to Amersham chemiluminescence reagent and X-ray film.

Ten of eleven transformants containing the chimeric gene, phaseolin 5' region/cts/lysC/phaseolin 3' region, and ten of eleven transformants containing the chimeric gene, phaseolin 5' region/cts/lysC-M4/phaseolin 3' region, produced AKIII protein (Table 3). In all cases protein which reacted with the AKIII antibody was of several sizes. Approximately equal quantities of proteins equal in size to AKIII produced in E. coli, and about 6 kd larger were evident in all samples, suggesting that the chloroplast targeting signal had been removed from about half of the protein synthesized. This further suggests that about half of the the protein entered the chloroplast. In addition, some proteins of lower molecular weight were observed, probably representing breakdown products of the AKIII polypeptide.

To measure free amino acid composition of the seeds, free amino acids were extracted from mature seeds as follows. Approximately 30-40 mg of seeds and an approximately equal amount of sterilized sand were put into a 1.5mL disposable plastic microfuge tube along with 0.2mL of methanol/chloroform/water mixed in ratio of 12v/5v/3v (MCW) at room temperature. The seeds were ground using a motorized grinder with disposable plastic shafts designed to fit into the microfuge tube. After grinding an additional 0.5mL of MCW was added, the mixture was vortexed and then centrifuged in an eppendorf microcentrifuge for about 3 min. Approximately 0.6mL of supernatant was decanted and an additional 0.2mL of MCW was added to the pellet which was then vortexed and centrifuged as above. The second supernatant, about 0.2mL, was added to the first. To this, 0.2mL of chloroform was added followed by 0.3mL of water. The mixture was vortexed and then centrifuged in an eppendorf microcentrifuge for about 3 min, the upper aqueous phase, approximately 1.0mL, was removed, and was dried down in a Savant Speed Vac Concentrator. The samples were hydrolyzed in 6N hydrochloric acid, 0.4% β-mercaptoethanol under nitrogen for 24 h at 110-120°C; 1/4 of the sample was run on a Beckman Model 6300 amino acid analyzer using post-column ninhydrin detection. Relative free amino acid levels in the seeds were compared as ratios of lysine, methionine, threonine or isoleucine to leucine, thus using leucine as an internal standard.

To measure the total amino acid composition of the seeds, 6 seeds were hydrolyzed in 6N hydrochloric acid, 0.4% β-mercaptoethanol under nitrogen for 24 h at 110-120°C; 1/10 of the sample was run on a Beckman Model 6300 amino acid analyzer using post-column ninhydrin detection. Relative amino acid levels in the seeds were compared as ratios of lysine, methionine, threonine or isoleucine to leucine, thus using leucine as an internal standard. Because the transgene was segregating in these self-pollinated progeny of the primary transformant and only six seeds were analyzed, there was expected to be some sampling error. Therefore, the measurement was repeated multiple times for some of the lines (Table 3).

Expression of the cts/lysC gene in the seeds resulted in a 2 to 4-fold increase in the level of free threonine in the seeds and a 2 to 3-fold increase in the level of free lysine in some cases. There was a good correlation between transformants expressing higher levels of AKIII protein and those having higher levels of free threonine, but this was not the case for lysine. These relatively small increases of free threonine or lysine were not sufficient to yield detectable increases in the levels of total threonine or lysine in the seeds. Expression of the cts/lysC-M4 gene in the seeds resulted in a 4 to 23-fold increase in the level of free threonine in the seeds and a 2 to 3-fold increase in the level of free lysine in some cases. There was a good correlation between transformants expressing higher levels of AKIII protein and those having higher levels of free threonine, but this was again not the case for lysine. The larger increases of free threonine were sufficient to yield detectable increases in the levels of total threonine in the seeds. Sixteen to twenty-five percent increases in total threonine content of the seeds were observed in three lines which were sampled multiple times. (Isoleucine to leucine ratios are shown for comparison.) The lines that showed increased total threonine were the same ones the showed the highest levels of increase in free threonine and high expression of the AKIII-M4 protein. From these results it can be estimated that free threonine represents about 1% of the total threonine present in a normal tobacco seed, but about 18% of the total threonine present in seeds expressing high levels of AKIII-M4.

**TABLE 3**

| BT548 Transformants: phaseolin 5' region/cts/lysC/phaseolin 3' BT549 Transformants: phaseolin 5' region/cts/lysC-M4/phaseolin 3' | | | | | | | |
|---|---|---|---|---|---|---|---|
| | SEED FREE AMINO ACID | | | SEED TOTAL AMINO ACID | | | |
| LINE | K/L | T/L | I/L | K/L | T/L | I/L | WESTERN |
| NORMAL | 0.49 | 1.34 | 0.68 | 0.35 | 0.68 | 0.63 | - |
| 548-2A | 1.15 | 2.3 | 0.78 | 0.43 | 0.71 | 0.67 | + |
| 548-4D | 0.69 | 5.3 | 0.80 | 0.35 | 0.69 | 0.65 | +++ |
| 548-6A | 0.39 | 3.5 | 0.85 | 0.35 | 0.69 | 0.64 | + |
| 548-7A | 0.82 | 4.2 | 0.83 | 0.36 | 0.68 | 0.65 | ++ |
| 548-14A | 0.41 | 3.1 | 0.82 | 0.32 | 0.67 | 0.65 | + |
| 548-18A | 0.51 | 1.5 | 0.69 | 0.37 | 0.67 | 0.63 | - |
| 548-22A | 1.41 | 2.9 | 0.75 | 0.47 | 0.74 | 0.65 | +++ |
| 548-24A | 0.73 | 3.7 | 0.81 | 0.38 | 0.68 | 0.65 | ++ |
| 548-41A | 0.40 | 2.8 | 0.77 | 0.37 | 0.68 | 0.65 | + |
| 548-50A | 0.46 | 4.0 | 0.81 | 0.33 | 0.68 | 0.65 | + |
| 548-57A | 0.50 | 3.8 | 0.80 | 0.33 | 0.67 | 0.65 | ++ |
| | | | | | | | |
| 549-5A | 0.63 | 5.9 | 0.69 | 0.32 | 0.65 | 0.65 | + |
| 549-7A | 0.51 | 8.3 | 0.78 | 0.33 | 0.67 | 0.63 | ++ |
| 549-20A | 0.67 | 30 | 0.88 | 0.38* | 0.82* | 0.65* | ++++ |
| 549-34A | 0.43 | 1.3 | 0.69 | 0.32 | 0.64 | 0.63 | - |
| 549-39D | 0.83 | 16 | 0.83 | 0.35 | 0.71 | 0.63 | +++ |
| 549-40A | 0.80 | 4.9 | 0.74 | 0.33 | 0.63 | 0.64 | + |
| 549-41C | 0.99 | 13 | 0.80 | 0.38* | 0.79* | 0.65* | +++ |
| 549-46A | 0.48 | 7.7 | 0.84 | 0.34 | 0.70 | 0.64 | + |
| 549-52A | 0.81 | 9.2 | 0.80 | 0.39 | 0.70 | 0.65 | ++ |
| 549-57A | 0.60 | 15 | 0.77 | 0.35* | 0.85* | 0.64* | +++ |
| 549-60D | 0.85 | 11 | 0.79 | 0.37 | 0.73 | 0.65 | ++ |
| NORMAL WAS CALCULATED AS THE AVERAGE OF 6 SAMPLES FOR FREE AMINO ACID AND 23 SAMPLES FOR TOTAL AMINO ACIDS. | | | | | | | |

| | | | | | | | |
|---|---|---|---|---|---|---|---|
| * INDICATES AVERAGE OF AT LEAST 5 SAMPLES | | | | | | | |

Seeds derived from self-pollination of two plants transformed with the phaseolin 5' region/cts/lysC-M4/phaseolin 3' region, plants 549-5A and 549-40A, showed 3 kanamycin resistant to 1 kanamycin sensitive seedlings, indicative of a single site of insertion of the transgene. Progeny plants were grown, self-pollinated and seed was analyzed for segregation of the kanamycin marker gene. Progeny plants that were homozygous for the transgene insert, thus containing two copies of the gene cassette, accumulated approximately 2 times as much threonine in their seed as their sibling heterozygous progeny with one copy of the gene cassette and about 8 times as much as seed without the gene. This demonstrates that the level of expression of the E. coli enzyme controls the accumulation of free threonine.

### EXAMPLE 9

### Transformation of Tobacco with the 35S Promoter/ecodapA Chimeric Genes

The 35S promoter/Cab leader/ecodapA/Nos 3' and 35S promoter/Cab leader/cts/ecodapA/Nos 3', chimeric genes were isolated as 3.1, and 3.3 kb BamH I-Sal I fragments, respectively and inserted into BamH I-Sal I digested binary vector pZS97K (Figure 2), yielding plasmids pBT462 and pBT463, respectively. The binary vector is described in Example 7.

The binary vectors containing the chimeric ecodapA genes were transferred by tri-parental matings to Agrobacterium strain LBA4404/pAL4404, the Agrobacterium transformants used to inoculate tobacco leaf disks and the resulting transgenic plants regenerated by the methods set out in Example 7.

To assay for expression of the chimeric genes in leaves of the transformed plants, protein was extracted as described in Example 7, with the following modifications. The supernatant from the first ammonium sulfate precipitation, approximately 18mL, was mixed with an additional 12mL of cold, saturated ammonium sulfate. The mixture was set on ice for 30 min and centrifuged as described in Example 7. The supernatant was decanted and the pellet, which contained the DHDPS protein, was resuspended in 1 mL of TNE and desalted by passage over a Sephadex G-25 M column (Column PD-10, Pharmacia).

The leaf extracts were assayed for DHDPS activity as described in Example 4. E. coli DHDPS could be distinguished from tobacco DHDPS activity by its increased resistance to lysine; E. coli DHDPS retained 80-90% of its activity at 0.1mM lysine, while tobacco DHDPS was completely inhibited at that concentration of lysine. One of ten transformants containing the chimeric gene, 35S promoter/Cab leader/ecodapA/Nos 3', showed E. coli DHDPS expression, while five of ten transformants containing the chimeric gene, 35S promoter/Cab leader/cts/ecodapA/Nos 3' showed E. coli DHDPS expression.

Free amino acids were extracted from leaves as described in Example 7. Expression of the chimeric gene, 35S promoter/Cab leader/cts/ecodapA/Nos 3', but not 35S promoter/Cab leader/ecodapA/Nos 3' resulted in substantial increases in the level of free lysine in the leaves. Free lysine levels from two to 90-fold higher than untransformed tobacco were observed.

The transformed plants were allowed to flower, self-pollinate and go to seed. Seeds from several lines transformed with the 35S promoter/Cab leader/cts/ecodapA/Nos 3' gene were surface sterilized and germinated on agar plates in the presence of kanamycin. Lines that showed 3 kanamycin resistant to 1 kanamycin sensitive seedlings, indicative of a single site of insertion of the transgenes, were identified. Progeny that were homozygous for the transgene insert were obtained from these lines using standard genetic analysis. The homozygous progeny were then characterized for expression of E. coli DHDPS in young and mature leaves and for the levels of free amino acids accumulated in young and mature leaves and in mature seeds.

Expression of active E. coli DHDPS enzyme was clearly evident in both young and mature leaves of the homozygous progeny of the transformants (Table 4). High levels of free lysine, 50 to 100-fold higher than normal tobacco plants, accumulated in the young leaves of the plants, but a much smaller accumulation of free lysine (2 to 8-fold) was seen in the larger leaves. Experiments that measure lysine in the phloem suggest that lysine is exported from the large leaves. This exported lysine may contribute to the accumulation of lysine in the small growing leaves, which are known to take up, rather than export nutrients. Since the larger leaves make up the major portion of the biomass of the plant, the total increased accumulation of lysine in the plant is more influenced by the level of lysine in the larger leaves. No effect on the free lysine levels in the seeds of these plants was observed (Table 4).

**TABLE 4**

| Progeny of BT463 transformants homozygous for 35S promoter/Cab leader/cts/ecodapA/Nos 3' | | | | | |
|---|---|---|---|---|---|
| | LEAF | LEAF FREE AMINO ACID | | E. COLI DHDPS | SEED FREE AMINO ACID |
| LINE | SIZE | K/L | K/TOT | OD/60'/mg | K/L |
| NORMAL | 3 in. | 0.5 | 0.006 | 0 | 0.5 |
| 463-18C-2 | 3 in. | 47 | 0.41 | 7.6 | 0.4 |
| 463-18C-2 | 12 in. | 1 | 0.02 | 5.5 | --- |
| 463-25A-4 | 3 in. | 58 | 0.42 | 6.6 | 0.4 |
| 463-25A-4 | 12 in. | 4 | 0.02 | 12.2 | --- |
| 463-38C-3 | 3 in. | 28 | 0.28 | 6.1 | 0.5 |
| 463-38C-3 | 12 in. | 2 | 0.04 | 8.3 | --- |

### EXAMPLE 10

### Transformation of Tobacco with the Phaseolin Promoter/ecodapA Chimeric Genes

The chimeric gene cassettes, phaseolin 5' region/ecodapA/phaseolin 3' region, and phaseolin 5' region/cts/ecodapA/phaseolin 3' region (Example 6) were isolated as approximately 2.6 and 2.8 kb Hind III fragments, respectively. These fragments were inserted into the unique Hind III site of the binary vector pZS97 (Figure 3), yielding pBT506 and pBT534, respectively. This vector is described in Example 8.

The binary vectors containing the chimeric ecodapA genes were transferred by tri-parental matings to Agrobacterium strain LBA4404/pAL4404, the Agrobacterium transformants used to inoculate tobacco leaf disks and the resulting transgenic plants were regeneratedby the methods set out in Example 7.

To assay for expression of the chimeric genes, the transformed plants were allowed to flower, self-pollinate and go to seed. Total seed proteins were extracted as described in Example 8 and immunologically analyzed as described in Example 7, with the following modification. The Western blot membranes were exposed to the DHDPS antibodies prepared in Example 4 at a 1:5000 dilution of the rabbit serum using standard protocol provided by BioRad with their Immun-Blot Kit.

Thirteen of fourteen transformants containing the chimeric gene, phaseolin 5' region/ecodapA/phaseolin 3' region and nine of thirteen transformants containing the chimeric gene, phaseolin 5' region/cts/ecodapA/phaseolin 3' region, produced DHDPS protein detectable via Western blotting (Table 3). Protein which reacted with the DHDPS antibody was of several sizes. Most of the protein was equal in size to DHDPS produced in E. coli, whether or not the chimeric gene included the chloroplast transit sequence. This indicated that the chloroplast targeting signal had been efficiently removed from the precursor protein synthesized. This further suggests the majority of the protein entered the chloroplast. In addition, some proteins of lower molecular weight were observed, probably representing breakdown products of the DHDPS polypeptide.

To measure free amino acid composition and total amino acid composition of the seeds, free amino acids and total amino acids were extracted from mature seeds and analyzed as described in Example 8. Expression of either the ecodapA gene or cts/ecodapA had no effect on the total lysine or threonine composition of the seeds in any of the transformed lines (Table 5). Several of the lines that were transformed with the phaseolin 5' region/cts/ecodasA/phaseolin 3' chimeric gene were also tested for any effect on the free amino acid composition. Again, not even a modest effect on the lysine or threonine composition of the seeds was observed in lines expressing high levels of E. coli DHDPS protein (Table 5). This was a surprising result, given the dramatic effect (described in Example 9) that expression of this protein has on the free lysine levels in leaves.

One possible explanation for this was that the DHDPS protein observed via Western blot was not functional. To test this hypothesis, total protein extracts were prepared from mature seeds and assayed for DHDPS activity. Approximately 30-40 mg of seeds were put into a 1.5 mL disposable plastic microfuge tube and ground in 0.25mL of 50mM Tris-HCl, 50mM NaCl, 1mM EDTA (TNE). The grinding was done using a motorized grinder with disposable plastic shafts designed to fit into the microfuge tube. The resultant suspensions were centrifuged for 5 min at room temperature in a microfuge to remove particulates. Approximately 0.1 mL of aqueous supernatant was removed between the pelleted material and the upper oil phase. The seed extracts were assayed for DHDPS activity as described in Example 4. E. coli DHDPS could be distinguished from tobacco DHDPS activity by its increased resistance to lysine; E. coli DHDPS retained about 50% of its activity at 0.4mM lysine, while tobacco DHDPS was completely inhibited at that concentration of lysine. High levels of E. coli DHDPS activity were seen in all four seed extracts tested eliminating this explanation.

The presence of the cts sequence in the chimeric ecodapA gene was essential for eliciting accumulation of high levels of lysine in leaves. Thus another possible explanation was that the cts sequence had somehow been lost during the insertion of the chimeric phaseolin 5' region/cts/ecodapA/phaseolin 3' gene into the binary vector. PCR analysis of several of the transformed lines demonstrated the presence of the cts sequence, however, ruling out this possibility.

A third explanation was that amino acids are not normally synthesized in seeds, and therefore the other enzymes in the pathway were not present in the seeds. The results of experiments presented in Example 8, wherein expression of phaseolin 5' region/cts/lysC-M4/phaseolin 3' gene resulted in accumulation of high levels of free threonine in seeds, indicate that this is not the case.

Taken together these results and the results presented in Example 9, demonstrate that expression of a lysine-insensitive DHDPS in either seeds or leaves is not sufficient to achieve accumulation of increased free lysine in seeds.

**TABLE 5**

| BT506 Transformants: phaseolin 5' region/ecodapA /phaseolin 3' BT534 Transformants: phaseolin 5' region/cts/ecodapA/phaseolin 3' | | | | | | |
|---|---|---|---|---|---|---|
| | SEED: FREE AMINO ACIDS | | SEED: TOTAL AMINO ACIDS | | E. COLI DHDPS | |
| LINE | K/L | | K/L | T/L | OD/60'/MG | WESTERN |
| NORMAL | 0.49 | 1.34 | 0.35 | 0.68 | | |
| 506-2B | | | 0.34 | 0.66 | | + |
| 506-4B | | | 0.33 | 0.67 | | + |
| 506-16A | | | 0.34 | 0.67 | | + |
| 506-17A | | | 0.36 | 0.55 | 7.7 | +++ |
| 506-19A | | | 0.37 | 0.45 | | ++ |
| 506-22A | | | 0.34 | 0.67 | | ++ |
| 506-23B | | | 0.35 | 0.67 | | ++ |
| 506-33B | | | 0.34 | 0.67 | | ++ |
| 506-38B | | | 0.36 | 0.69 | 8.7 | +++ |
| 506-39A | | | 0.37 | 0.70 | | ++ |
| 506-40A | | | 0.36 | 0.68 | | - |
| 506-47A | | | 0.32 | 0.68 | | +++ |
| 506-48A | | | 0.33 | 0.69 | | +++ |
| 506-49A | | | 0.33 | 0.69 | | +++ |
| | | | | | | |
| 534-8A | | | 0.34 | 0.66 | | - |
| 534-9A | | | 0.36 | 0.67 | | ++ |
| 534-22B | 0.43 | 1.32 | 0.39 | 0.51 | 4.9 | +++ |
| 534-31A | | | 0.34 | 0.66 | | - |
| 534-38A | 0.35 | 1.49 | 0.42 | 0.33 | | +++ |
| 534-39A | | | 0.38 | 0.69 | | + |
| 534-7A | | | 0.34 | 0.67 | | +++ |
| 534-25B | | | 0.35 | 0.67 | | +++ |
| 534-34B | 0.80 | 1.13 | 0.42 | 0.70 | | - |
| 534-35A | 0.43 | 1.18 | 0.33 | 0.67 | | +++ |
| 534-37B | 0.42 | 1.58 | 0.37 | 0.68 | | - |
| 534-43A | | | 0.35 | 0.68 | | +++ |
| 534-48A | 0.46 | 1.24 | 0.35 | 0.68 | 6.2 | +++ |

### EXAMPLE 11

### Transformation of Tobacco with the 35S Promoter/cts/dapA plus 35S Promoter/cts/lysC-M4 Chimeric Genes

The 35S promoter/Cab leader/cts/ecodapA/Nos 3', and 35S promoter/Cab leader/cts/lysC-M4/Nos 3' chimeric genes were combined in the binary vector pZS97K (Figure 2). The binary vector is described in Example 7. An oligonucleotide adaptor was synthesized to convert the BamH I site at the 5' end of the 35S promoter/Cab leader/cts/lysC-M4/Nos 3' chimeric gene (see Figure 1) to an EcoR I site. The 35S promoter/Cab leader/cts/lysC-M4/Nos 3' chimeric gene was then isolated as a 3.6 kb EcoR I fragment from plasmid pBT540 (Example 6) and inserted into pBT463 (Example 9) digested with EcoR I, yielding plasmid pBT564. This vector has both the 35S promoter/Cab leader/cts/ecodapA/Nos 3', and 35S promoter/Cab leader/cts/lysC-M4/Nos 3' chimeric genes inserted in the same orientation.

The binary vector containing the chimeric ecodapA and lysC-M4 genes was transferred by tri-parental matings to Agrobacterium strain LBA4404/pAL4404, the Agrobacterium transformants used to inoculate tobacco leaf disks and the resulting transgenic plants regenerated by the methods set out in Example 7.

To assay for expression of the chimeric genes in leaves of the transformed plants, protein was extracted as described in Example 7 for AKIII, and as described in Example 9 for DHDPS. The leaf extracts were assayed for DHDPS activity as described in Examples 4 and 9. E. coli DHDPS could be distinguished from tobacco DHDPS activity by its increased resistance to lysine; E. coli DHDPS retained 80-90% of its activity at 0.1mM lysine, while tobacco DHDPS was completely inhibited at that concentration of lysine. Extracts were characterized immunologically for expression of AKIII and DHDPS proteins via Western blots as described in Examples 7 and 10.

Ten of twelve transformants expressed E. coli DHDPS enzyme activity (Table 6). There was a good correlation between the level of enzyme activity and the amount of DHDPS protein detected immunologically. As described in Example 7, the AK assay was not sensitive enough to detect enzyme activity in these extracts. However, AKIII-M4 protein was detected immunologically in eight of the twelve extracts. In some transformants, 564-21A and 47A, there was a large disparity between the level of expression of DHDPS and AKIII-M4, but in 10 of 12 lines there was a good correlation.

Free amino acids were extracted from leaves and analyzed for amino acid composition as described in Example 7. In the absence of significant AKIII-M4, the level of expression of the chimeric gene, 35S promoter/Cab leader/cts/ecodapA/Nos 3' determined the level of lysine accumulation (Table 6). Compare lines 564-21A, 47A and 39C, none of which expresses significant AKIII-M4. Line 564-21A accumulates about 10-fold higher levels of lysine than line 564-47A which expresses a lower level of E. coli DHDPS and 40-fold higher levels of lysine than 564-39C which expresses no E. coli DHDPS. However, in transformants that all expressed similar amounts of E. coli DHDPS (564-18A, 56A, 36E, 55B, 47A), the level of expression of the chimeric gene, 35S promoter/Cab leader/cts/lysC-M4/Nos 3', controlled the level of lysine accumulation. Thus it is clear that although expression of 35S promoter/Cab leader/cts/lysC-M4/Nos 3' has no effect on the free amino acid levels of leaves when expressed alone (see Example 7), it can increase lysine accumulation when expressed in concert with the 35S promoter/Cab leader/cts/ecodapA/Nos 3' chimeric gene. Expression of these genes together did not effect the level of any other free amino acid in the leaves.

**TABLE 6**

| BT564 Transformants: 35S promoter/Cab leader/cts/ecodapA/Nos 3' 35S promoter/Cab leader/cts/lysC-M4/Nos 3' | | | | | | | |
|---|---|---|---|---|---|---|---|
| | | | | | E. COLI | | |
| LINE | FREE AA LEAF nmol/4mg | | FREE AA LEAF | | DHDPS U/MG/HR | WESTERN DHDPS | WESTERN AK-III |
| | TOT | K | K/L | K/TOT | | | |
| 564-21A | 117 | 57 | 52 | 0.49 | 2.4 | +++ | +/- |
| 564-18A | 99 | 56 | 69 | 0.57 | 1.1 | ++ | ++ |
| 564-56A | 104 | 58 | 58 | 0.56 | 1.5 | ++ | ++ |
| 564-36E | 85 | 17 | 17 | 0.20 | 1.5 | ++ | +++ |
| 564-55B | 54 | 5 | 9.1 | 0.10 | 1.0 | ++ | + |
| 564-47A | 18 | 1 | 4.8 | 0.06 | 0.8 | ++ | - |
| 564-35A | 37 | 7 | 13 | 0.18 | 0.3 | + | ++ |
| 564-60D | 61 | 3 | 4.5 | 0.06 | 0.2 | + | ++ |
| 564-45A | 46 | 4 | 8.1 | 0.09 | 0.4 | + | + |
| 564-44B | 50 | 1 | 1.7 | 0.02 | 0.1 | +/- | - |
| 564-49A | 53 | 1 | 1.0 | 0.02 | 0 | +/- | - |
| 564-39C | 62 | 1 | 1.4 | 0.02 | 0 | - | - |

Free amino acids were extracted from mature seeds derived from self-pollinated plants and quantitated as described in Example 8. There was no significant difference in the free amino acid content of seeds from untransformed plants compared to that from the plants showing the highest free lysine accumulation in leaves, i.e. plants 564-18A, 564-21A, 564-36E, 564-56A.

### EXAMPLE 12

### Transformation of Tobacco with the Phaseolin Promoter/cts/ecodapA plus Phaseolin Promoter/cts/lysC-M4 Chimeric Genes

The chimeric gene cassettes, phaseolin 5'region/cts/ecodapA/phaseolin 3' region and phaseolin 5' region/cts/lysC-M4/phaseolin 3' (Example 6) were combined in the binary vector pZS97 (Figure 3). The binary vector is described in Example 8. To accomplish this the phaseolin 5' region/cts/ecodapA/phaseolin 3' chimeric gene was isolated as a 2.7 kb Hind III fragment and inserted into the Hind III site of vector pUC1318 [Kay et al (1987) Nucleic Acids Res. 6:2778], yielding pBT568. It was then possible to digest pBT568 with BamH I and isolate the chimeric gene on a 2.7 kb BamH I fragment. This fragment was inserted into BamH I digested pBT549 (Example 8), yielding pBT570. This binary vector has both chimeric genes, phaseolin 5' region/cts/ecodapA/phaseolin 3' gene and phaseolin 5' region/cts/lysC-M4/phaseolin 3' inserted in the same orientation.

The binary vector pBT570 was transferred by tri-parental mating to Agrobacterium strain LBA4404/pAL4404, the Agrobacterium transformants used to inoculate tobacco leaf disks and the resulting transgenic plants regenerated by the methods set out in Example 7.

To assay for expression of the chimeric genes in the seeds of the transformed plants, the plants were allowed to flower, self-pollinate and go to seed. Total proteins were extracted from mature seeds and analyzed via western blots as described in Example 8.

Twenty-one of twenty-five transformants expressed the DHDPS protein and nineteen of these also expressed the AKIII protein (Table 7). The amounts of the proteins expressed were related to the number of gene copies present in the transformants; the highest expressing lines, 570-4B, 570-12C, 570-59B and 570-23B, all had two or more sites of insertion of the gene cassette based on segregation of the kanamycin marker gene. Enzymatically active E. coli DHDPS was observed in mature seeds of all the lines tested wherein the protein was detected.

To measure free amino acid composition of the seeds, free amino acids were extracted from mature seeds and analyzed as described in Example 8. There was a good correlation between transformants expressing higher levels of both DHDPS and AKIII protein and those having higher levels of free lysine and threonine. The highest expressing lines (marked by asterisk in Table 7) showed up to a 2-fold increase in free lysine levels and up to a 4-fold increase in the level of free threonine in the seeds.

In the highest expressing lines it was possible to detect a high level of α-aminoadipic acid. This compound is known to be an intermediate in the catabolism of lysine in cereal seeds, but is normally detected only via radioactive tracer experiments due to its low level of accumulation. The build-up of high levels of this intermediate indicates that a large amount of lysine is being produced in the seeds of these transformed lines and is passing through the catabolic pathway. The build-up of α-aminoadipic acid was not observed in transformants expressing only E. coli DHDPS or only AKIII-M4 in seeds. These results show that it is necessary to express both enzymes simultaneously to produce high levels of free lysine.

**TABLE 7**

| BT570 Transformants: phaseolin 5'region/cts/lysC-M4/phaseolin 3' region phaseolin 5'region/cts/ecodapA/phaseolin 3' region | | | | | | | | |
|---|---|---|---|---|---|---|---|---|
| | FREE AMINO ACIDS/SEED | | TOTAL AMINO ACIDS/SEED | | WESTERN E. COLI | WESTERN E. COLI | E. COLI DHDPS | Progeny |
| LINE | K/L | T/L | K/L | T/L | DHDPS | AKIII | U/MG/HR | Kan^{r}:Kan^{s} |
| NORMAL | 0.49 | 1.3 | 0.35 | 0.68 | - | - | | |
| 570-4B | 0.31 | 2.6 | 0.34 | 0.64 | +++ | ++ | | 15:1 |
| 570-7C | 0.39 | 2.3 | 0.34 | 0.64 | ++ | + | | |
| 570-8B | 0.29 | 2.1 | 0.34 | 0.63 | + | - | | |
| 570-12C* | 0.64 | 5.1 | 0.36 | 0.68 | ++++ | ++++ | > 4.3 | >15:1 |
| 570-18A | 0.33 | 3.0 | 0.35 | 0.65 | ++ | ++ | | 15:1 |
| 570-24A | 0.33 | 2.0 | 0.34 | 0.65 | ++ | - | | |
| 570-37A | 0.33 | 2.1 | 0.34 | 0.64 | +/- | +/- | | |
| 570-44A | 0.29 | 2.1 | 0.34 | 0.64 | ++ | + | | |
| 570-46B | 0.41 | 2.1 | 0.35 | 0.65 | ++ | + | | |
| 570-51B | 0.33 | 1.5 | 0.33 | 0.64 | - | - | 0 | |
| 570-59B* | 0.46 | 3.0 | 0.35 | 0.65 | +++ | +++ | 2.6 | >15:1 |
| 570-80A | 0.31 | 2.2 | 0.34 | 0.64 | ++ | + | | |
| 570-11A | 0.28 | 2.3 | 0.34 | 0.67 | ++ | ++ | | 3:1 |
| 570-17B | 0.27 | 1.6 | 0.34 | 0.65 | - | - | | |
| 570-20A | 0.41 | 2.3 | 0.35 | 0.67 | ++ | + | | |
| 570-21B | 0.26 | 2.4 | 0.34 | 0.68 | ++ | + | | |
| 570-23B* | 0.40 | 3.6 | 0.34 | 0.68 | +++ | +++ | 3.1 | 63:1 |
| 570-25D | 0.30 | 2.3 | 0.35 | 0.66 | ++ | +/- | | |
| 570-26A | 0.28 | 1.5 | 0.34 | 0.64 | - | - | | |
| 570-32A | 0.25 | 2.5 | 0.34 | 0.67 | ++ | + | | |
| 570-35A | 0.25 | 2.5 | 0.34 | 0.63 | ++ | ++ | | 3:1 |
| 570-38A-1 | 0.25 | 2.6 | 0.34 | 0.64 | ++ | ++ | | 3:1 |
| 570-38A-3 | 0.33 | 1.6 | 0.35 | 0.63 | - | - | | |
| 570-42A | 0.27 | 2.5 | 0.34 | 0.62 | ++ | ++ | | 3:1 |
| 570-45A | 0.60 | 3.4 | 0.39 | 0.64 | ++ | ++ | | 3:1 |

| | | | | | | | | |
|---|---|---|---|---|---|---|---|---|
| * indicates free amino acid sample has α-aminoadipic acid | | | | | | | | |

### EXAMPLE 13

### Use of the cts/lysC-M4 Chimeric Gene as a Selectable Marker for Tobacco Transformation

The 35S promoter/Cab leader/cts/lysC-M4/Nos 3' chimeric gene in the binary vector pZS97K (pBT542, see Example 7) was used as a selectable genetic marker for transformation of tobacco. High concentrations of lysine plus threonine inhibit growth of shoots from tobacco leaf disks. Expression of active lysine and threonine insensitive AKIII-M4 reverses this growth inhibition (see Example 7).

The binary vector pBT542 was transferred by tri-parental mating to Agrobacterium strain LBA4404/pAL4404, the Agrobacterium transformants used to inoculate tobacco leaf disks and the resulting transformed shoots were selected on shooting medium containing 3mM lysine plus 3mM threonine. Shoots were transferred to rooting media containing 3mM lysine plus 3mM threonine. Plants were grown from the rooted shoots. Leaf disks from the plants were placed on shooting medium containing 3mM lysine plus 3mM threonine. Transformed plants were identified by the shoot proliferation which occurred around the leaf disks on this medium.

### EXAMPLE 14

### Transformation of Tobacco with the 35S Promoter/cts/cordapA Chimeric Gene

The 35S promoter/Cab leader/cts/cordapA/Nos 3' chimeric gene was isolated as a 3.0 kb BamH I-Sal I fragment and inserted into BamH I-Sal I digested binary vector pZS97K (Figure 2), yielding plasmid pFS852. The binary vector is described in Example 7.

The binary vector containing the chimeric cordapA gene was transferred by tri-parental mating to Agrobacterium strain LBA4404/pAL4404, the Agrobacterium transformant was used to inoculate tobacco leaf disks and the resulting transgenic plants were regenerated by the methods set out in Example 7.

To assay for expression of the chimeric gene in leaves of the transformed plants, protein was extracted as described in Example 7, with the following modifications. The supernatant from the first ammonium sulfate precipitation, approximately 18mL, was mixed with an additional 12mL of cold, saturated ammonium sulfate. The mixture was set on ice for 30 min and centrifuged as described in Example 7. The supernatant was decanted and the pellet, which contained the DHDPS protein, was resuspended in 1 mL of TNE and desalted by passage over a Sephadex G-25 M column (Column PD-10, Pharmacia).

The leaf extracts were assayed for DHDPS protein and enzyme activity as described in Example 4. Corynebacteria DHDPS enzyme activity could be distinguished from tobacco DHDPS activity by its insensitivity to lysine inhibition. Eight of eleven transformants showed Corynebacteria DHDPS expression, both as protein detected via western blot and as active enzyme.

Free amino acids were extracted from leaves as described in Example 7. Expression of Corynebacteria DHDPS resulted in large increases in the level of free lysine in the leaves (Table 8). However, there was not a good correlation between the level of expression of DHDPS and the amount of free lysine accumulated. Free lysine levels from 2 to 50-fold higher than untransformed tobacco were observed. There was also a 2 to 2.5-fold increase in the level of total leaf lysine in the lines that showed high levels of free lysine.

**TABLE 8**

| FS586 transformants: 35S promoter/Cab leader/cts/cordapA/Nos 3' | | | | |
|---|---|---|---|---|
| | FREE AMINO ACIDS/LEAF | TOTAL AMINO ACIDS/LEAF | WESTERN CORYNE. | CORYNE. DHDPS |
| LINE | K/L | K/L | DHDPS | U/MG/HR |
| NORMAL | 0.5 | 0.8 | - | - |
| FS586-2A | 1.0 | 0.8 | - | - |
| FS586-4A | 0.9 | 0.8 | + | 6.1 |
| FS586-11B | 3.6 | 0.8 | + | 3.4 |
| FS586-11D | 26 | 2.0 | + | 3.5 |
| FS586-13A | 2.4 | 0.8 | + | 3.5 |
| FS586-19C | 5.1 | 0.8 | + | 3.1 |
| FS586-22B | >15 | 1.5 | + | 2.3 |
| FS586-30B | | 0.8 | - | - |
| FS586-38B | 18 | 1.5 | ++ | 3.9 |
| FS586-51A | 1.3 | 0.8 | - | - |
| FS586-58C | 1.2 | 0.8 | + | 5.1 |

The plants were allowed to flower, self-pollinate and go to seed. Mature seed was harvested and assayed for free amino acid composition as described in Example 8. There was no difference in the free lysine content of the transformants compared to untransformed tobacco seed.

### EXAMPLE 15

### Transformation of Tobacco with the KTI3 promoter/cts/cordapA or Phaseolin Promoter/cts/cordapA plus Phaseolin Promoter/cts/lysC-M4 Chimeric Genes

The chimeric gene cassettes, KTI3 5' region/cts/cordapA/KTI3 3' region and phaseolin 5' region/cts/lysC-M4/phaseolin 3' as well as phaseolin 5' region/cts/cordapA/phaseolin 3' region and phaseolin 5' region/cts/lysC-M4/phaseolin 3' (Example 6) were combined in the binary vector pZS97 (Figure 3). The binary vector is described in Example 8.

To accomplish this the KTI3 5' region/cts/cordapA/KTI3 3' region chimeric gene cassette was isolated as a 3.3 kb BamH I fragment and inserted into BamH I digested pBT549 (Example 8), yielding pFS883. This binary vector has the chimeric genes, KTI3 5' region/cts/cordapA/KTI3 3' region and phaseolin 5' region/cts/lysC-M4/phaseolin 3' region inserted in opposite orientations.

The phaseolin 5' region/cts/cordapA/phaseolin 3'region chimeric gene cassette was modified using oligonucleotide adaptors to convert the Hind III sites at each end to BamH I sites. The gene cassette was then isolated as a 2.7 kb BamH I fragment and inserted into BamH I digested pBT549 (Example 8), yielding pFS903. This binary vector has both chimeric genes, phaseolin 5' region/cts/cordapA/phaseolin 3' region and phaseolin 5' region/cts/lysC-M4/phaseolin 3' region inserted in the same orientation.

The binary vectors pFS883 and pFS903 were transferred by tri-parental mating to Agrobacterium strain LBA4404/pAL4404, the Agrobacterium transformants were used to inoculate tobacco leaf disks and the resulting transgenic plants were regenerated by the methods set out in Example 7.

To assay for expression of the chimeric genes in the seeds of the transformed plants, the plants were allowed to flower, self-pollinate and go to seed. Total proteins were extracted from mature seeds and analyzed via western blots as described in Example 8.

Twenty-one of twenty-two transformants tested expressed the DHDPS protein and eighteen of these also expressed the AKIII protein (Table 8). Enzymatically active Corynebacteria DHDPS was observed in mature seeds of all the lines tested wherein the protein was detected except one.

To measure free amino acid composition of the seeds, free amino acids were extracted from mature seeds and analyzed as described in Example 8. There was a good correlation between transformants expressing higher levels of both DHDPS and AKIII protein and those having higher levels of free lysine and threonine. The highest expressing lines showed up to a 3-fold increase in free lysine levels and up to a 8-fold increase in the level of free threonine in the seeds. As was described in Example 12, a high level of α-aminoadipic acid, indicative of lysine catabolism, was observed in many of the transformed lines (indicated by asterisk in Table 9). There was no major difference in the free amino acid composition or level of protein expression between the transformants which had the KTI3 or Phaseolin regulatory sequences driving expression of the Corynebacteria DHDPS gene.

**TABLE 9**

| FS883 Transformants: phaseolin 5' region/cts/lysC-M4/phaseolin 3' KTI3 5' region/cts/cordapA/KTI3 3' FS903 Transformants: phaseolin 5' region/cts/lysC-M4/phaseolin 3' phaseolin 5' region/cts/cordapA/phaseolin 3' | | | | | | |
|---|---|---|---|---|---|---|
| | FREE AMINO ACIDS/SEED | | WESTERN CORYNE. | WESTERN E. COLI | CORYNE. DHDPS | Progeny |
| LINE | K/L | T/L | DHDPS | AKIII | U/MG/HR | Kan^{r}:Kan^{s} |
| NORMAL | 0.5 | 1.3 | - | - | | |
| FS883-4A | 0.9 | 4.0 | + | + | | >15:1 |
| FS883-11A | 1.0 | 3.5 | ++ | ++ | 3.1 | 3:1 |
| FS883-14B | 0.5 | 2.5 | ++ | ++ | | |
| FS883-16A* | 0.7 | 10.5 | + | +++ | 0 | |
| FS883-17A* | 1.0 | 5.0 | +++ | +++ | 7.0 | |
| FS883-18C* | 1.2 | 3.5 | ++ | + | 5.8 | 3:1 |
| FS883-21A | 0.5 | 1.5 | + | +/- | | |
| FS883-26B* | 1.1 | 3.6 | ++ | ++ | 2.4 | |
| FS883-29B | 0.5 | 1.5 | + | - | 0.4 | |
| FS883-32B | 0.7 | 2.4 | ++ | + | 1.5 | 3:1 |
| FS883-38B* | 1.1 | 11.3 | + | ++ | 2.0 | |
| FS8B3-59C* | 1.4 | 6.1 | + | + | 0.5 | 15:1 |
| FS903-3C | 0.5 | 1.8 | + | +++ | | |
| FS903-8A* | 0.8 | 2.1 | +++ | ++++ | | |
| FS903-9B | 0.6 | 1.8 | ++ | ++ | 4.3 | |
| FS903-10A | 0.5 | 1.5 | - | - | | |
| FS903-22F | 0.5 | 1.8 | ++ | ++ | 0.9 | |
| FS903-35B* | 0.8 | 2.1 | ++ | ++ | | |
| FS903-36B | 0.7 | 1.5 | + | - | | |
| FS903-40A | 0.6 | 1.8 | + | + | | |
| FS903-41A* | 1.2 | 2.0 | ++ | +++ | | |
| FS903-42A | 0.7 | 2.2 | ++ | +++ | 5.4 | |
| FS903-44C | 0.5 | 1.9 | | | | |
| FS903-53B | 0.6 | 1.9 | | | | |

| | | | | | | |
|---|---|---|---|---|---|---|
| * indicates free amino acid sample has α-aminoadipic acid | | | | | | |

Free amino acid composition and expression of bacterial DHDPS and AKIII proteins was also analyzed in developing seeds of two lines that segregated as single gene cassette insertions (see Table 10). Expression of the DHDPS protein under control of the KTI3 promoter was detected at earlier times than that of the AKIII protein under control of the Phaseolin promoter, as expected. At 14 days after flowering both proteins were expressed at a high level and there was about an 8-fold increase in the level of free lysine compared to normal seeds. These results confirm that simultaneous expression of lysine insensitive DHDPS and lysine-insensitive AK results in the production of high levels of free lysine in seeds. Free lysine does not continue to accumulate to even higher levels, however. In mature seeds free lysine is at a level 2 to 3-fold higher than in normal mature seeds, and the lysine breakdown product α-aminoadipic acid accumulates. These results provide further evidence that lysine catabolism occurs in seeds and prevents accumulation of the high levels of free lysine produced in transformants expressing lysine insensitive DHDPS and lysine insensitive AK.

**TABLE 10**

| Developing seeds of FS883 Transformants: phaseolin 5' region/cts/lysC-M4/phaseolin 3' region KTI3 5' region/cts/cordapA/KTI3 3' region | | | | | |
|---|---|---|---|---|---|
| | DAYS AFTER | FREE AMINO ACIDS/SEED | | WESTERN CORYNE. | WESTERN E. COLI |
| LINE | FLOWERING | K/L | T/L | DHDPS | AKIII |
| FS883-18C | 9 | 1.1 | 2.1 | - | - |
| FS883-18C | 10 | 1.4 | 3.3 | +/- | - |
| FS883-18C | 11 | 1.4 | 2.5 | + | - |
| FS883-18C | 14 | 4.3 | 1.0 | ++ | ++ |
| FS883-18C* | MATURE | 1.2 | 3.5 | +++ | ++ |
| FS883-32B | 9 | 1.3 | 2.9 | + | - |
| FS883-32B | 10 | 1.6 | 2.7 | + | - |
| FS883-32B | 11 | 1.4 | 2.3 | + | - |
| FS883-32B* | 14 | 3.9 | 1.3 | ++ | ++ |
| FS883-32B* | MATURE | 0.7 | 2.4 | +++ | ++ |

| | | | | | |
|---|---|---|---|---|---|
| * indicates free amino acid sample has α-aminoadipic acid | | | | | |

### EXAMPLE 16

### Transformation of Canola with the Phaseolin Promoter/cts/cordapA and Phaseolin Promoter/cts/lysC-M4 Chimeric Genes

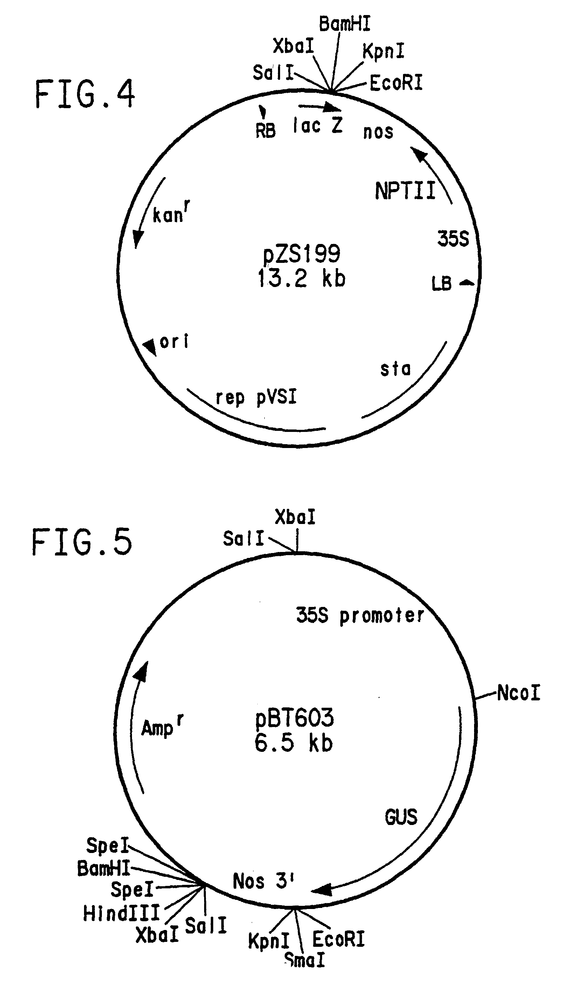

The chimeric gene cassettes, phaseolin 5' region/cts/cordapA/phaseolin 3' region, phaseolin 5' region/cts/lysC-M4/phaseolin 3', and phaseolin 5' region/cts/cordapA/phaseolin 3' region plus phaseolin 5' region/cts/lysC-M4/phaseolin 3' (Example 6) were inserted into the binary vector pZS199 (Figure 4), which is similar to pSZ97K described in Example 8. In pZS199 the 35S promoter from Cauliflower Mosaic Virus replaced the nos promoter driving expression of the NPT II to provide better expression of the marker gene, and the orientation of the polylinker containing the multiple restriction endonuclease sites was reversed.

To insert the phaseolin 5' region/cts/cordapA/phaseolin 3'region 3', the gene cassette was isolated as a 2.7 kb BamH I fragment (as described in Example 15) and inserted into BamH I digested pZS199, yielding plasmid pFS926. This binary vector has the chimeric gene, phaseolin 5' region/cts/cordapA/phaseolin 3' region inserted in the same orientation as the 35S/NPT II/nos 3' marker gene.

To insert the phaseolin 5' region/cts/ lysC-M4/phaseolin 3' region, the gene cassette was isolated as a 3.3 kb EcoR I to Spe I fragment and inserted into EcoR I plus Xba I digested pZS199, yielding plasmid pBT593. This binary vector has the chimeric gene, phaseolin 5' region/cts/lysC-M4/phaseolin 3' region inserted in the same orientation as the 35S/NPT II/nos 3' marker gene.

To combine the two cassettes, the EcoR I site of pBT593 was converted to a BamH I site using oligonucleotide adaptors, the resulting vector was cut with BamH I and the phaseolin 5' region/cts/cordapA/phaseolin 3' region gene cassette was isolated as a 2.7 kb BamH I fragment and inserted, yielding pBT597. This binary vector has both chimeric genes, phaseolin 5' region/cts/cordapA/phaseolin 3' region and phaseolin 5' region/cts/lysC-M4/phaseolin 3' region inserted in the same orientation as the 35S/NPT II/nos 3' marker gene.

Brassica napus cultivar "Westar" was transformed by co-cultivation of seedling pieces with disarmed Agrobacterium tumefaciens strain LBA4404 carrying the the appropriate binary vector.

B. napus seeds were sterilized by stirring in 10% Chlorox, 0.1% SDS for thirty min, and then rinsed thoroughly with sterile distilled water. The seeds were germinated on sterile medium containing 30 mM CaCl2 and 1.5% agar, and grown for six d in the dark at 24°C.

Liquid cultures of Agrobacterium for plant transformation were grown overnight at 28°C in Minimal A medium containing 100 mg/L kanamycin. The bacterial cells were pelleted by centrifugation and resuspended at a concentration of 10⁸ cells/mL in liquid Murashige and Skoog Minimal Organic medium containing 100 uM acetosyringone.

B. napus seedling hypocotyls were cut into 5 mm segments which were immediately placed into the bacterial suspension. After 30 min, the hypocotyl pieces were removed from the bacterial suspension and placed onto BC-35 callus medium containing 100 uM acetosyringone. The plant tissue and Agrobacteria were co-cultivated for three d at 24°C in dim light.

The co-cultivation was terminated by transferring the hypocotyl pieces to BC-35 callus medium containing 200 mg/L carbenicillin to kill the Agrobacteria, and 25 mg/L kanamycin to select for transformed plant cell growth. The seedling pieces were incubated on this medium for three weeks at 24°C under continuous light.

After three weeks, the segments were transferred to BS-48 regeneration medium containing 200 mg/L carbenicillin and 25 mg/L kanamycin. Plant tissue was subcultured every two weeks onto fresh selective regeneration medium, under the same culture conditions described for the callus medium. Putatively transformed calli grew rapidly on regeneration medium; as calli reached a diameter of about 2mm, they were removed from the hypocotyl pieces and placed on the same medium lacking kanamycin

Shoots began to appear within several weeks after transfer to BS-48 regeneration medium. As soon as the shoots formed discernable stems, they were excised from the calli, transferred to MSV-1A elongation medium, and moved to a 16:8-h photoperiod at 24°C.

Once shoots had elongated several internodes, they were cut above the agar surface and the cut ends were dipped in Rootone. Treated shoots were planted directly into wet Metro-Mix 350 soiless potting medium. The pots were covered with plastic bags which were removed when the plants were clearly growing, after about ten d. Results of the transformation are shown in Table 11. Transformed plants were obtained with each of the binary vectors.

Plants were grown under a 16:8-h photoperiod, with a daytime temperature of 23°C and a nightime temperature of 17°C. When the primary flowering stem began to elongate, it was covered with a mesh pollen-containment bag to prevent outcrossing. Self-pollination was facilitated by shaking the plants several times each day. Mature seeds derived from self-pollinations were harvested about three months after planting.

### Minimal A Bacterial Growth Medium

Dissolve in distilled water:
   10.5 g potassium phosphate, dibasic
   4.5 g potassium phosphate, monobasic
   1.0 g ammonium sulfate
   0.5 g sodium citrate, dihydrate
Make up to 979 mL with distilled water
Autoclave
Add 20 mL filter-sterilized 10% sucrose
Add 1 mL filter-sterilized 1 M MgSO4

### Brassica callus Medium BC-35

Per liter:
Murashige and Skoog Minimal Organic Medium (MS salts, 100 mg/L i-inositol, 0.4 mg/L thiamine; GIBCO #510-3118)
30 g sucrose
18 g mannitol
0.5 mg/L 2,4-D
0.3 mg/L kinetin
0.6% agarose
pH 5.8

### Brassica Regeneration Medium BS-48

Murashige and Skoog Minimal Organic Medium
Gamborg B5 Vitamins (SIGMA #1019)
10 g glucose
250 mg xylose
600 mg MES
0.4% agarose
pH 5.7

Filter-sterilize and add after autoclaving:
2.0 mg/L zeatin
0.1 mg/L IAA

### Brassica Shoot Elongation Medium MSV-1A

Murashige and Skoog Minimal Organic Medium
Gamborg B5 Vitamins
10 g sucrose
0.6% agarose
pH 5.8

**TABLE 11**

| Canola transformants | | | | |
|---|---|---|---|---|
| BINARY VECTOR | NUMBER OF CUT ENDS | NUMBER OF KAN^{R} CALLI | NUMBER OF SHOOTING CALLI | NUMBER OF PLANTS |
| pZS199 | 120 | 41 | 5 | 2 |
| pFS926 | 600 | 278 | 52 | 28 |
| pBT593 | 600 | 70 | 10 | 3 |
| pBT597 | 600 | 223 | 40 | 23 |

### EXAMPLE 17

### Transformation of Maize Using a Chimeric lysC-M4 Gene as a Selectable Marker

Embryogenic callus cultures were initiated from immature embryos (about 1.0 to 1.5 mm) dissected from kernels of a corn line bred for giving a "type II callus" tissue culture response. The embryos were dissected 10 to 12 d after pollination and were placed with the axis-side down and in contact with agarose-solidified N6 medium [Chu et al. (1974) Sci Sin 18:659-668] supplemented with 0.5 mg/L 2,4-D (N6-0.5). The embryos were kept in the dark at 27°C. Friable embryogenic callus consisting of undifferentiated masses of cells with somatic proembryos and somatic embryos borne on suspensor structures proliferated from the scutellum of the immature embryos. Clonal embryogenic calli isolated from individual embryos were identified and sub-cultured on N6-0.5 medium every 2 to 3 weeks.

The particle bombardment method was used to transfer genes to the callus culture cells. A Biolistic, PDS-1000/He (BioRAD Laboratories, Hercules, CA) was used for these experiments.

The plasmid pBT573, containing the chimeric gene HH534 5' region/ mcts/lysC-M4/HH2-1 3' region (see Example 6) designed for constitutive gene expression in corn, was precipitated onto the surface of gold particles. To accomplish this 2.5 µg of pBT573 (in water at a concentration of about 1 mg/mL) was added to 25 mL of gold particles (average diameter of 1.5 µm) suspended in water (60 mg of gold per mL). Calcium chloride (25 mL of a 2.5 M solution) and spermidine (10 mL of a 1.0 M solution) were then added to the gold-DNA suspension as the tube was vortexing. The gold particles were centrifuged in a microfuge for 10 sec and the supernatant removed. The gold particles were then resuspended in 200 mL of absolute ethanol, were centrifuged again and the supernatant removed. Finally, the gold particles were resuspended in 25 mL of absolute ethanol and sonicated twice for one sec. Five µL of the DNA-coated gold particles were then loaded on each macro carrier disk and the ethanol was allowed to evaporate away leaving the DNA-covered gold particles dried onto the disk.

Embryogenic callus (from the callus line designated #132.2.2) was arranged in a circular area of about 6 cm in diameter in the center of a 100 X 20 mm petri dish containing N6-0.5 medium supplemented with 0.25M sorbitol and 0.25M mannitol. The tissue was placed on this medium for 2 h prior to bombardment as a pretreatment and remained on the medium during the bombardment procedure. At the end of the 2 h pretreatment period, the petri dish containing the tissue was placed in the chamber of the PDS-1000/He. The air in the chamber was then evacuated to a vacuum of 28 inch of Hg. The macrocarrier was accelerated with a helit shock wave using a rupture membrane that bursts when the He pressure in the shock tube reaches 1100 psi. The tissue was placed approximately 8 cm from the stopping screen. Four plates of tissue were bombarded with the DNA-coated gold particles. Immediately following bombardment, the callus tissue was transferred to N6-0.5 medium without supplemental sorbitol or mannitol.

Seven d after bombardment small (2-4 mm diameter) clumps of callus tissue were transferred to N6-0.5 medium lacking casein or proline, but supplemented with 2mM each of lysine and threonine (LT). The tissue continued to grow slowly on this medium and was transferred to fresh N6-0.5 medium supplemented with LT every 2 weeks. After 12 weeks two clones of actively growing callus was identified on two separate plates containing LT-supplemented medium. These clones continued to grow when sub-cultured on the selective medium. The presence of the lysC-M4 gene in the selected clones was confirmed by PCR analysis. Callus was transferred to medium that promotes plant regeneration.

### EXAMPLE 18

### Transformation of Corn with the Constitutive Corn Promoter/cts/ecodapA and Constitutive Corn Promoter/cts/lysC-M4

The chimeric gene cassettes, HH534 5' region/mcts/ecodapA/HH2-1 3' region plus HH534 5' region/mcts/lysC-M4/HH2-1 3' region, (Example 6) were inserted into the vector pGem9z to generate a corn transformation vector. Plasmid pBT583 (Example 6) was digested with Sal I and an 1850 bp fragment containing the HH534 5' region/mcts/ecodapA/HH2-1 3' region gene cassette was isolated. This DNA fragment was inserted into pBT573 (Example 6), which carries the HH534 5' region/mcts/lysC-M4/HH2-1 3' region, digested with Xho I. The resulting vector with both chimeric genes in the same orientation was designated pBT586.

Vector pBT586 was introduced into embryogenic corn callus tissue using the particle bombardment method. The establishment of the embryogenic callus cultures and the parameters for particle bombardment were as described in Example 13.

Either one of two plasmid vectors containing selectable markers were used in the transformations. One plasmid, pALSLUC [Fromm et al. (1990) Biotechnology 8:833-839], contained a cDNA of the maize acetolactate synthase (ALS) gene. The ALS cDNA had been mutated in vitro so that the enzyme coded by the gene would be resistant to chlorsulfuron. This plasmid also contains a gene that uses the 35S promoter from Cauliflower Mosaic Virus and the 3' region of the nopaline synthase gene to express a firefly luciferase coding region [de Wet et al. (1987) Molec. Cell Biol. 7:725-737]. The other plasmid, pDETRIC, contained the bar gene from Streptomyces hygroscopicus that confers resistance to the herbicide glufosinate [Thompson et al. (1987 The EMBO Journal 6:2519-2523]. The bacterial gene had its translation codon changed from GTG to ATG for proper translation initiation in plants [De Block et al. (1987) The EMBO Journal 6:2513-2518]. The bar gene was driven by the 35S promoter from Cauliflower Mosaic Virus and uses the termination and polyadenylation signal from the octopine synthase gene from Agrobacterium tumefaciens.

For bombardment, 2.5 µg of each plasmid, pBT586 and one of the two selectable marker plasmids, was coprecipitated onto the surface of gold particles as described in Example 13. Bombardment of the embryogenic tissue cultures was also as described in Example 13.

Seven d after bombardment the tissue was transferred to selective medium. The tissue bombarded with the selectable marker pALSLUC was transferred to N6-0.5 medium that contained chlorsulfuron (30 ng/L) and lacked casein or proline. The tissue bombarded with the selectable marker, pDETRIC, was transferred to N6-0.5 medium that contained 2 mg/L glufosinate and lacked casein or proline. The tissue continued to grow slowly on these selective media. After an additional 2 weeks the tissue was transferred to fresh N6-0.5 medium containing the selective agents.

Chlorsulfuron- and glufosinate-resistance callus clones could be identified after an additional 6-8 weeks. These clones continued to grow when transferred to the selective media.

The presence of pBT586 in the transformed clones has been confirmed by PCR analysis. Functionality of the introduced AK enzyme was tested by plating out transformed clones on N6-0.5 media containing 2 mM each of lysine and threonine (LT selection; see example 13). All of the clones were capable of growing on LT medium indicating that the E. coli aspartate kinase was expressed and was functioning properly. To test that the E. coli DHDPS enzyme was functional, transformed callus was plated on N6-0.5 media containing 2µm 2-aminoethylcysteine (AEC), a lysine analog and potent inhibitor of plant DHDPS. The transformed callus tissue was resistant to AEC indicating that the introduced DHDPS, which is about 16-fold less sensitive to AEC than the plant enzyme, was being produced and was functional. Plants have been regenerated from several transformed clones and are being grown to maturity.

### EXAMPLE 19

### Transformation of Soybean with the Phaseolin Promoter/cts/cordapA and Phaseolin Promoter/cts/lysC-M4 Chimeric Genes

The chimeric gene cassettes, phaseolin 5' region/cts/cordapA/phaseolin 3' region plus phaseolin 5' region/cts/lysC-M4/phaseolin 3', (Example 6) were inserted into the soybean transformation vector pBT603 (Figure 5). This vector has a soybean transformation marker gene consisting of the 35S promoter from Cauliflower Mosaic Virus driving expression of the E. coli β-glucuronidase gene [Jefferson et al. (1986) Proc. Natl. Acad. Sci. USA 83:8447-8451] with the Nos 3' region in a modified pGEM9Z plasmid.

To insert the phaseolin 5' region/cts/lysC-M4/phaseolin 3' region, the gene cassette was isolated as a 3.3 kb Hind III fragment and inserted into Hind III digested pBT603, yielding plasmid pBT609. This binary vector has the chimeric gene, phaseolin 5' region/cts/lysC-M4/phaseolin 3' region inserted in the opposite orientation from the 35S/GUS/Nos 3' marker gene.

To insert the phaseolin 5' region/cts/cordapA/phaseolin 3'region 3', the gene cassette was isolated as a 2.7 kb BamH I fragment (as described in Example 15) and inserted into BamH I digested pBT609, yielding plasmid pBT614. This vector has both chimeric genes, phaseolin 5' region/cts/cordapA/phaseolin 3' region inserted in the same orientation, and both are in the opposite orientation from the 35S/GUS/Nos 3' marker gene.

Soybean was transformed with plasmid pBT614 according to the procedure described in United States Patent No. 5,015,580. Soybean transformation was performed by Agracetus Company (Middleton, WI).

### EXAMPLE 20

### Isolation of a Plant Lysine Ketoglutarate Reductase Gene

Lysine Ketoglutarate Reductase (LKR) enzyme activity has been observed in immature endosperm of developing maize seeds [Arruda et al. (1982) Plant Physiol. 69:988-989]. LKR activity increases sharply from the onset of endosperm development, reaches a peak level at about 20 d after pollination, and then declines [Arruda et al. (1983) Phytochemistry 22:2687-2689].

In order to clone the corn LKR gene, RNA was isolated from developing seeds 19 d after pollination. This RNA was sent to Clontech Laboratories, Inc., (Palo Alto, CA) for the custom synthesis of a cDNA library in the vector Lambda Zap II. The conversion of the Lambda Zap II library into a phagemid library, then into a plasmid library was accomplished following the protocol provided by Clontech. Once converted into a plasmid library the ampicillin-resistant clones obtained carry the cDNA insert in the vector pBluescript SK(-). Expression of the cDNA is under control of the lacZ promoter on the vector.

Two phagemid libraries were generated using the mixtures of the Lambda Zap II phage and the filamentous helper phage of 100 µL to 1 µL. Two additional libraries were generated using mixtures of 100 µL Lambda Zap II to 10 µL helper phage and 20 µL Lambda Zap II to 10 µL helper phage. The titers of the phagemid preparations were similar regardless of the mixture used and were about 2 x 10³ ampicillin-resistant-transfectants per mL with E. coli strain XL1-Blue as the host and about 1 x 10³ with DE126 (see below) as host.

To select clones that carried the LKR gene a specially designed E. coli host, DE126 was constructed. Construction of DE126 occurred in several stages.
(1) A generalized transducing stock of coliphage P1vir was produced by infection of a culture of TST1 [F^{-,} araD139, delta(argF-lac)205, flb5301, ptsF25, relA1, rpsL150, malE52::Tn10, deoC1, λ⁻] (E. coli Genetic Stock Center #6137) using a standard method (for Methods see J. Miller, Experiments in Molecular Genetics).
(2) This phage stock was used as a donor in a transductional cross (for Method see J. Miller, Experiments in Molecular Genetics) with strain GIF106M1 [F⁻, arg⁻, ilvA296, lysC1001, thrA1101, metL1000, λ^{-,} rpsL9, malT1, xyl-7, mtl-2, thil(?), supE44(?)] (E. coli Genetic Stock Center #5074) as the recipient. Recombinants were selected on rich medium [L supplemented with DAP] containing the antibiotic tetracycline. The transposon Tn10, conferring tetracycline resistance, is inserted in the malE gene of strain TST1. Tetracycline-resistant transductants derived from this cross are likely to contain up to 2 min of the E. coli chromosome in the vicinity of malE. The genes malE and lysC are separated by less than 0.5 minutes, well within cotransduction distance.
(3) 200 tetracycline-resistant transductants were thoroughly phenotyped; appropriate fermentation and nutritional traits were scored. The recipient strain GIF106M1 is completely devoid of aspartokinase isozymes due to mutations in thrA, metL and lysC, and therefore requires the presence of threonine, methionine, lysine and meso-diaminopimelic acid (DAP) for growth. Transductants that had inherited lysC⁺ with malE::Tn10 from TST1 would be expected to grow on a minimal medium that contains vitamin B1, L-arginine, L-isoleucine and L-valine in addition to glucose which serves as a carbon and energy source. Moreover strains having the genetic constitution of lysC⁺, metL- and thrA- will only express the lysine sensitive aspartokinase. Hence addition of lysine to the minimal medium should prevent the growth of the lysC⁺ recombinant by leading to starvation for threonine, methionine and DAP. Of the 200 tetracycline resistant transductants examined, 49 grew on the minimal medium devoid of threonine, methionine and DAP.
   Moreover, all 49 were inhibited by the addition of L-lysine to the minimal medium. One of these transductants was designated DE125. DE125 has the phenotype of tetracycline resistance, growth requirements for arginine, isoleucine and valine, and sensitivity to lysine. The genotype of this strain is F⁻ malE52::Tn10 arg- ilvA296 thrA1101 metL1000 lambda-rpsL9 malT1 xyl-7 mtl-2 thi1(?) supE44(?).
(4) This step involves production of a male derivative of strain DE125. Strain DE125 was mated with the male strain AB1528 [F'16/delta(gpt-proA)62, lacY1 or lacZ4, glnV44, galK2 rac⁻(?), hisG4, rfbd1, mgl-51, kdgK51(?), ilvC7, argE3, thi-1] (E. coli Genetic Stock Center #1528) by the method of conjugation. F'16 carries the ilvGMEDAYC gene cluster. The two strains were cross streaked on rich medium permissive for the growth of each strain. After incubation, the plate was replica plated to a synthetic medium containing tetracycline, arginine, vitamin B1 and glucose. DE125 cannot grow on this medium because it cannot synthesize isoleucine. Growth of AB1528 is prevented by the inclusion of the antibiotic tetracycline and the omission of proline and histidine from the synthetic medium. A patch of cells grew on this selective medium. These recombinant cells underwent single colony isolation on the same medium. The phenotype of one clone was determined to be Ilv^{+,} Arg⁻, TetR, Lysine-sensitive, male specific phage (MS2)-sensitive, consistent with the simple transfer of F'16 from AB1528 to DE125. This clone was designated DE126 and has the genotype F'16/malE52::Tn10, arg⁻, ilvA296, thrA1101, metL100, lysC⁺, λ⁻, rpsL9, malT1, xyl-7, mtl-2, thi-1?, supE44?. It is inhibited by 20 µg/mL of L-lysine in a synthetic medium.

To select for clones from the corn cDNA library that carried the LKR gene, 100 µL of the phagemid library was mixed with 100 µL of an overnight culture of DE126 grown in L broth and the cells were plated on synthetic media containing vitamin B1, L-arginine, glucose as a carbon and energy source, 100 µg/mL ampicillin and L-lysine at 20, 30 or 40 µg/mL. Four plates at each of the three different lysine concentrations were prepared. The amount of phagemid and DE126 cells was expected to yield about 1 x 10⁵ ampicillin-resistant transfectants per plate. Ten to thirty lysine-resistant colonies grew per plate (about 1 lysine-resistant per 5000 ampicillin-resistant colonies).

Plasmid DNA was isolated from 10 independent clones and retransformed into DE126. Seven of the ten DNAs yielded lysine-resistant clones demonstrating that the lysine-resistance trait was carried on the plasmid. The reported size of corn LKR is about 140,000 daltons, requiring an mRNA of at least 3.8 kb. Restriction enzyme analysis of the DNAs showed that clone 11D contained a plasmid with an approximately 4 kb insert of corn DNA, large enough to carry a full-length cDNA.

## Claims

1. A chimeric gene comprising an isolated nucleic acid fragment encoding all or part of an antisense lysine ketoglutarate reductase, wherein expression of the chimeric gene reduces or eliminates ketoglutarate reductase expression in seeds of transformed plants.

2. A chimeric gene of claim 1, wherein said nucleic acid fragment is linked in reverse orientation to a seed-specific promoter sequence.

3. A chimeric gene as claimed in claim 1 or claim 2, said gene comprising an isolated nucleic acid fragment comprising
(a) a first nucleic acid subfragment encoding aspartokinase which is insensitive to inhibition by lysine and
(b) a second nucleic acid subfragment encoding dihydrodipicolinic acid synthase which is at least 20-fold less sensitive to inhibition by lysine than plant DHDPS and
(c) a third nucleic acid fragment encoding all or part of a lysine ketoglutarate reductase as defined in claim 1,
wherein the first and second subfragments are each operably linked to a suitable plant chloroplast transit sequence and to a suitable regulatory sequence to promote expression in seeds of transformed plants and the third nucleic acid subfragment is linked to a suitable regulatory sequence to promote expression of antisense RNA in seeds of transformed plants.

4. The chimeric gene of claim 3 in which:
(a) the first nucleic acid subfragment comprises a nucleotide sequence as shown in SEQ ID NO: 1 encoding a lysine-insensitive variant of *E. coli* AKIII and further **characterised in that** at least one of the following conditions is met:
(1) the amino acid at position 318 is an amino acid other than methionine, or
(2) the amino acid at position 352 is an amino acid other than threonine; or
(b) the second nucleic acid subfragment is derived from bacteria.

5. A plant comprising in its genome the nucleic acid fragment of any one of claims 1 to 4.

6. Seeds obtained from the plant of claim 5 comprising in its genome the nucleic acid fragment of any one claims 1 to 4.

7. A method for increasing the lysine content of the seeds of plants comprising:
(a) transforming plant cells with the nucleic acid fragment of claim 3;
(b) growing fertile mature plants from the transformed plant cells obtained from step (a) under conditions suitable to obtain seeds; and
(c) selecting from the progeny seed of step (b) those seeds containing increased levels of lysine.

8. A plant produced by the method of claim 7 for increasing the lysine content of the seeds of plants, wherein the plant is capable of transmitting said nucleic acid fragments to a progeny plant and wherein the progeny plant has the ability to produce levels of free lysine at least two times greater than free lysine levels of plants not containing the nucleic acid fragment.

9. A method of preparing a transgenic plant having an increased lysine content in the seeds, comprising transforming a plant with the nucleic acid fragment of claim 3.

## Patentansprüche

1. Ein chimäres Gen, umfassend ein isoliertes Nukleinsäurefragment, welches für eine Antisense-Lysin-Ketoglutarat-Reduktase ganz oder zum Teil codiert, wobei die Expression des chimären Gens die Expression der Ketoglutarat-Reduktase in Samen transformierter Pflanzen reduziert oder eliminiert.

2. Ein chimäres Gen nach Anspruch 1, worin das Nukleinsäurefragment in umgekehrter Orientierung mit einer Samen-spezifischen Promotor-Sequenz verkettet ist.

3. Ein chimäres Gen wie in Anspruch 1 oder Anspruch 2 beansprucht, wobei das Gen ein isoliertes Nukleinsäurefragment umfasst, welches
(a) ein erstes Nukleinsäureunterfragment umfasst, das für Aspartokinase codiert, welche gegenüber Hemmung durch Lysin unempfindlich ist, und
(b) ein zweites Nukleinsäureunterfragment umfasst, das für Dihydrodipicolinsäure-Synthase codiert, welche mindestens 20fach weniger empfindlich gegenüber Hemmung durch Lysin ist als Pflanzen-DHDPS, und
(c) ein drittes Nukleinsäurefragment umfasst, welches für eine Lysin-Ketoglutarat-Reduktase ganz oder zum Teil codiert wie in Anspruch 1 definiert,
wobei das erste und das zweite Unterfragment beide jeweils wirksam verkettet sind mit einer geeigneten pflanzlichen Chloroplasten-Transit-Sequenz und mit einer geeigneten regulatorischen Sequenz, um Expression im Samen transformierter Pflanzen zu fördern, und das dritte Nukleinsäureunterfragment mit einer geeigneten regulatorischen Sequenz verkettet ist, um Expression der Antisense-RNS in Samen transformierter Pflanzen zu fördern.

4. Das chimäre Gen nach Anspruch 3, in welchem:
(a) das erste Nukleinsäureunterfragment eine Nukleotidsequenz umfasst, wie in SEQ ID NO: 1 gezeigt, welche für eine Lysin-unempfindliche Variante von *E*. *coli* AKIII codiert und weiterhin **dadurch gekennzeichnet ist, dass** mindestens eine der nachfolgenden Bedingungen zutrifft:
(1) Die Aminosäure an Position 318 ist eine andere Aminosäure als Methionin, oder
(2) die Aminosäure an Position 352 ist eine andere Aminosäure als Threonin; oder
(b) das zweite Nukleinsäureunterfragment von Bakterien stammt.

5. Eine Pflanze, welche in ihrem Genom das Nukleinsäurefragment nach einem der Ansprüche 1 bis 4 umfasst.

6. Samen, welche von der Pflanze nach Anspruch 5, die in ihrem Gen das Nukleinsäurefragment nach einem der Ansprüche 1 bis 4 umfasst, erhalten wurden.

7. Ein Verfahren zur Steigerung des Lysingehaltes der Pflanzensamen, umfassend:
(a) Transformieren der Pflanzenzellen mit dem Nukleinsäurefragment nach Anspruch 3;
(b) Aufziehen fertiler reifer Pflanzen aus den transformierten Pflanzenzellen, erhalten nach Schritt (a), unter geeigneten Bedingungen, um Samen zu erhalten; und
(c) Selektion solcher Samen vom Samen der Nachkommenschaft nach Schritt (b), welche erhöhte Spiegel an Lysin enthalten.

8. Eine Pflanze, produziert nach dem Verfahren von Anspruch 7 zur Steigerung des Lysin-Gehaltes der Pflanzensamen, wobei die Pflanze fähig ist zur Vererbung der Nukleinsäurefragmente an eine Pflanze der Nachkommenschaft und wobei die Pflanze der Nachkommenschaft die Fähigkeit hat, Spiegel von freiem Lysin zu produzieren, die mindestens zweimal höher sind als freie Lysin-Spiegel von Pflanzen, die nicht das Nukleinsäurefragment enthalten.

9. Ein Verfahren zur Bereitung einer transgenen Pflanze mit einem gesteigerten Lysingehalt in den Samen, umfassend die Transformation einer Pflanze mit dem Nukleinsäurefragment nach Anspruch 3.

## Revendications

1. Gène chimère comprenant un fragment d'acide nucléique isolé codant pour tout ou partie d'une lysine cétoglutarate réductase antisens, dans lequel l'expression du gène chimère réduit ou élimine l'expression de cétoglutarate réductase dans des graines de plantes transformées.

2. Gène chimère selon la revendication 1, dans lequel ledit fragment d'acide nucléique est lié en orientation inverse à une séquence promotrice spécifique de graines.

3. Gène chimère selon la revendication 1 ou la revendication 2, ledit gène comprenant un fragment d'acide nucléique isolé comprenant
(a) un premier soul-fragment d'acide nucléique codant pour l'aspartokinase qui est insensible à l'inhibition par de la lysine et
(b) un second soul-fragment d'acide nucléique codant pour l'acide dihydrodipicolinique synthase qui est au moins 20 fois moins sensible à l'inhibition par la lysine que la DHDPS de plante et
(c) un troisième fragment d'acide nucléique codant pour tout ou partie d'une lysine cétoglutarate réductase telle que définie dans la revendication 1,
dans lequel les premier et second sous-fragments sont chacun liés de façon opératoire à une séquence de transit de chloroplaste végétal appropriée et à une séquence régulatrice appropriée pour activer l'expression dans des graines de plantes transformées, et le troisième sous-fragment d'acide nucléique est lié à une séquence régulatrice appropriée pour activer l'expression d'ARN antisens dans des graines de plantes transformées.

4. Gène chimère selon la revendication 3, dans lequel:
(a) le premier soul-fragment d'acide nucléique comprend une séquence nucléotidique telle que présentée dans SEQ ID NO:1 codant pour une variante insensible à la lysine de *E*. *coli* AKIII et est en outre **caractérisé en ce qu'**au moins l'une des conditions suivantes est satisfaite:
(1) l'acide aminé en position 318 est un acide aminé autre que la méthionine, ou
(2) l'acide aminé en position 352 est un acide aminé autre que la thréonine; ou
(b) le second sons-fragment d'acide nucléique provient de bactéries.

5. Plante comprenant dans son génome le fragment d'acide nucléique selon l'une quelconque des revendications 1 à 4:

6. Graines obtenues à partir de la plante selon la revendication 5, comprenant dans son gène le fragment d'acide nucléique selon l'une quelconque des revendications 1 à 4.

7. Procédé pour augmenter la teneur en lysine des graines de plantes, comprenant:
(a) la transformation de cellules de plantes avec le fragment d'acide nucléique selon la revendication 3;
(b) la croissance de plantes matures fertiles à partir des cellules de plantes transformées obtenues dans l'étape (a) dans des conditions appropriées pour obtenir des graines; et
(c) le choix, dans la descendance de graines de l'étape (b), des graines contenant des teneurs accrues en lysine.

8. Plante produite par le procédé de la revendication 7 pour augmenter la teneur en lysine des graines de plantes, la plante étant apte à transmettre lesdits fragments d'acide nucléique à une descendance de plante, et dans laquelle la descendance de plante a l'aptitude de produire des taux de lysine libre au moins deux fois plus grands que les taux de lysine libre de plantes ne contenant pas le fragment d'acide nucléique.

9. Procédé de préparation d'une plante transgénique ayant une teneur en lysine accrue dans les graines, comprenant la transformation d'une plante avec le fragment d'acide nucléique selon la revendication 3.
